# EUROPÄISCHE PATENTSCHRIFT

(11) **EP 3 080 229 B1**
(45) Veröffentlichungstag und Bekanntmachung des Hinweises auf die Patenterteilung: **17.01.2018**
(21) Anmeldenummer: 14818868.3
(22) Anmeldetag: 18.11.2014
(51) Int. Cl.: C09K 11/06, C07C 211/00, H01L 51/50

(54) **MATERIALIEN FÜR ELEKTRONISCHE VORRICHTUNGEN**
MATERIALS FOR ELECTRONIC DEVICES
MATÉRIAUX POUR DISPOSITIFS ÉLECTRONIQUES

(30) Priorität: 12.12.2013 EP 13005800
(43) Veröffentlichungstag der Anmeldung: 19.10.2016
(73) Patentinhaber: Merck Patent GmbH, 64293 Darmstadt (DE)
(72) Erfinder: STOESSEL, Philipp, 60389 Frankfurt am Main (DE); MARTYNOVA, Irina, 64347 Griesheim (DE); MONTENEGRO, Elvira, 69469 Weinheim (DE); MUJICA-FERNAUD, Teresa, 64283 Darmstadt (DE); VOGES, Frank, 67098 Bad Duerkheim (DE); KROEBER, Jonas Valentin, 60311 Frankfurt am Main (DE); STIEBER, Frank, 64683 Einhausen (DE)
(86) Internationale Anmeldenummer: PCT/EP2014/003072
(87) Internationale Veröffentlichungsnummer: WO 2015/086108

(56) Entgegenhaltungen:
- WO-A1-2010/050779
- WO-A1-2011/136484
- US-A1- 2004 023 060

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen enthaltend diese Materialien.

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektronischen Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs, in denen organische Halbleiter als funktionelle Materialien eingesetzt werden, ist dem Fachmann bekannt und unter anderem in US 4539507, US 5151629, EP 0676461 und WO 1998/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6).

Gemäß dem Stand der Technik werden als Lochtransportmaterialien in der Lochtransportschicht bzw. in der Lochinjektionsschicht insbesondere Triarylaminderivate verwendet, welche entweder mindestens zwei Triarylaminogruppen oder mindestens eine Triarylaminogruppe und mindestens eine Carbazolgruppe aufweisen. Diese Verbindungen leiten sich häufig von Diarylamino-substituierten Triphenylaminen (TPA-Typ), von Diarylamino-substituierten Biphenyl-Derivaten (TAD-Typ) oder Kombinationen dieser Grundverbindungen ab. Weiterhin werden beispielsweise Spirobifluorenderivate eingesetzt, welche mit zwei oder vier Diarylaminogruppen substituiert sind (z. B. gemäß EP 676461 oder US 7,714,145). Bei diesen Verbindungen gibt es sowohl bei fluoreszierenden wie auch bei phosphoreszierenden OLEDs weiterhin Verbesserungsbedarf, insbesondere hinsichtlich Effizienz, Lebensdauer und Betriebsspannung bei Verwendung in einer organischen Elektrolumineszenzvorrichtung sowie hinsichtlich der thermischen Stabilität bei der Sublimation.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Lochtransportmaterial in einer Lochtransport bzw. Exzitonenblockierschicht oder als Matrixmaterial in einer emittierenden Schicht.

Überraschend wurde gefunden, dass bestimmte, unten näher beschriebene Verbindungen diese Aufgabe lösen und zu deutlichen Verbesserungen der organischen Elektrolumineszenzvorrichtung führen, insbesondere hinsichtlich der Lebensdauer, der Effizienz und der Betriebsspannung. Dies gilt für phosphoreszierende und fluoreszierende Elektrolumineszenzvorrichtungen, vor allem bei Einsatz der erfindungsgemäßen Verbindungen als Lochtransportmaterial oder als Matrixmaterial. Die Materialien weisen im Allgemeinen eine hohe thermische Stabilität auf und lassen sich daher unzersetzt und rückstandsfrei sublimieren. Diese Materialien sowie elektronische Vorrichtungen, welche derartige Verbindungen enthalten, sind daher der Gegenstand der vorliegenden Erfindung.

Die Schrift WO 02/088274 A1 zeigt die Synthese von Dispiro[fluoren-9,9'-anthracen-10',9"-fluoren]-Derivaten, welche insbesondere an der Anthracen-Einheit substituiert sind.

Dispiro[fluoren-9,9'-anthracen-10',9"-fluoren]-Derivate, die eine Diarylaminogruppe aufweisen, die wiederum mit einer Diarylaminogruppe substituiert ist, sind in WO 2011/136484 offenbart.

Der Übersichtlichkeit halber ist die Nummerierung des Dispiro[fluoren-9,9'-anthracen-10',9"-fluoren]-Gerüsts im Folgenden abgebildet:

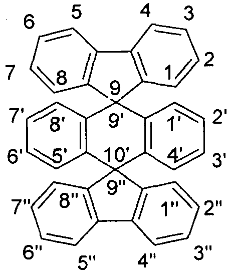

Gegenstand der vorliegenden Erfindung ist daher eine Verbindung gemäß Anspruch 1.

Eine Arylgruppe im Sinne dieser Erfindung enthält 6 bis 60 aromatische Ringatome; eine Heteroarylgruppe im Sinne dieser Erfindung enthält prinzipiell 5 bis 60 aromatische Ringatome, von denen mindestens eines ein Heteroatom darstellt. Die Heteroatome sind bevorzugt ausgewählt aus N, O und/oder S. Die stellt die grundlegende Definition dar. Werden in der Beschreibung der vorliegenden Erfindung andere Bevorzugungen angegeben, wie beispielsweise bezüglich der Zahl der aromatischen Ringatome oder der enthaltenen Heteroatome, so gelten diese.

Dabei wird unter einer Arylgruppe entweder ein einfacher aromatischer Cyclus, also Benzol, oder ein kondensierter (anellierter) aromatischer bzw. heteroaromatischer Polycyclus, beispielsweise Naphthalin, Anthracen, Phenanthren, Chinolin, Isochinolin, oder Carbazol, verstanden. Ein kondensierter (anellierter) aromatischer Polycyclus besteht im Sinne der vorliegenden Anmeldung aus zwei oder mehr miteinander kondensierten einfachen aromatischen Cyclen. Miteinander durch Einfachbindung verknüpfte Aromaten, wie zum Beispiel Biphenyl oder Fluoren, werden dagegen nicht als Aryl- oder Heteroarylgruppe, sondern als aromatisches Ringsystem bezeichnet.

Dabei umfasst eine Heteroarylgruppe im Sinne dieser Erfindung mindestens ein Heteroatom im aromatischen Cyclus oder Polycyclus, bevorzugt ist ein Heteroatom ausgewählt aus N, O oder S. Eine Heteroarylgruppe ist beispielsweise ein einfacher heteroaromatischer Cyclus, beispielsweise Pyridin, Pyrimidin, Thiophen, etc., oder ein kondensierter (anellierter) heteroaromatischer Polycyclus, beispielsweise Carbazol oder Chinolin.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 aromatische Ringatome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³- hybridisiertes C-, Si-, oder O-Atom, ein sp²-hybridisiertes C-Atom oder ein sp-hybridisiertes C-Atom, verbunden sein können. So sollen beispielsweise auch Systeme wie 9,9'-Spirobifluoren, 9,9'-Diarylfluoren, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Quaterphenyl. Dabei enthält das aromatische oder heteroaromatische Ringsystem definitionsgemäß keine Aminogruppen. Triarylaminogruppen sind somit von der Definition eines aromatischen oder heteroaromatischen Ringsystems nicht umfasst.

Unter einem aromatischen oder heteroaromatischen Ringsystem mit 5 bis 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis-oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8- Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3- Oxadiazol, 1,2,4-Oxadiazol, 1,2,5-Oxadiazol, 1,3,4-Oxadiazol, 1,2,3- Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyciooctylthio, 2- Ethylhexylthio, Trifluormethylthio, Pentafluorethylthio, 2,2,2- Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung verknüpft sind. Dies wird durch das folgende Schema verdeutlicht: Ringbildung

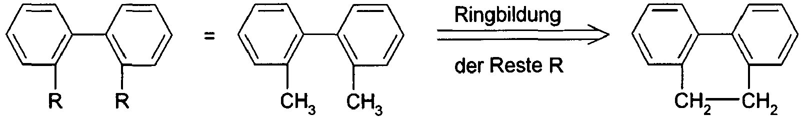

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

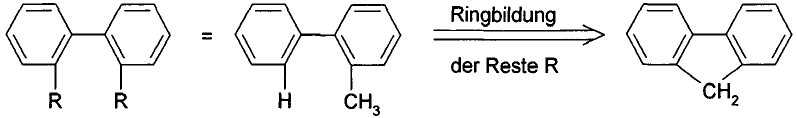

In einer bevorzugten Ausführungsform der Erfindung ist s+t+u gleich 0 oder 1. Die Verbindung umfasst dann eine oder zwei Diarylaminogruppen.

In einer weiteren bevorzugten Ausführungsform der Erfindung sind m, n, o, p, q, r gleich oder verschieden 0 oder 1, bevorzugt sind m, o, p, r gleich 0, ganz besonders bevorzugt sind m, o, p, r gleich 0 und n, q gleich 0 oder 1.

In einer weiteren bevorzugten Ausführungsform der Erfindung sind alle Index i in der Formel (1) bei jedem Auftreten gleich, d.h. alle Index i haben beispielsweise den Wert 0. Wenn i gleich 0 ist, ist das Stickstoffatom direkt an das Grundgerüst gebunden.

In einer weiteren bevorzugten Ausführungsform der Erfindung umfasst mindestens eine Gruppe -(Ar^{S})ᵢNAr¹Ar² insgesamt mindestens 18 aromatische Ringatome, besonders bevorzugt mindestens 18 und weniger als 50 aromatische Ringatome.

In einer bevorzugten Ausführungsform ist die Verbindung der Formel (1) eine Verbindung gemäß einer der folgenden Formeln (2) bis (5): 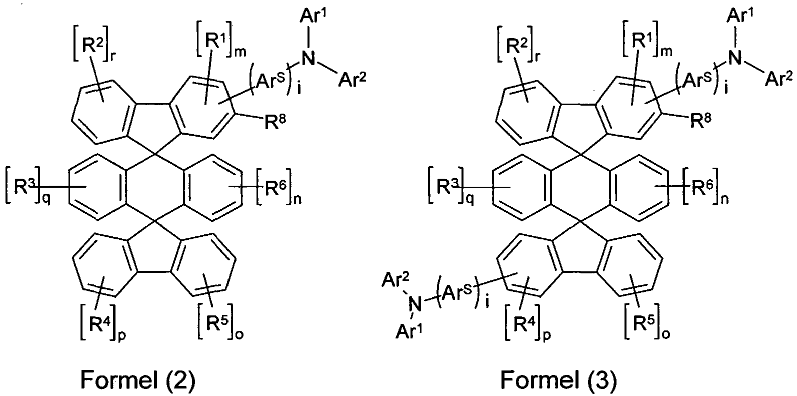 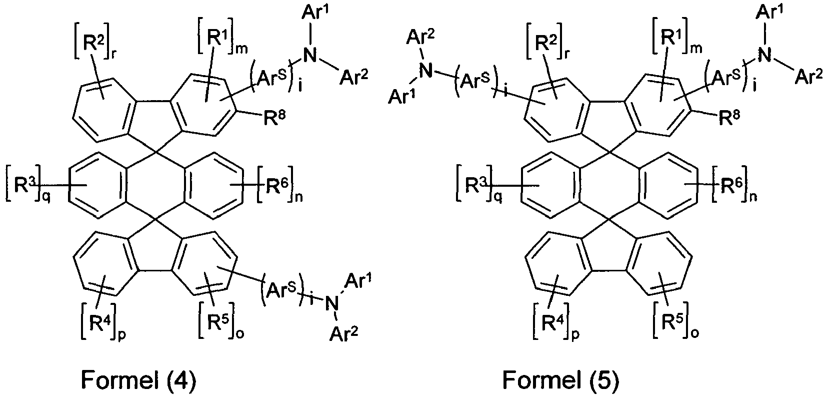 wobei die Symbole und Indizes die gleiche Bedeutung wie für Formel (1) haben.

In einer besonders bevorzugten Ausführungsform ist die Verbindung der Formel (1) eine Verbindung gemäß einer der folgenden Formeln (6) bis (28): 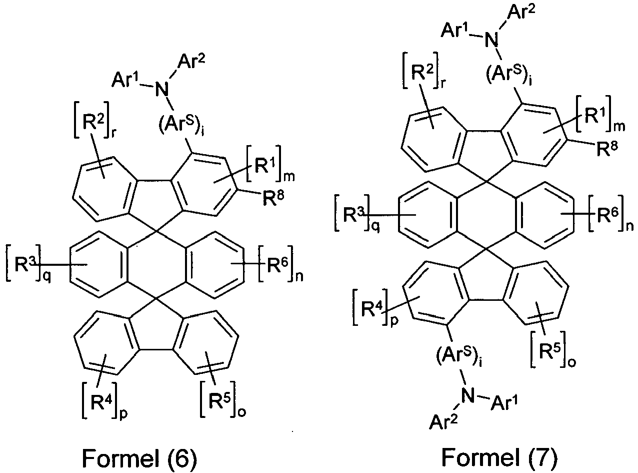 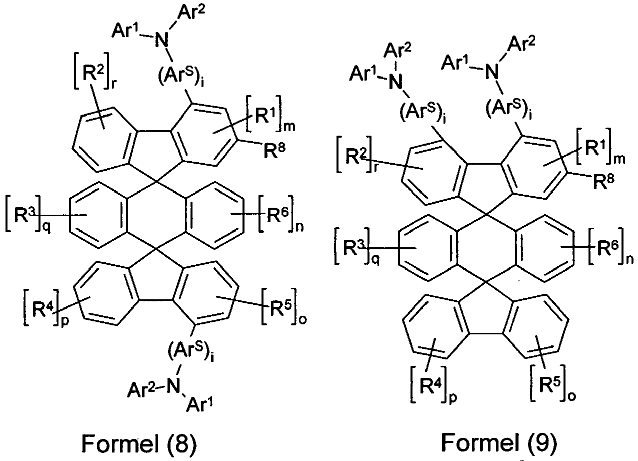 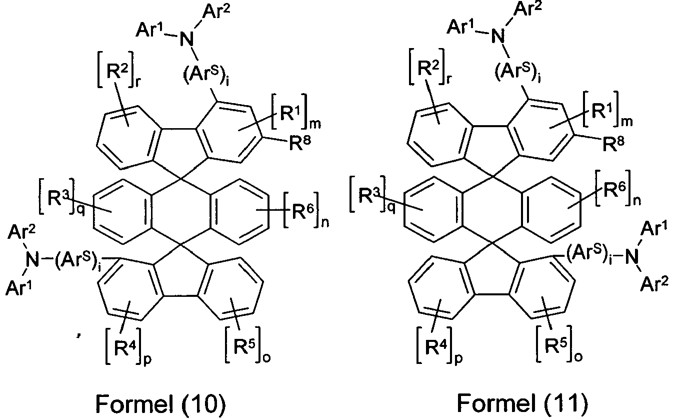 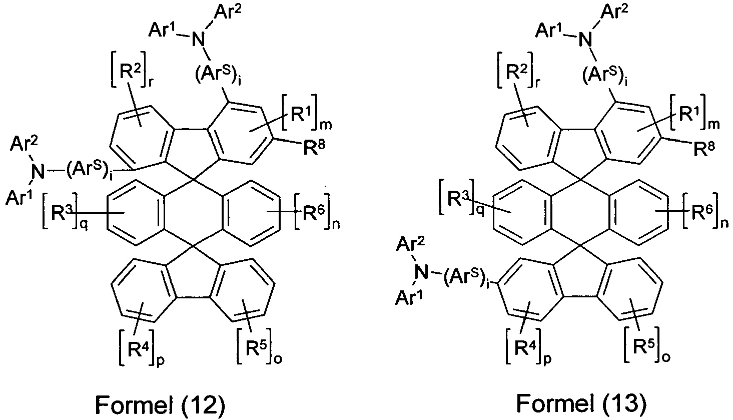 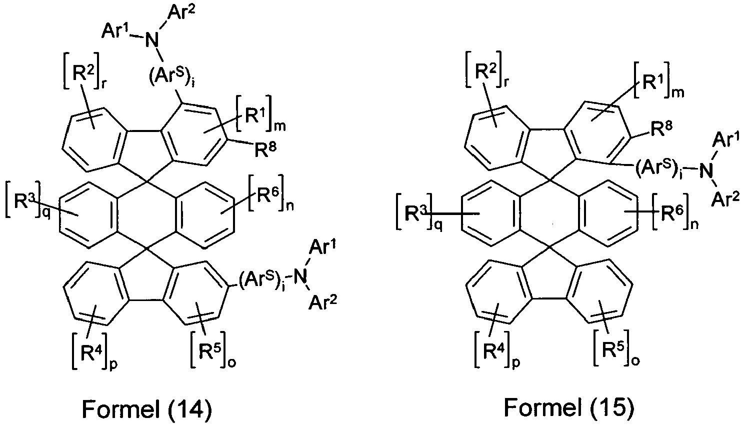 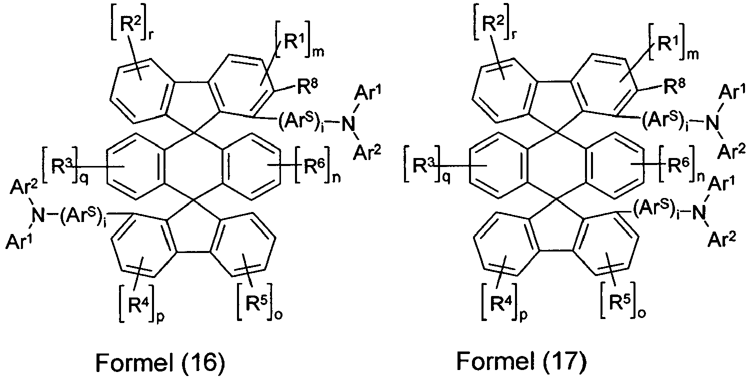 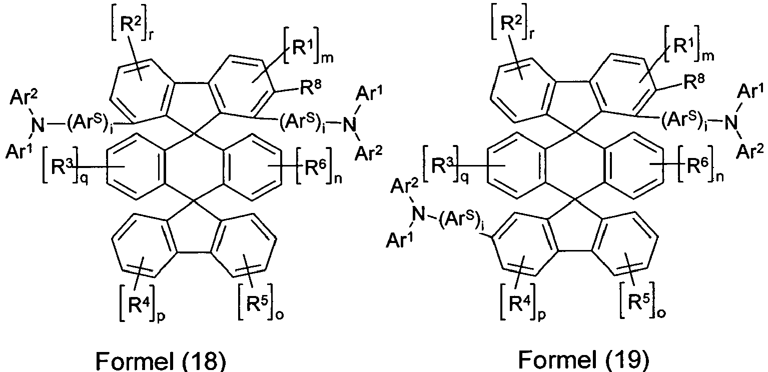 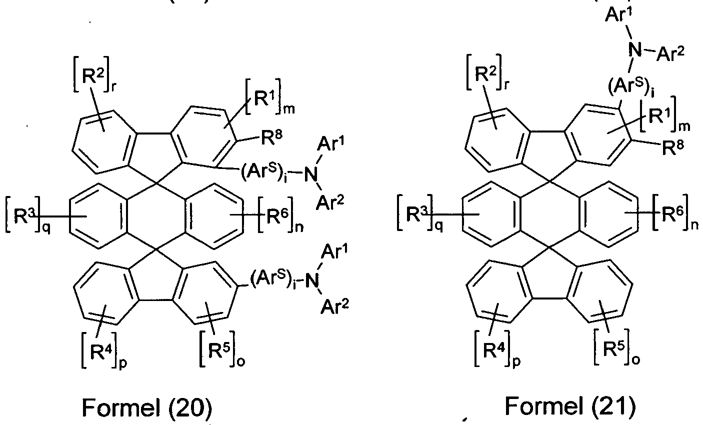 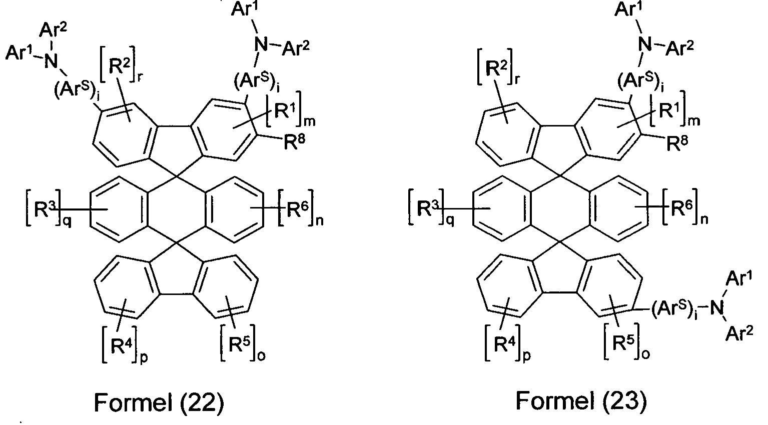 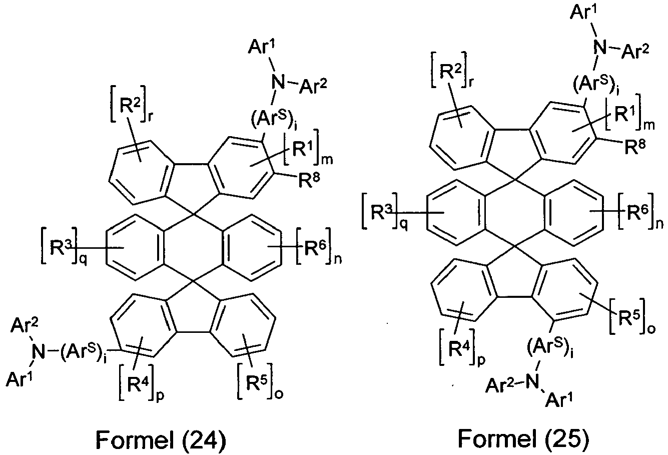 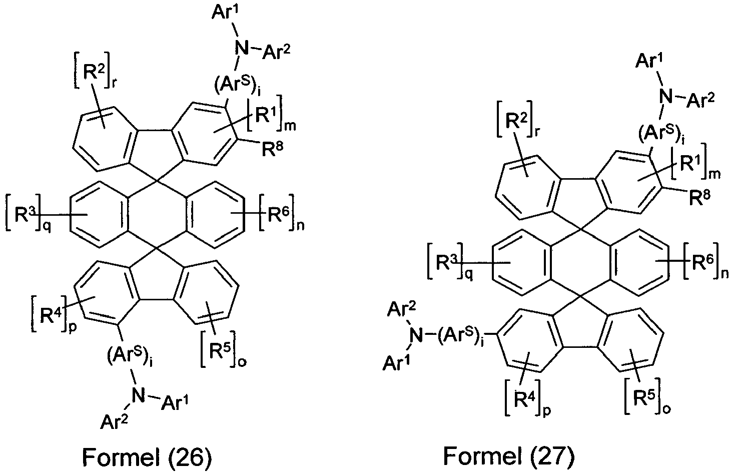 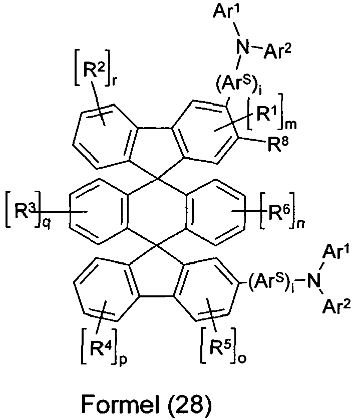 wobei die Symbole und Indizes die gleiche Bedeutung wie für Formel (1) haben.

In einer weiteren besonders bevorzugten Ausführungsform ist die Verbindung der Formel (1) eine Verbindung gemäß einer der folgenden Formeln (6a) bis (28a): 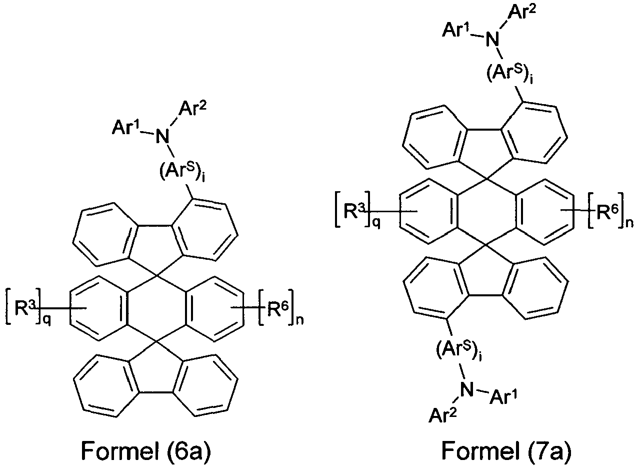 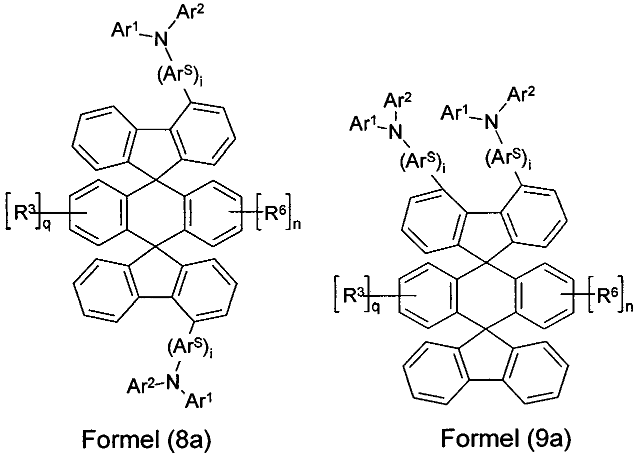 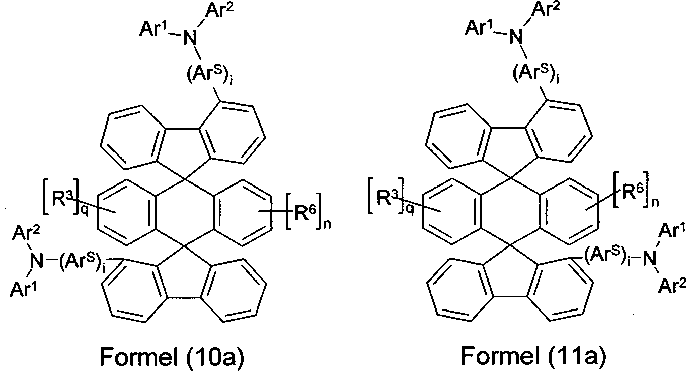 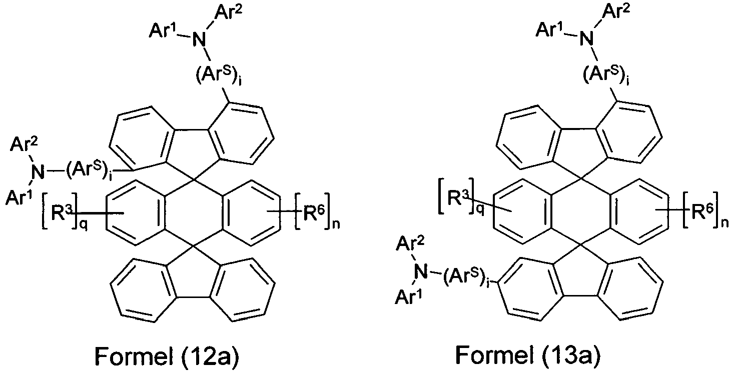 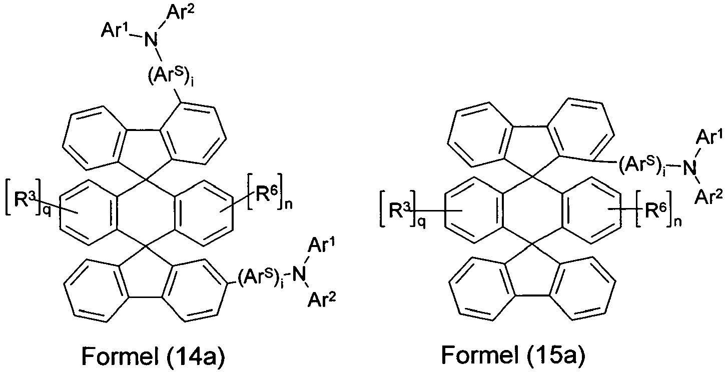 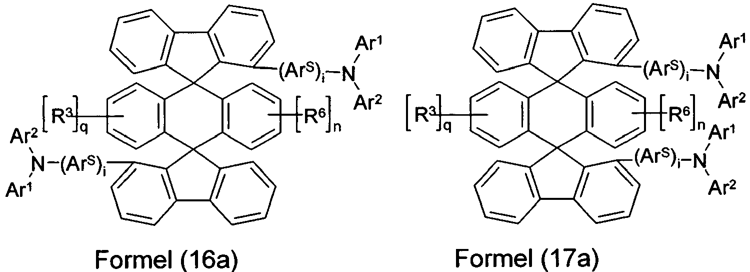 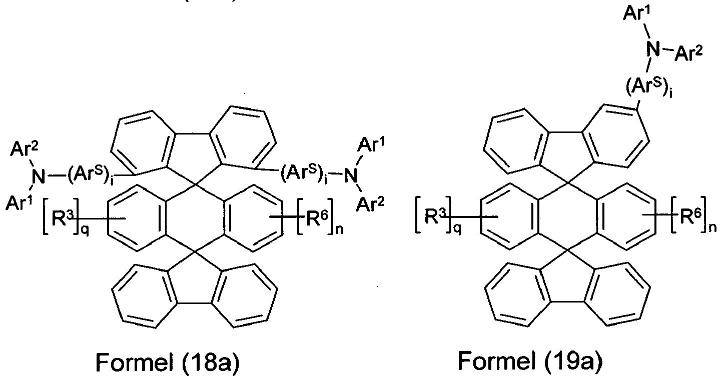 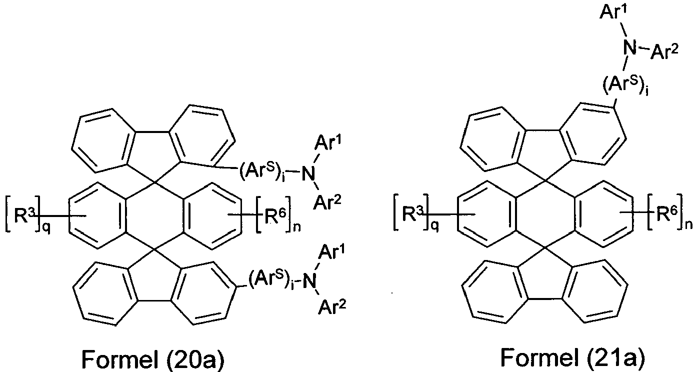 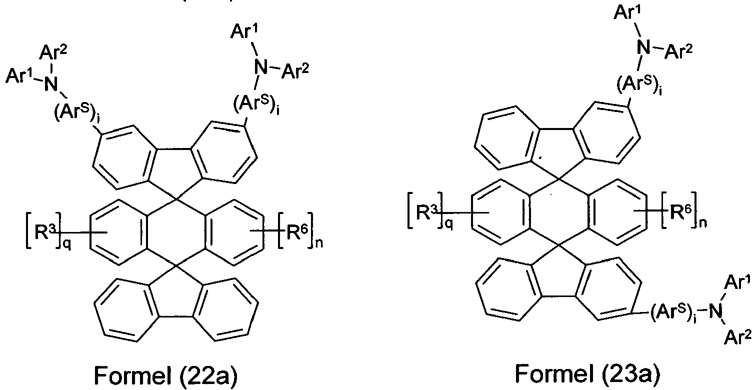 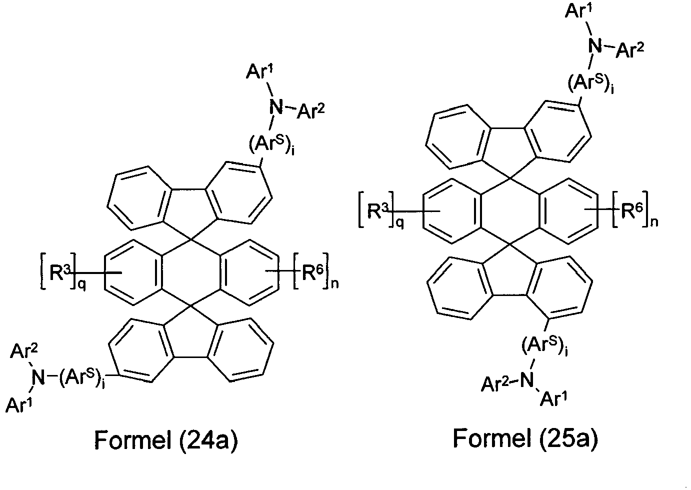 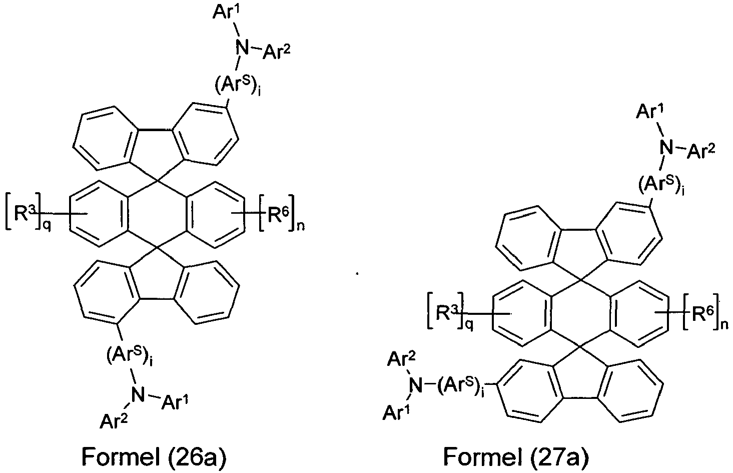 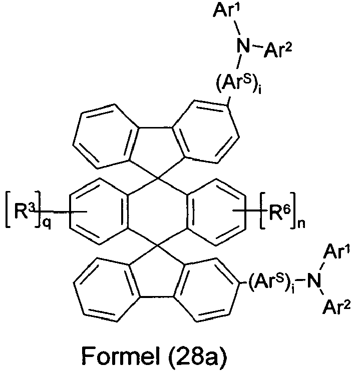 wobei die Symbole und Indizes die gleiche Bedeutung wie für Formel (1) haben.

In einer bevorzugten Ausführungsform der Erfindung ist eine Gruppe -NAr¹Ar², bzw. (Ar^{S})ᵢ-NAr¹Ar² an der Position 1, 3 oder 4 angeordnet und, soweit vorhanden, ist mindestens eine weitere Gruppe -NAr¹Ar², bzw. (Ar^{S})ᵢ-NAr¹Ar² an einer der Positionen 1",2", 4", 5", 7" oder 8" angeordnet. Besonders bevorzugt sind die zwei Gruppen an den Positionen 4 und 5, 4 und 4", 4 und 5", 4 und 2", 4 und 7", 3 und 4", 3 und 5", 3 und 2", 3 und 7", 1 und 4", 1 und 5", 1 und 2", 1 und 7"' angeordnet. Besonders bevorzugt sind Verbindungen mit genau zwei Gruppen -NAr¹Ar², bzw. (Ar^{S})ᵢ-NAr¹Ar². Daher sind Verbindungen der Formeln (6), (7), (8), (9), (10), (11), (13), (14), (15), (19), (20), (21), (25), (26), (27) und (28) bevorzugt, besonders bevorzugt die Verbindungen der Formeln (6a), (7a), (8a), (9a), (10a), (11a), (13a), (14a), (15a), (19a), (20a), (21a), (25a), (26a), (27a) und (28a).

Die erfindungsgemäßen Verbindung kann auch als Gemisch von zwei oder mehreren Substitutionsisomeren der Formel (1), vorliegen. Unter Substitutionsisomeren werden Verbindungen verstanden, welche am Grundgerüst die gleichen Reste tragen, aber diese an anderen Positionen am Grundgerüst angeordnet sind. Bevorzugt handelt es sich dabei um Substitutionsisomere, welche an mindestens einer Fluoreneinheit und/oder der Anthraceneinheit ein zueinander spiegelbildliches Substitutionsmuster aufweisen. Dies bedeutet, dass für diese Verbindungen der Substituent and Position 1' der einen Verbindung dem Substituenten an Position 8' der anderen Verbindung entspricht. Gleiches gilt auch für die jeweiligen Substituenten an den Positionen 2'/7', 3'/6', 4'/5', 5'/4', 6'/3', 2'/7', 1'/8' und/oder 1"/8", 2"/7", 3"/6", 4"/5". 5"/4", 6"/3", 7"/2", 8"/1", wobei die erste Ziffer die Position des Substituenten der einen Verbindung und die zweite Ziffer die Position des Substituenten der anderen Verbindung angibt. Bevorzugt ist das Substitutionsmuster an den Positionen 1, 2, 3, 4, 5, 6, 7 und 8 der Substitionsisomere identisch.

So kann beispielsweise bei Synthese der Verbindung (7), ein entsprechendes Substituionsisomer gemäß der Formel (8) erhalten werden. Wenn die Isomere nicht getrennt werden, liegt die erfindungsgemäße Verbindung als Gemisch von Substituionsisomeren der Formeln (7) und (8) vor.

Bei Anwesenheit von Stereozentren können die Verbindungen auch als Enantionmerengemisch oder als aufgereinigte Enantionmere vorliegen, bzw. als Gemisch von Diastereomeren oder aufgereinigte Diastereomere. Die Verbindung kann auch als Gemisch von mehreren erfindungsgemäßen Verbindungen eingesetzt werden.

In einer weiteren bevorzugten Ausführungsform der Erfindung sind q und n jeweils 0 oder 1. Besonders bevorzugt sind die Reste R³ und R⁶, soweit vorhanden, an den Positionen 2', 3', 6' und/oder 7' angeordnet.

In einer bevorzugten Ausführungsform der Erfindung sind die Gruppen Ar¹ und Ar² bei jedem Auftreten gleich oder verschieden ausgewählt aus den Gruppen mit den folgenden Formeln (Ar-1) bis (Ar-33) 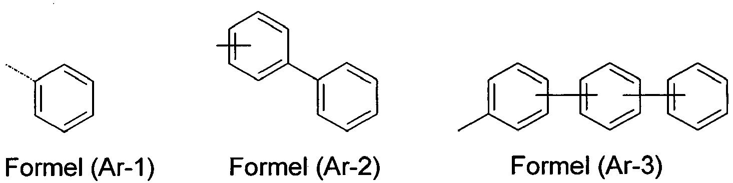 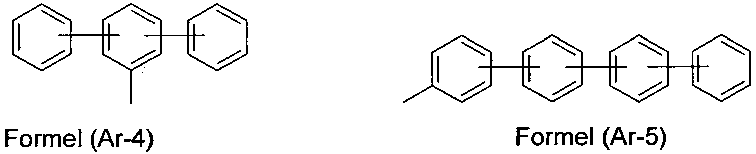 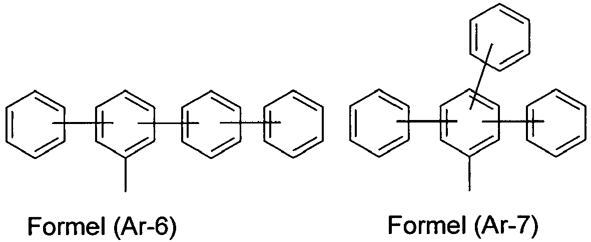 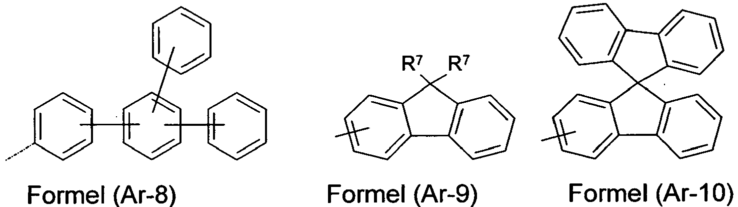 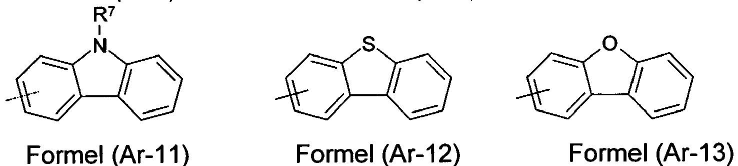 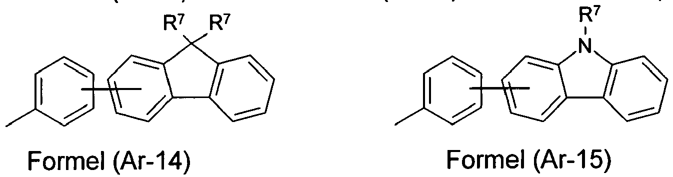 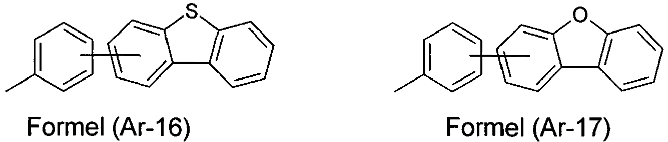 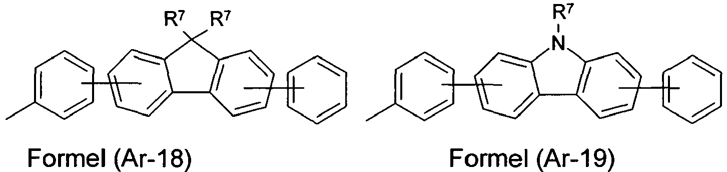 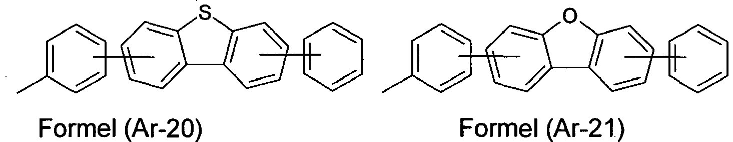 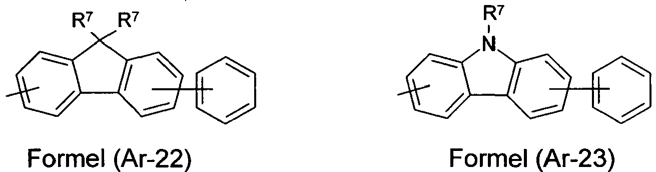 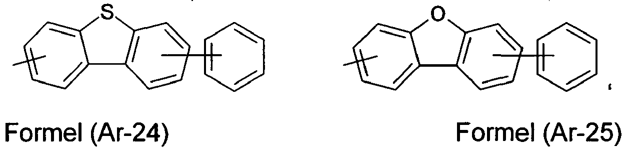 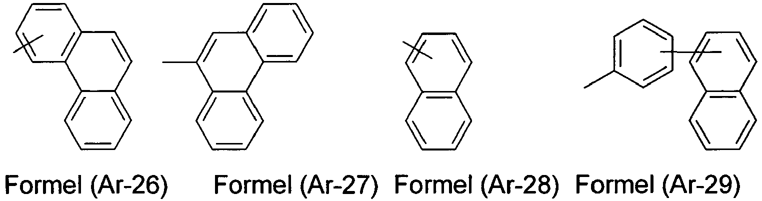 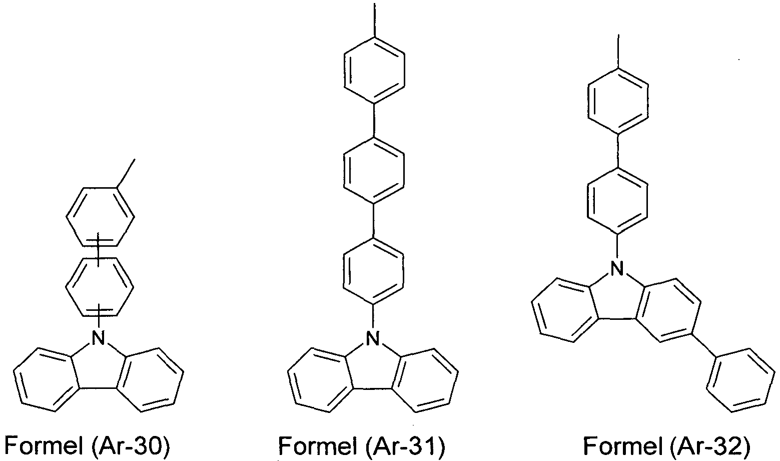 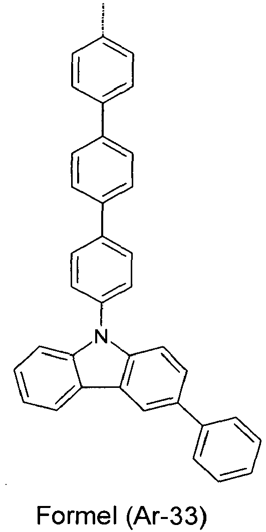 wobei die verwendeten Symbole die oben genannten Bedeutungen haben und die endständige Einfachbindung die Position der Bindung der Gruppe an den Stickstoff andeutet. Die Gruppen können dabei an den freien Positionen mit R⁷ substituiert sein, sind aber bevorzugt unsubstituiert.

In einer besonders bevorzugten Ausführungsform der Erfindung sind die Gruppen Ar¹ und Ar² bei jedem Auftreten gleich oder verschieden ausgewählt aus den Gruppen mit den Strukturen der Formeln (Ar-1) bis (Ar-33), wobei die allgemeinen Formeln durch die bevorzugten Ausführungsformen gemäß der folgenden Formeln (Ar-1a) bis (Ar-33a) ersetzt werden (z. B. Ar-2 wird ersetzt durch (Ar-2a), (Ar-2b), (Ar-2c)): 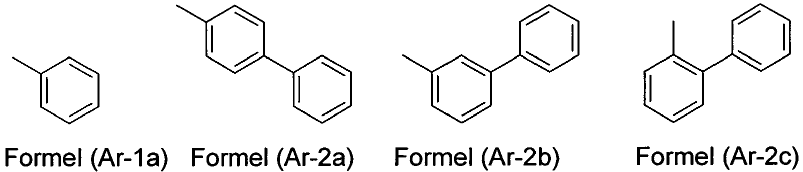 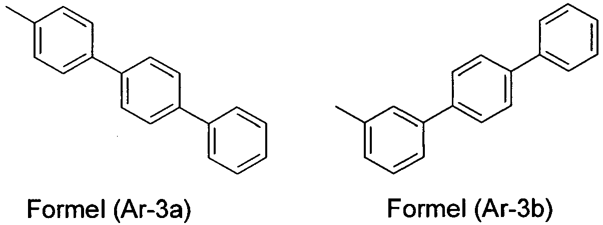 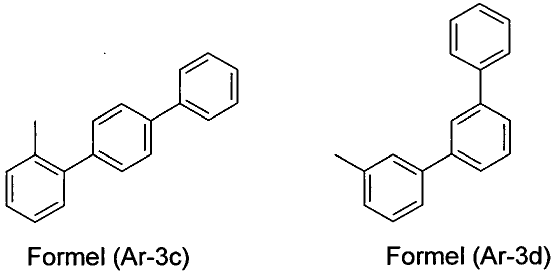 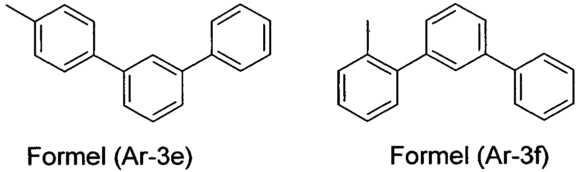 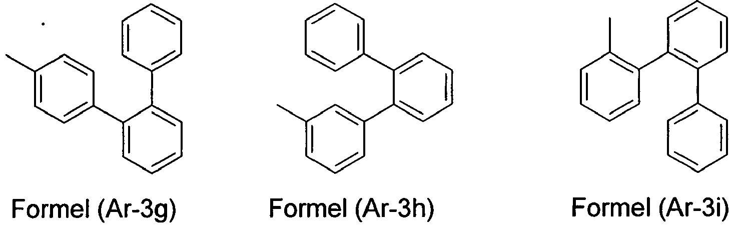 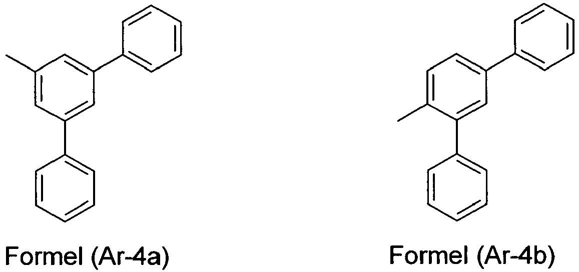 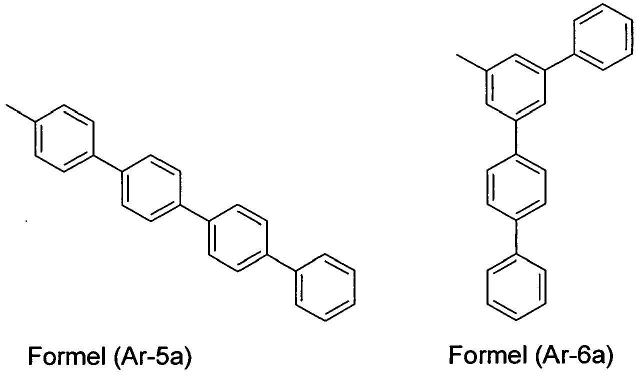 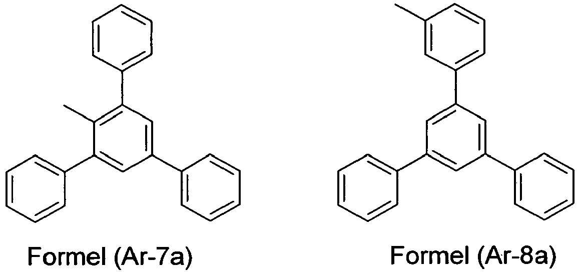 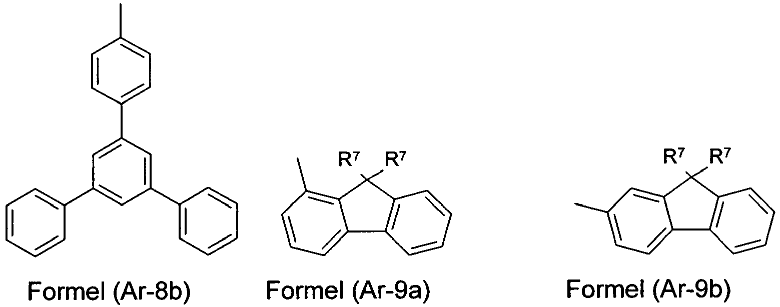 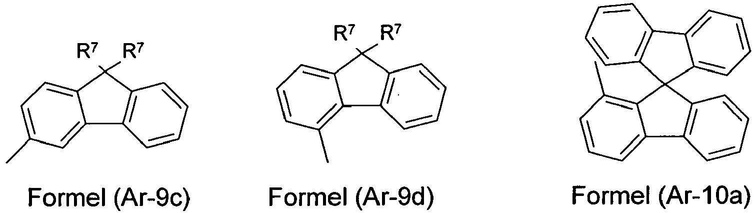 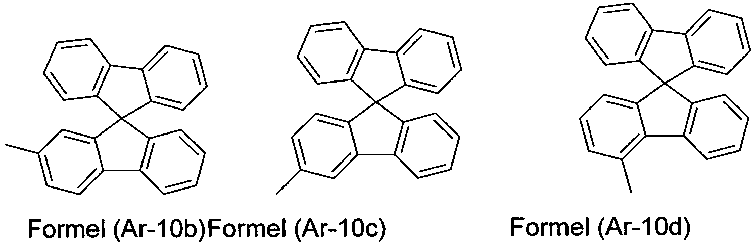 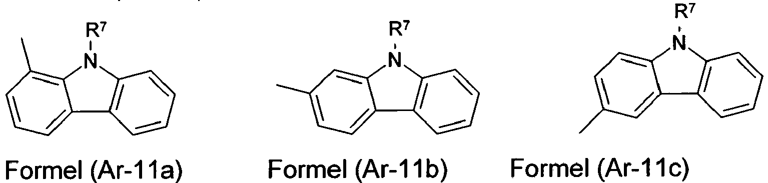 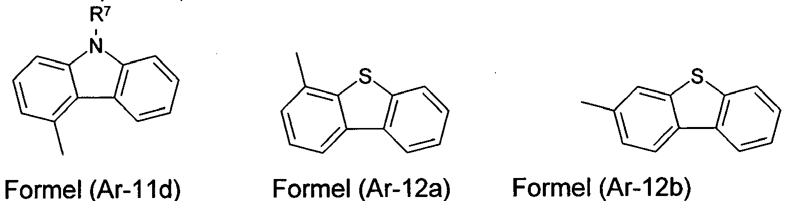 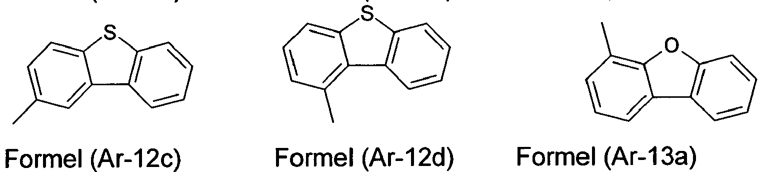 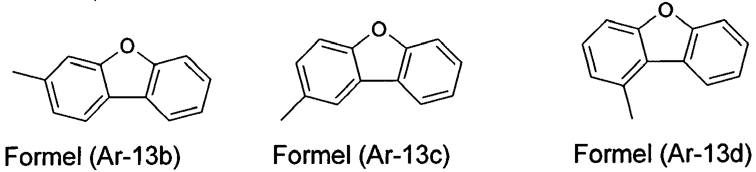 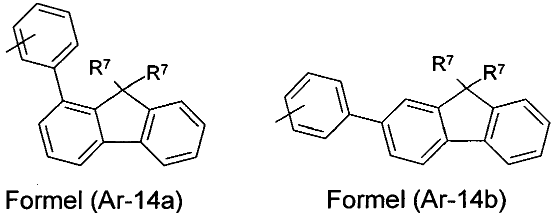 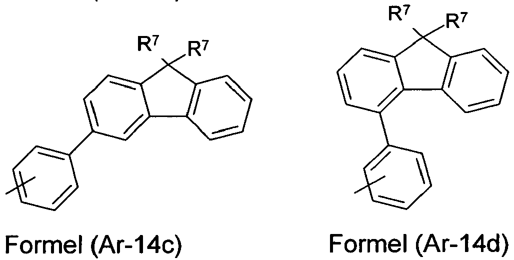 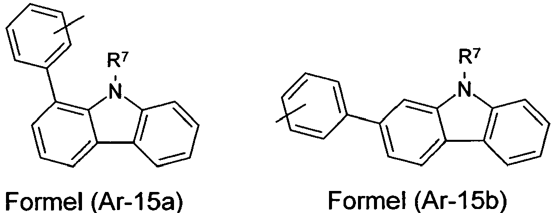 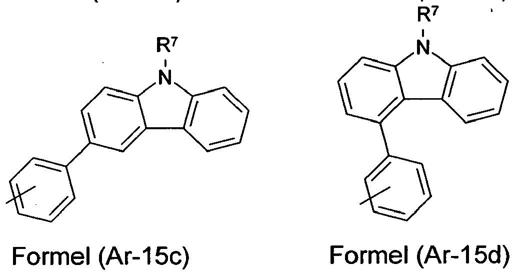 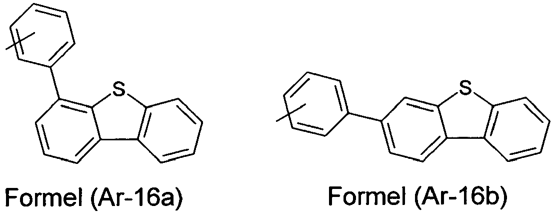 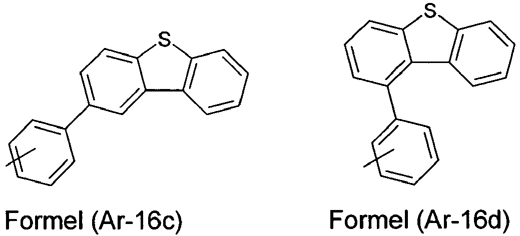 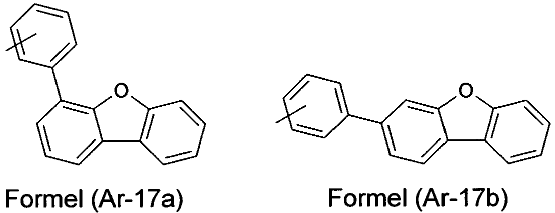 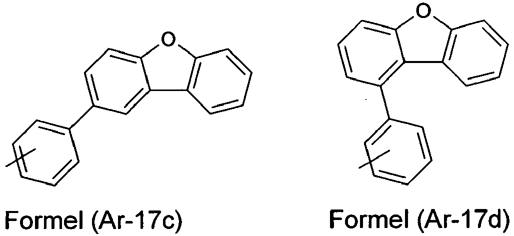 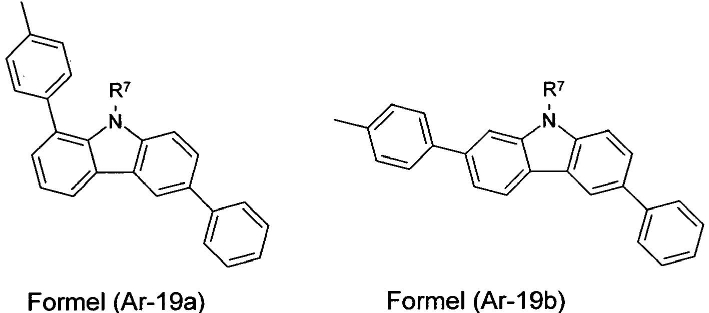 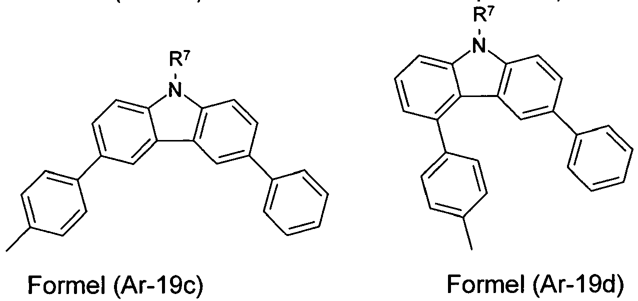 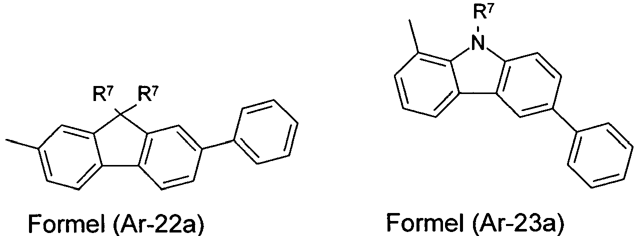 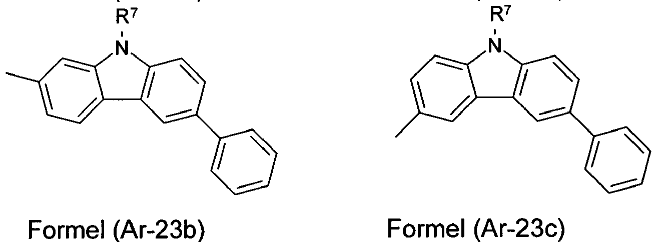 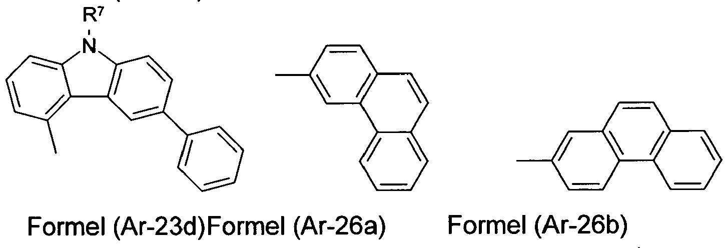 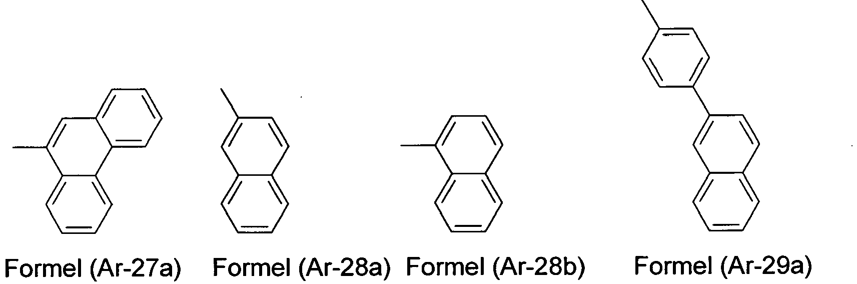 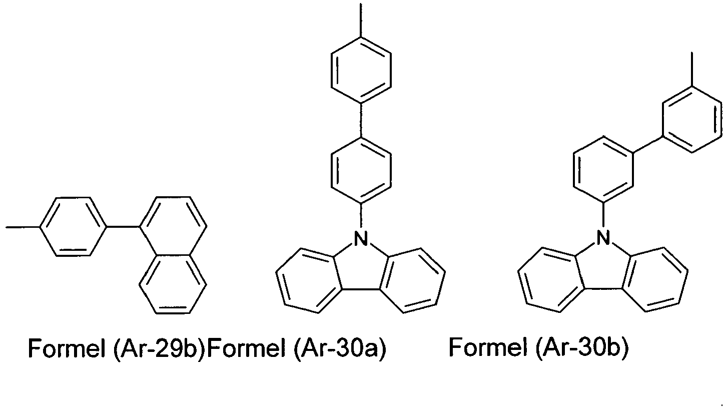 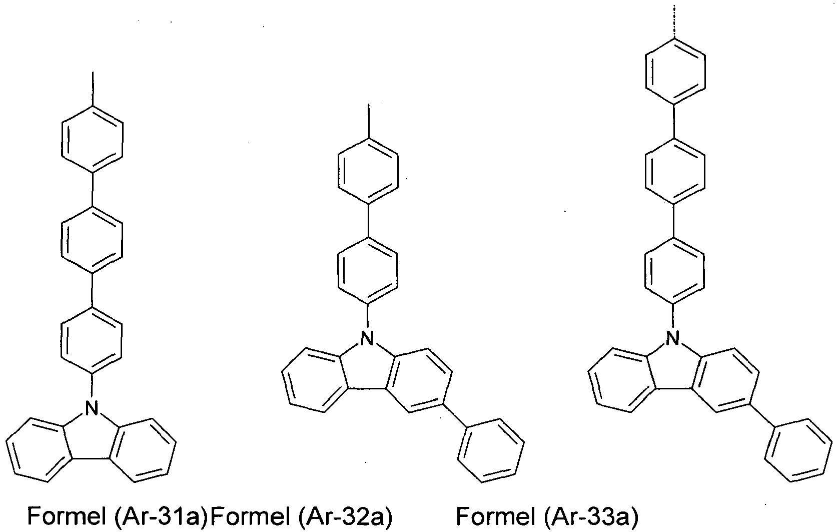 wobei die verwendeten Symbole die oben genannten Bedeutungen haben und die endständige Einfachbindung die Position der Bindung der Gruppe an den Stickstoff andeutet. Die Gruppen können dabei an den freien Positionen mit R⁷ substituiert sein. Sie sind bevorzugt unsubstituiert.

In einer weiteren besonders bevorzugten Ausführungsform der Erfindung sind die Gruppen Ar¹ und Ar² bei jedem Auftreten gleich oder verschieden ausgewählt aus den Gruppen mit den Strukturen der Formeln (Ar-1a) bis (Ar-33a).

Eine besonders bevorzugte Struktur der Gruppe gemäß der Formel (Ar-14a) zeigt die folgende Formel (Ar-14a1). Eine besonders bevorzugte Struktur der Gruppe gemäß der Formel (Ar-14b) zeigt die folgende Formel (Ar-14b1). Eine besonders bevorzugte Struktur der Gruppe gemäß der Formel (Ar-14c) zeigt die folgende Formel (Ar-14c1). Eine besonders bevorzugte Struktur der Gruppe gemäß der Formel (Ar-14d) zeigt die folgende Formel (Ar-14d1). Eine besonders bevorzugte Struktur der Gruppe gemäß der Formel (Ar-15a) zeigt die folgende Formel (Ar-15a1). Eine besonders bevorzugte Struktur der Gruppe gemäß der Formel (Ar-15b) zeigt die folgende Formel (Ar-15b1). Eine besonders bevorzugte Struktur der Gruppe gemäß der Formel (Ar-15c) zeigt die folgende Formel (Ar-15c1). Eine besonders bevorzugte Struktur der Gruppe gemäß der Formel (Ar-15d) zeigt die folgende Formel (Ar-15d1). Eine besonders bevorzugte Struktur der Gruppe gemäß der Formel (Ar-16a) zeigt die folgende Formel (Ar-16a1). Eine besonders bevorzugte Struktur der Gruppe gemäß der Formel (Ar-16b) zeigt die folgende Formel (Ar-16b1). Eine besonders bevorzugte Struktur der Gruppe gemäß der Formel (Ar-16c) zeigt die folgende Formel (Ar-16c1). Eine besonders bevorzugte Struktur der Gruppe gemäß der Formel (Ar-16d) zeigt die folgende Formel (Ar-16d1). Besonders bevorzugte Strukturen der Gruppe gemäß der Formel (Ar-17a) zeigen die folgenden Formeln (Ar-17a1), (Ar-17a2) und (Ar-17a3). Eine besonders bevorzugte Struktur der Gruppe gemäß der Formel (Ar-17b) zeigt die folgende Formel (Ar-17b1). Eine besonders bevorzugte Struktur der Gruppe gemäß der Formel (Ar-17c) zeigen die folgenden Formeln (Ar-17c1) und (Ar-17c2). Eine besonders bevorzugte Struktur der Gruppe gemäß der Formel (Ar-17d) zeigt die folgende Formel (Ar-17d1).

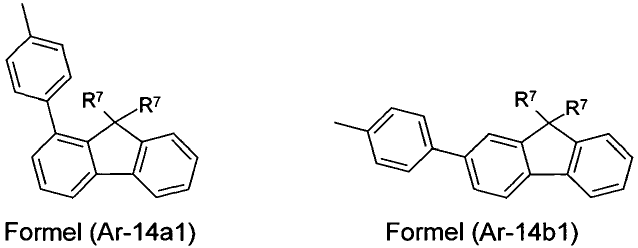

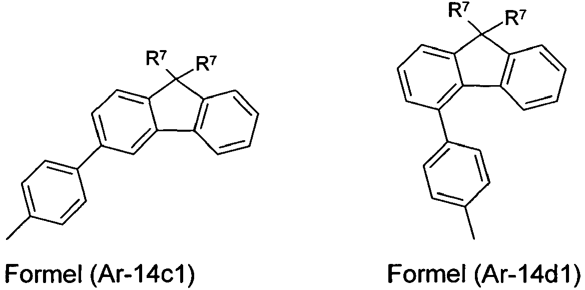

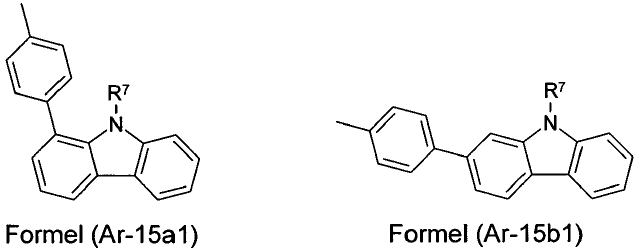

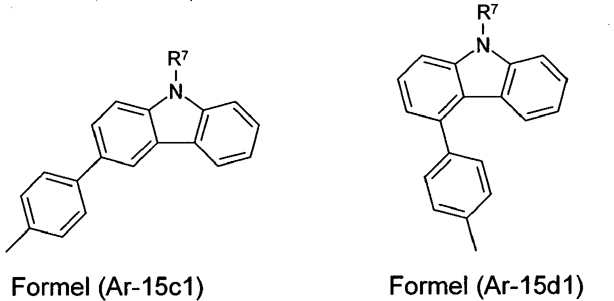

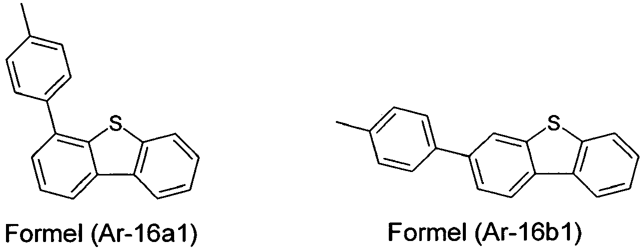

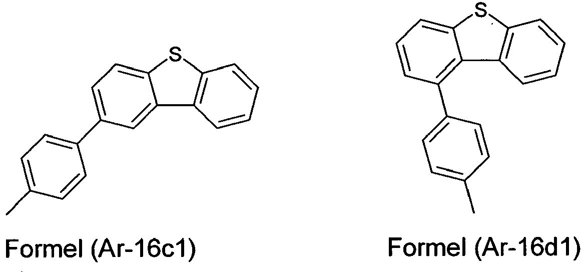

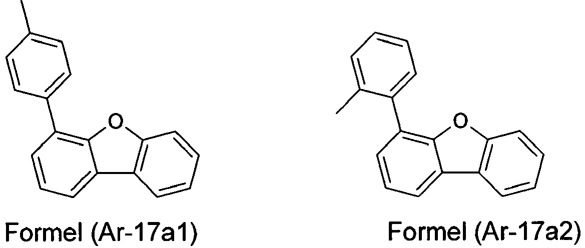

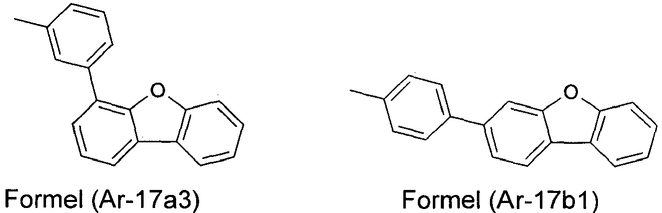

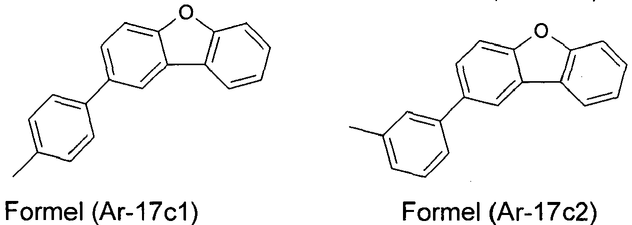

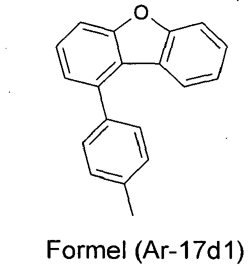

Wobei die verwendeten Symbole die oben genannten Bedeutungen haben und die endständige Einfachbindung die Position der Bindung der Gruppe an den Stickstoff andeutet. Die Gruppen können dabei an den freien Positionen mit R⁷ substituiert sein. Sie sind bevorzugt unsubstituiert.

Dabei können die beiden Gruppen Ar¹ und Ar² der oben genannten Formeln (Ar-1) bis (Ar-33) beliebig miteinander kombiniert werden. Die Gruppen der Formeln (Ar-1), (Ar-2), (Ar-3), (Ar-4), (Ar-8), (Ar-9), (Ar-10), (Ar-11) (Ar-12), (Ar-13), (Ar-14), (Ar-15), (Ar-16), (Ar-17) und (Ar-29) sind dabei besonders bevorzugt.

Ganz besonders bevorzugte Gruppen -NAr¹Ar² sind daher Gruppen, die die folgende Kombination für Ar¹ und Ar² aufweisen:

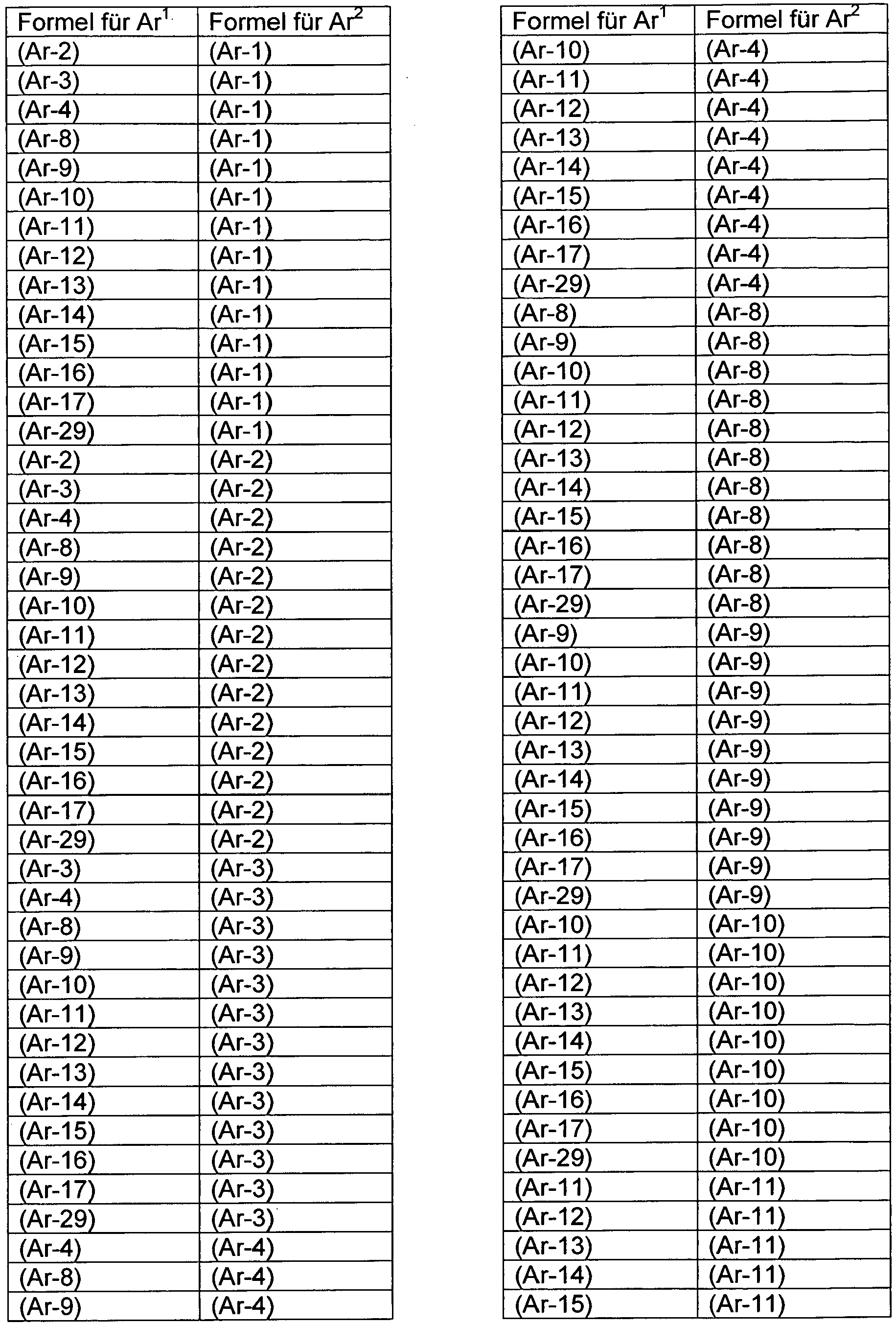

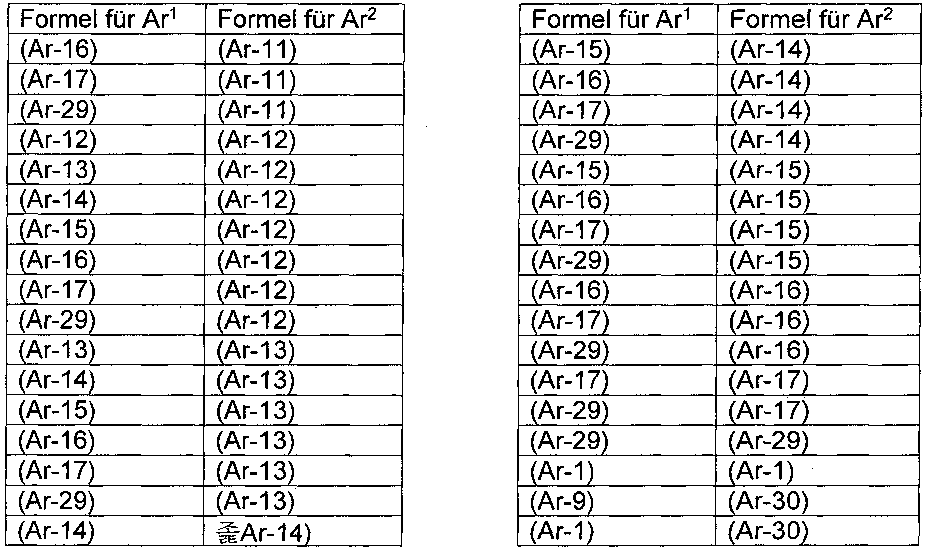

Ganz bevorzugte Gruppen -NAr¹Ar² sind Gruppen, die die Kombinationen aus der oben genannten Tabelle aufweisen, wobei statt Formel (Ar-1), (Ar-2), (Ar-3), (Ar-4), (Ar-8), (Ar-9), (Ar-10), (Ar-11) (Ar-12), (Ar-13), (Ar-14), (Ar-15), (Ar-16),(Ar-17) und (Ar-29), jeweils die bevorzugten Formeln der jeweiligen Formeln, z. B. (Ar-2a), (Ar-2b) oder (Ar-2c) statt (Ar-2), eingesetzt werden.

Wenn in den Verbindungen den Formeln (1) bis (28) oder (6a) bis (28a) die Gruppen Ar¹ und Ar² miteinander verknüpft sind, dann hat die Gruppe - NAr¹Ar² bevorzugt eine Struktur gemäß einer der folgenden Formeln (Ar1-1) bis (Ar1-19) 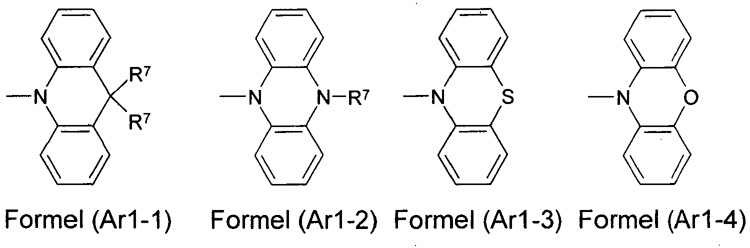 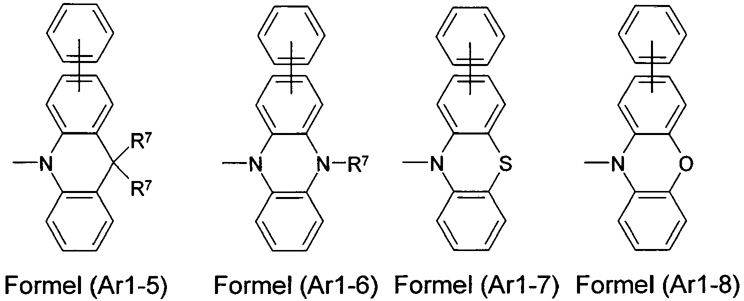 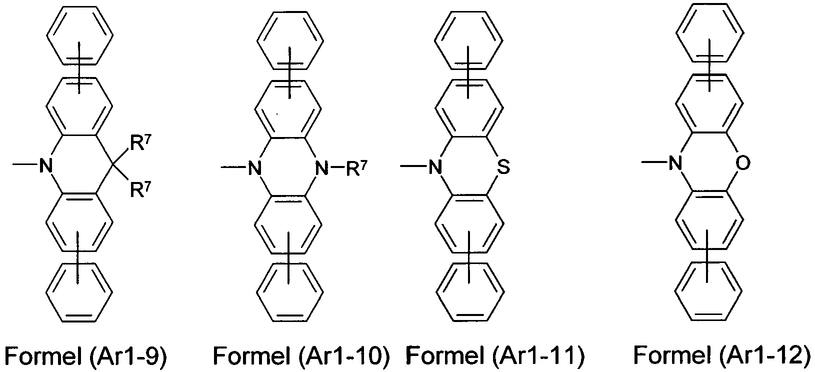 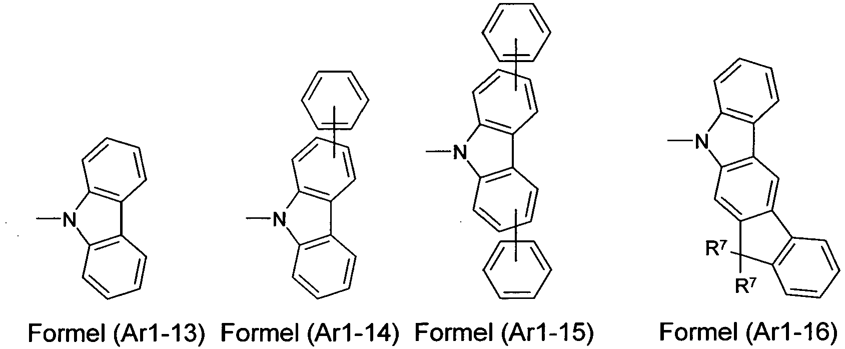 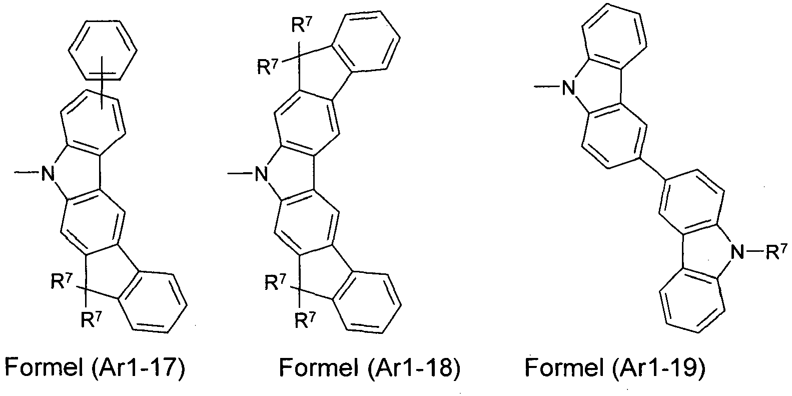 wobei die verwendeten Symbole die oben genannten Bedeutungen haben und die endständige Einfachbindung die Position der Bindung an das Grundgerüst, bzw. an Ar^{S} andeutet. Die Gruppen können dabei an den freien Positionen mit R⁷ substituiert sein.

In einer besonders bevorzugten Ausführungsform der Erfindung sind die Verbindungen der Formeln (Ar1-5), (Ar1-6), (Ar1-7), (Ar1-8), (Ar1-9), (Ar1-10), (Ar1-11), (Ar1-12), (Ar1-14), (Ar1-15) und (Ar1-17) ausgewählt aus den Verbindungen mit den folgenden Formeln 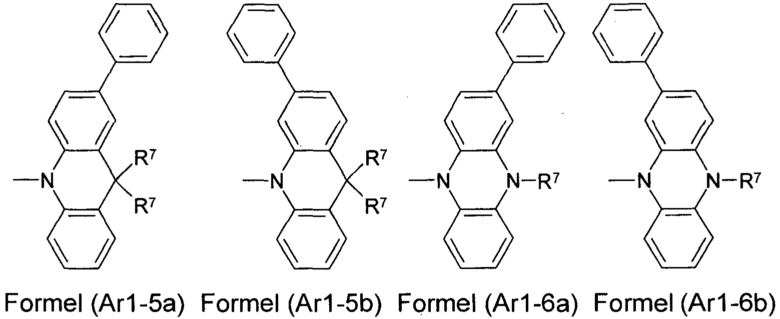 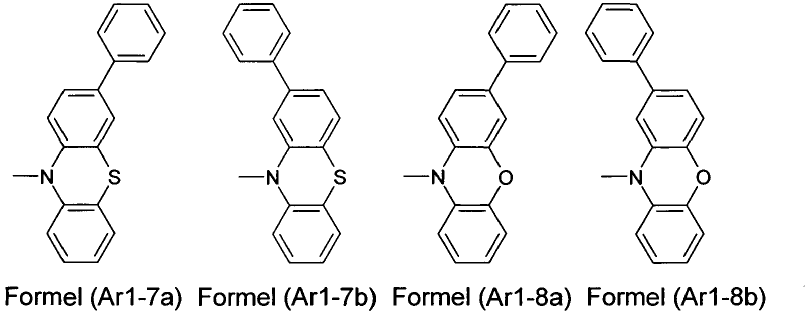 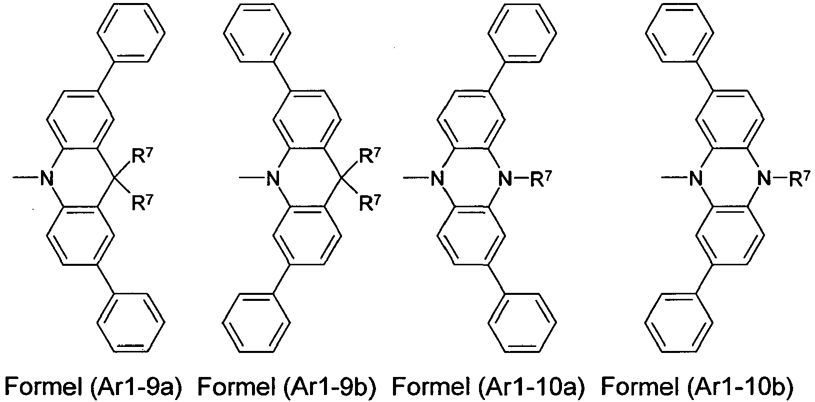 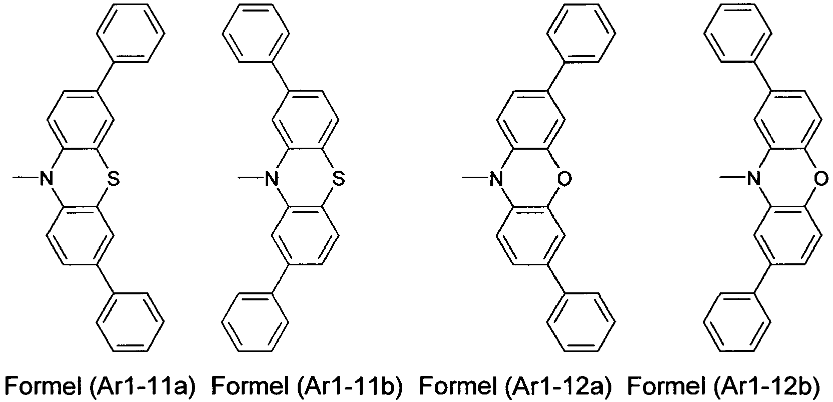 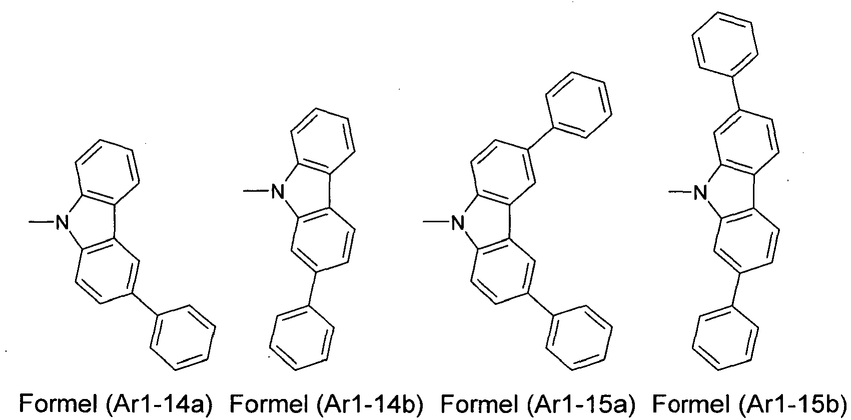 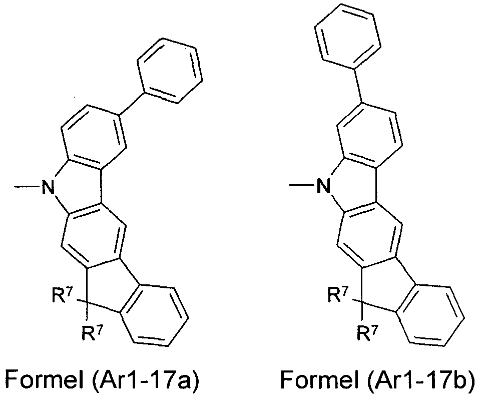 wobei die verwendeten Symbole die oben genannten Bedeutungen haben und die endständige Einfachbindung die Position der Bindung an das Grundgerüst, bzw. an Ar^{S} andeutet. Die Gruppen können dabei an den freien Positionen mit R⁷ substituiert sein. Bevorzugt sind sie unsubstituiert.

Wenn in den Verbindungen der Formeln (1) bis (28) oder (6a) bis (28a) eine Gruppe Ar^{S} mit Ar¹ miteinander verknüpft ist, dann hat die Gruppe - Ar^{S}-NAr¹Ar² bevorzugt die Struktur einer der folgenden Formeln (Ar2-1) bis (Ar2-9). Analoges gilt für eine Verknüpfung der Gruppe Ar^{S} mit Ar². 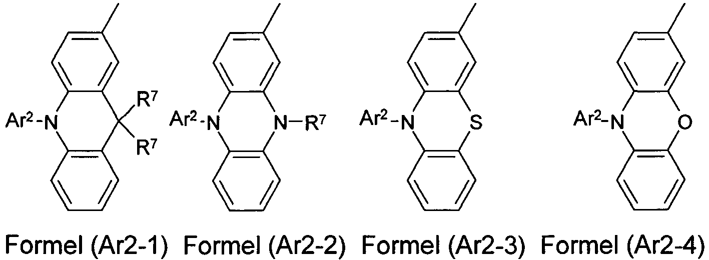 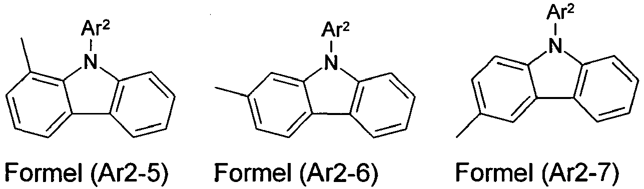 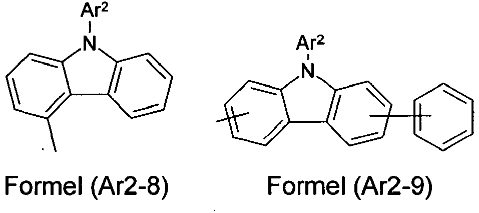 wobei die verwendeten Symbole die oben genannten Bedeutungen aufweisen. Die Gruppen können dabei an den freien Positionen mit R⁷ substituiert sein. Bevorzugt sind sie unsubstituiert.

Bevorzugte Ausführungsformen der Formeln (Ar2-9) zeigen die folgenden Formeln: 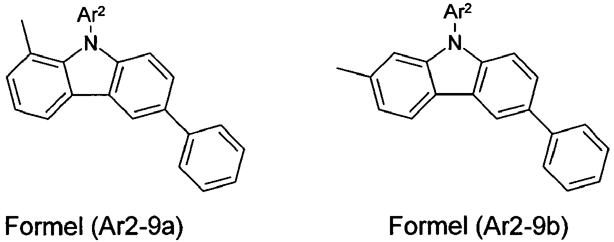 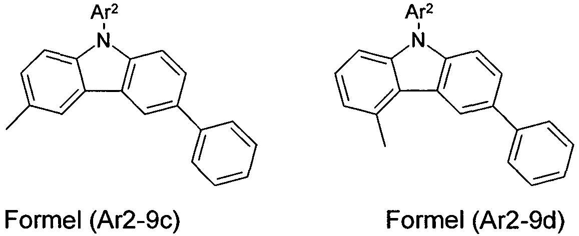 wobei die verwendeten Symbole die oben genannten Bedeutungen aufweisen. Die Gruppen können dabei an den freien Positionen mit R⁷ substituiert sein. Bevorzugt sind sie unsubstituiert.

Die Gruppe Ar^{S} ist bevorzugt gleich oder verschieden bei jedem Auftreten ein aromatisches oder heteroaromatisches Ringsystem mit 6 bis 18 Ringatomen, welches jeweils mit einem oder mehreren Resten R⁹ substituiert sein kann.

In einer weiteren bevorzugten Ausführungsform der Erfindung ist der Index i gleich 1 oder 2 und die Gruppe Ar^{S} steht für eine Gruppe gemäß einer der folgenden Formeln (Ar3-1) bis (Ar3-12). 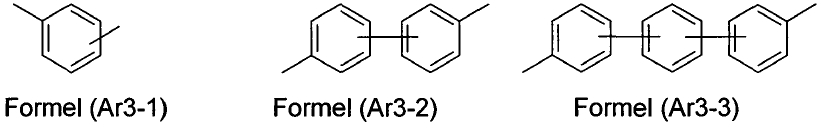 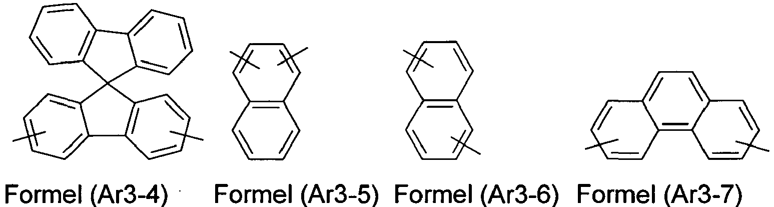 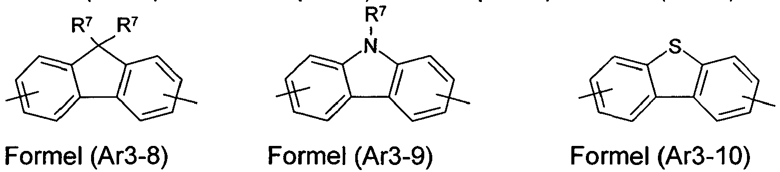 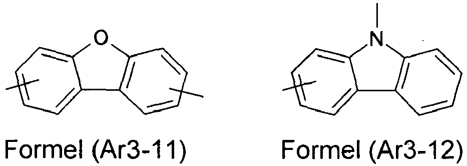 wobei die verwendeten Symbole die genannten Bedeutungen aufweisen und die beiden endständigen Einfachbindungen die Bindungen an die benachbarten Gruppen darstellen. Die Gruppen können dabei an den freien Positionen mit R⁷ substituiert sein. Bevorzugt sind sie unsubstituiert.

Bevorzugte Ausführungsformen der Formeln zeigen die folgenden Formeln (Ar3-1a) bis (Ar3-12a): 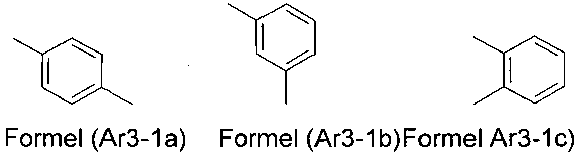 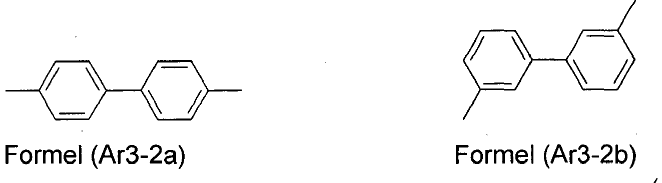 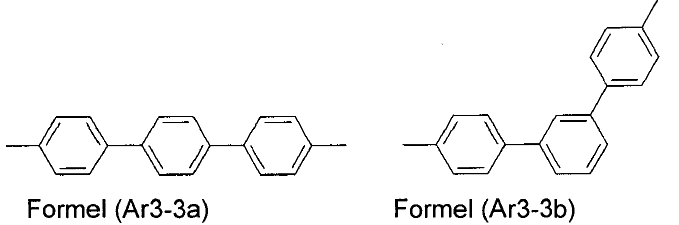 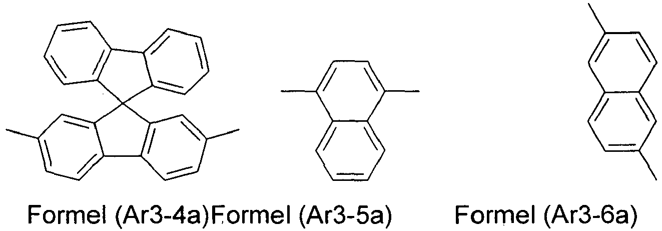 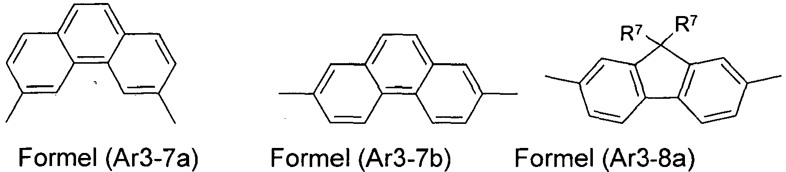 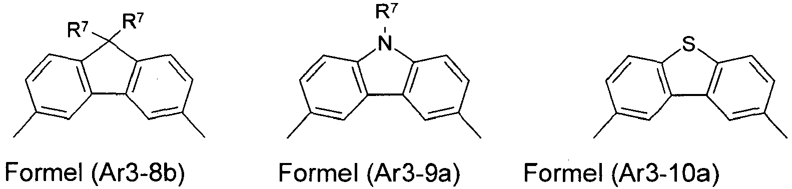 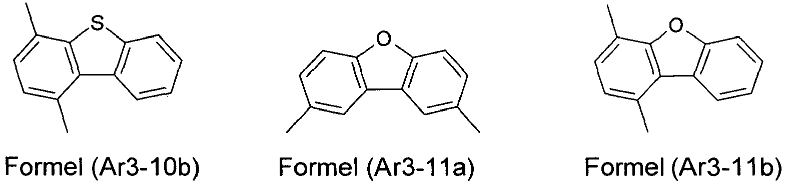 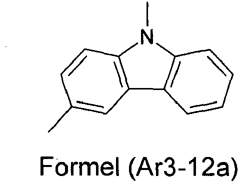 wobei die verwendeten Symbole die genannten Bedeutungen aufweisen und die beiden endständigen Einfachbindungen die Bindungen an die benachbarten Gruppen darstellen. Die Gruppen können dabei an den freien Positionen mit R⁷ substituiert sein. Bevorzugt sind sie unsubstituiert.

Bevorzugt ist für jede Gruppe (Ar^{S})ᵢ maximal ein Ar^{S} eine Gruppe der Formel (Ar3-9), (Ar3-12), (Ar3-9a) oder (Ar3-12a).

Gemäß eine bevorzugten Ausführungsform ist R⁷ bei jedem Auftreten gleich oder verschieden ausgewählt aus der Gruppe bestehend aus H, D, F, Cl, Br, I, CN, Si(R⁹)₃, einer geradkettigen Alkyl-, Alkoxy- oder Thioalkylgruppe mit 1 bis 40 C- Atomen oder einer verzweigten oder cyclischen Alkyl-, Alkoxy- oder Thioalkylgruppe mit 3 bis 40 C-Atomen, die jeweils mit einem oder mehreren Resten R⁹ substituiert sein kann, wobei jeweils eine oder mehrere nicht-benachbarte CH₂-Gruppen durch Si(R⁹)₂, C=NR⁹, P(=O)(R⁹), SO, SO₂, NR⁹, O, S oder CONR⁹ ersetzt sein können und wobei ein oder mehrere H-Atome durch D, F, Cl, Br oder I ersetzt sein können, einem aromatischen oder heteroaromatischen Ringsystem mit 6 bis 60 aromatischen Ringatomen, das jeweils mit einem oder mehreren Resten R⁹ substituiert sein kann, einer Aryloxygruppe oder Heteroaryloxygruppe mit 5 bis 60 aromatischen Ringatomen, die mit einem oder mehreren Resten R⁹ substituiert sein kann, oder einer Aralkylgruppe oder Heteroaralkylgruppe mit 5 bis 60 aromatischen Ringatomen, die jeweils mit einem oder mehreren Resten R⁹ substituiert sein kann, wobei optional zwei oder mehr benachbarte Substituenten R⁷ ein mono- oder polycyclisches, aliphatisches oder aromatisches Ringsystem bilden können, das mit einem oder mehreren Resten R⁹ substituiert sein kann;

Gemäß der vorstehenden Definition von R⁷ enthält eine Verbindung der Formel (1) keine weiteren Arylaminogruppen außer den in der Verbindung explizit angegebenen.

In einer bevorzugten Ausführungsform der Erfindung sind R¹, R², R³, R⁴, R⁵, R⁶, R⁷. R⁸ in den Verbindungen der Formel (1) bis (28), bzw. (6a) bis (28a) gleich oder verschieden bei jedem Auftreten ausgewählt aus der Gruppe bestehend aus H, D, F, Si(R⁹)₃, CN, einer geradkettigen Alkyl-oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R⁹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 aromatischen Ringatomen, das jeweils mit einem oder mehreren Resten R⁹ substituiert sein kann, wobei optional zwei oder mehr benachbarte Substituenten R¹ bis R⁷, bzw.

R¹ und R⁸, ein mono- oder polycyclisches, aliphatisches Ringsystem bilden können, das mit einem oder mehreren Resten R⁹ substituiert sein kann.

In einer besonders bevorzugten Ausführungsform der Erfindung sind R¹, R², R³, R⁴, R⁵, R⁶, R⁷ ,R⁸ in den Verbindungen der Formel (1) bis (28), bzw. (6a) bis (28a) gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen, die jeweils mit einem oder mehreren Resten R⁹ substituiert sein kann, einem aromatischen Ringsystem mit 6 bis 60 C-Atomen, das jeweils mit einem oder mehreren Resten R⁹ substituiert sein kann, wobei optional zwei oder mehr benachbarte Substituenten R¹ bis R⁷, bzw. R¹ und R⁸, ein mono- oder polycyclisches, aliphatisches Ringsystem bilden können, das mit einem oder mehreren Resten R⁹ substituiert sein kann. In einer ganz besonders bevorzugten Ausführungsform der Erfindung sind R¹ bis R⁶ in den Verbindungen der Formel (1) bis (28), bzw. (6a) bis (28a) gleich H.

In einer weiteren bevorzugten Ausführungsform der Erfindung ist der Rest R⁷, welcher an Ar¹ bzw. Ar² bzw. A^{S} bindet, gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen.

In einer weiteren bevorzugten Ausführungsform der Erfindung ist mindestens ein Rest R⁷, welcher in ortho-Position der Arylgruppe von Ar¹ bzw. Ar² bindet, welche direkt an den Stickstoff gebunden ist, ungleich Wasserstoff oder Deuterium. Dies gilt insbesondere dann, wenn an der Arylgruppe nicht bereits eine weitere Arylgruppe in ortho-Position gebunden ist, wie dies beispielsweise in Formel (Ar-2c) der Fall ist.

Weiterhin kann es bevorzugt sein, wenn die beiden Substituenten R⁷ in 9-Position eines Fluorens zusammen einen Cycloalkylring bilden, bevorzugt mit 3 bis 8 C-Atomen, besonders bevorzugt mit 5 oder 6 C-Atomen.

In einer weiteren bevorzugten Ausführungsform ist R⁷, welcher in Formel (Ar1-1), (Ar1-5), (Ar1-9), (Ar1-16), (Ar1-17), (Ar1-5a), (Ar1-5b), (Ar1-9a), (Ar1-9b), (Ar1-17a), (Ar1-17b), (Ar2-1) (Ar3-8), (Ar3-8a), (Ar3-8b) an die Kohlenstoffbrücke bindet, gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das wie oben definiert ist und das mit einem oder mehreren Resten R⁹ substituiert sein kann. Dabei können die beiden Gruppen R⁷ auch miteinander ein Ringsystem bilden, welches aliphatisch oder zusätzlich zur oben gegebenen Definition von R⁷ auch aromatisch sein kann. Durch Ringbildung wird ein Spiro-System aufgespannt.

In einer weiteren bevorzugten Ausführungsform ist R⁷, welcher in Formel (Ar1-2), (Ar1-6), (Ar1-10), (Ar1-19), (Ar1-6a), (Ar1-6b), (Ar1-10a), (Ar1-10b), (Ar2-2), (Ar3-9), (Ar3-9a) an die Stickstoffbrücke bindet, ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, insbesondere ein aromatisches Ringsystem mit 6 bis 24 C-Atomen, das wie oben definiert ist, und das mit einem oder mehreren Resten R⁹ substituiert sein kann.

In einer weiteren bevorzugten Ausführungsform sind R³ und R⁶, wenn sie ein aromatisches oder heteroaromatisches Ringsystem umfassen, ausgewählt aus den Gruppen der Formeln (Ar-1) bis (Ar-33), besonders bevorzugt (Ar-1) bis (Ar-10), sowie den bevorzugten Ausführungsformen dieser Gruppen. Die Reste R³ und R⁶ umfassen bevorzugt kein heteroaromatisches Ringsystem.

Dabei haben für Verbindungen, die durch Vakuumverdampfung verarbeitet werden, die Alkylgruppen bevorzugt nicht mehr als vier C-Atome, besonders bevorzugt nicht mehr als 1 C-Atom. Für Verbindungen, die aus Lösung verarbeitet werden, eignen sich auch Verbindungen, die mit linearen, verzweigten oder cyclischen Alkylgruppen mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

In einer bevorzugten Ausführungsform der Erfindung ist R⁹ gleich oder verschieden bei jedem Auftreten ausgewählt aus der Gruppe bestehend aus H, D, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 C-Atomen, das wie oben definiert ist und das jeweils mit einem oder mehreren Resten R¹⁰ substituiert sein kann.

In einer besonders bevorzugten Ausführungsform der Erfindung ist R⁹ gleich oder verschieden bei jedem Auftreten ausgewählt aus der Gruppe bestehend aus H, D, einer geradkettigen Alkylgruppe mit 1 bis 5 C-Atomen oder einer verzweigten oder cyclischen Alkylgruppe mit 3 bis 5 C-Atomen, oder einem aromatischen Ringsystem mit 6 bis 18 C-Atomen, das wie oben definiert ist.

In einer weiteren bevorzugten Ausführungsform der Erfindung umfassen die Reste R¹ bis R⁶ keine anelierten Aryl- oder Heteroarylgruppen, welche mehr als 12 aromatische Ringatome umfassen.

In einer weiteren bevorzugten Ausführungsform der Erfindung bilden zwei oder mehr benachbarte Reste R¹ bis R⁶, sowie benachbarte Reste R¹ und R⁸, kein mono- oder polycyclisches aromatisches Ringsystem.

Bevorzugt sind Verbindungen der Formel (1), (2) bis (28) und (6a) bis (28a), in denen die oben genannten bevorzugten Ausführungsformen gleichzeitig auftreten. Besonders bevorzugt sind daher Verbindungen für die gilt:
- Ar^{S}: ist gleich oder verschieden bei jedem Auftreten ein aromatisches oder heteroaromatisches Ringsystem, wobei für i=1 oder 2 Ar^{S} ausgewählt ist aus den Gruppen der Formel (Ar3-1) bis (Ar3-12), sowie deren bevorzugten Ausführungsformen Formel (Ar3-1a) bis (Ar3-12a), wobei Ar^{S} mit Ar¹ durch eine Gruppe E verbunden sein kann;
- Ar¹, Ar²: sind gleich oder verschieden bei jedem Auftreten ein aromatisches oder heteroaromatisches Ringsystem ausgewählt aus den Gruppen der Formel (Ar-1) bis (Ar-33), sowie deren bevorzugten Ausführungsformen;
oder -NAr¹Ar²- steht für eine Gruppe gemäß einer der Formeln (Ar1-1) bis (Ar1-19), sowie deren bevorzugten Ausführungsformen;
oder Ar^{S}-NAr¹-Ar² steht für eine Gruppe gemäß einer der Formeln (Ar2-1) bis (Ar2-9) sowie deren bevorzugten Ausführungsformen;
- E: ist gleich oder verschieden bei jedem Auftreten ausgewählt aus der Gruppe bestehend aus C(R⁷)₂, O, S und NR⁷;
- R¹, R², R³, R⁴, R⁵, R⁶, R⁸: sind bei jedem Auftreten gleich oder verschieden ausgewählt aus der Gruppe bestehend aus H, D, F, Si(R⁹)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R⁹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 aromatischen Ringatomen, das jeweils mit einem oder mehreren Resten R⁹ substituiert sein kann, wobei optional zwei oder mehr benachbarte Substituenten R¹ bis R⁶, bzw. R¹ und R⁸, ein mono- oder polycyclisches, aliphatisches Ringsystem bilden können, das mit einem oder mehreren Resten R⁹ substituiert sein kann;
- R⁷: ist bei jedem Auftreten gleich oder verschieden ausgewählt aus der Gruppe bestehend aus H, D, F, Si(R⁹)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R⁹ substituiert sein kann, wobei eine oder mehrere nichtbenachbarte CH₂-Gruppen durch O ersetzt sein können und wobei ein oder mehrere H-Atome durch D oder F ersetzt sein können, einem aromatischen Ringsystem mit 6 bis 60 aromatischen Ringatomen, das jeweils mit einem oder mehreren Resten R⁹ substituiert sein kann, wobei optional zwei oder mehr benachbarte Substituenten R⁷ ein mono- oder polycyclisches, aliphatisches Ringsystem bilden können, das mit einem oder mehreren Resten R⁹ substituiert sein kann;
oder R⁷, welcher in Formel (Ar1-1), (Ar1-5), (Ar1-9), (Ar1-16), (Ar1-17), (Ar1-5a), (Ar1-5b), (Ar1-9a), (Ar1-9b), (Ar1-17a), (Ar1-17b), (Ar2-1) (Ar3-8), (Ar3-8a), (Ar3-8b) an die Kohlenstoffbrücke bindet, ist gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R⁹ substituiert sein kann; dabei können die beiden Gruppen R⁷ auch miteinander ein Ringsystem bilden, welches aliphatisch oder aromatisch sein kann;
oder R⁷, welcher in Formel (Ar1-2), (Ar1-6), (Ar1-10), (Ar1-19), (Ar1-6a), (Ar1-6b), (Ar1-10a), (Ar1-10b), (Ar2-2), (Ar3-9), (Ar3-9a) an die Stickstoffbrücke bindet, ist ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R⁹ substituiert sein kann;
- R⁹: ist gleich oder verschieden bei jedem Auftreten ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 C-Atomen, das jeweils mit einem oder mehreren Resten R¹⁰ substituiert sein kann, wobei zwei oder mehr benachbarte Substituenten R⁹ miteinander ein mono- oder polycyclisches, aliphatisches, Ringsystem bilden können;
- R¹⁰: ist ausgewählt aus der Gruppe bestehend aus H, D, F, einem aliphatischem Kohlenwasserstoffrest mit 1 bis 20 C-Atomen, einem aromatischem Ringsystem mit 6 bis 30 C-Atomen, in dem ein oder mehrere H-Atome durch D oder F ersetzt sein können, wobei zwei oder mehr benachbarte Substituenten R¹⁰ miteinander ein mono- oder polycyclisches, aliphatisches, Ringsystem bilden können;
- i: ist bei jedem Auftreten 0, 1 oder 2;
- m: ist 0 oder 1; bevorzugt 0.
- o, p, r: sind bei jedem Auftreten gleich oder verschieden 0, 1 oder 2, bevorzugt 0;
- n, q: sind bei jedem Auftreten gleich oder verschieden 0, 1 oder 2, bevorzugt 0 oder 1;
- s, t, u: sind bei jedem Auftreten gleich oder verschieden 0 oder 1;
dabei gilt u+t+s ≤ 2.

Die vorgenannte Verbindung kann auch als Mischung von zwei oder mehreren Substitutionsisomeren vorliegen.

In einer Ausführungsform der Erfindung sind die oben genannten Bevorzugungen beliebig miteinander kombinierbar.

Beispiele für bevorzugte Strukturen der Verbindung gemäß Formel (1) sind in der folgenden Tabelle angegeben. Die Verbindungen basieren auf den Stukturen für das Grundgerüst (6a), (7a), (8a), (10a), (15a), (21a). Verbundene Zellen bedeuten, dass die Reste über eine Gruppe E miteinander verbunden sind.

| Struktur | Grundgerüst | i(Ar^{S}) | Ar¹ | Ar² |
|---|---|---|---|---|
| 6a-1-1 | 6a | 0 | Ar-9b | Ar-2a |
| 6a-1-2 | 6a | 0 | Ar-29b | Ar-2a |
| 6a-1-3 | 6a | 0 | Ar-1a | Ar-2a |
| 6a-1-4 | 6a | 0 | Ar-2a | Ar-2a |
| 6a-1-5 | 6a | 0 | Ar-17a1 | Ar-2a |
| 6a-1-6 | 6a | 0 | Ar-12a | Ar-2a |
| 6a-1-7 | 6a | 0 | Ar-13c | Ar-2a |
| 6a-1-8 | 6a | 0 | Ar-2c | Ar-9b |
| 6a-1-9 | 6a | 0 | Ar-9b | Ar-9b |
| 6a-1-10 | 6a | 0 | Ar1-5a | |
| 6a-1-11 | 6a | 0 | Ar-2a | Ar-2a |
| 6a-1-12 | 6a | 0 | Ar-2c | Ar-9b |
| 6a-1-13 | 6a | 0 | Ar-2a | Ar-1a |
| 6a-1-14 | 6a | 0 | Ar1-14a | |
| 6a-1-15 | 6a | 0 | Ar1-15a | |
| 6a-1-16 | 6a | 0 | Ar1-15b | |
| 6a-1-17 | 6a | 0 | Ar1-16 | |
| 6a-1-18 | 6a | 0 | Ar1-17a | |
| 6a-1-19 | 6a | 0 | Ar1-18 | |
| 6a-1-20 | 6a | 0 | Ar-8b | Ar-9b |
| 6a-1-21 | 6a | 0 | Ar-8a | Ar-9b |
| 6a-1-22 | 6a | 0 | Ar-3e | Ar-9b |
| 6a-1-23 | 6a | 0 | Ar-2a | Ar-2a |
| 6a-1-24 | 6a | 0 | Ar-2a | Ar-3g |
| 6a-1-25 | 6a | 0 | Ar-2a | Ar-15c1 |
| 6a-1-26 | 6a | 0 | Ar-9b | Ar-30a |
| 6a-1-27 | 6a | 0 | Ar-28b | Ar-2a |
| 6a-1-28 | 6a | 0 | Ar-1a | |
| | | | Ar-15c1 | |
| 6a-1-29 | 6a | 0 | Ar-1a | |
| | | | Ar-17a1 | |
| 6a-1-30 | 6a | 0 | Ar-11c | Ar-17a1 |
| 6a-1-31 | 6a | 0 | Ar-1a | Ar-30b |
| 6a-1-32 | 6a | 0 | Ar-1a | Ar-1a |
| 6a-1-33 | 6a | 0 | Ar-1a | Ar-13a |
| 6a-2-1 | 6a | 2 (Ar3-12a; Ar3-1a) | Ar-2a | Ar-2a |
| 6a-2-2 | 6a | 2 (Ar3-12a; Ar3-1b) | Ar-2a | Ar-2a |
| 6a-2-3 | 6a | 2 (Ar3-12a; Ar3-1b) | Ar-9b | Ar-2a |
| 6a-2-4 | 6a | 1 (Ar3-1b) | Ar-2a | Ar-2a |
| 6a-2-5 | 6a | 1 (Ar3-1b) | Ar-2b | Ar-2a |
| 6a-2-6 | 6a | 1 (Ar3-1b) | Ar-2b | Ar-2a |
| 6a-2-7 | 6a | 2 (Ar-1a, Ar2-7) | Ar-1a | |
| 6a-2-8 | 6a | 2 (Ar-1a, Ar2-7) | Ar-1a | |
| 6a-2-9 | 6a | 2 (Ar-1a, Ar2-7) | Ar-2c | |
| 6a-2-10 | 6a | 1 (Ar2-9c) | | Ar-2a |
| 6a-2-11 | 6a | 1 (Ar2-9c) | | Ar-2a |
| 6a-2-12 | 6a | 1 (Ar2-9c) | | Ar-2b |
| 6a-2-13 | 6a | 1 (Ar3-1a) | Ar-2a | Ar-2a |
| 6a-2-14 | 6a | 1 (Ar3-1a) | Ar-2a | Ar-2a |
| 6a-2-15 | 6a | 1 (Ar3-1a) | Ar-2a | Ar-2b |
| 6a-2-16 | 6a | 1 (Ar3-1a) | Ar-2a | Ar-17a2 |
| 6a-2-17 | 6a | 1 (Ar3-1a) | Ar-2a | Ar-17a3 |
| 6a-2-18 | 6a | 1 (Ar3-1a) | Ar-2a | Ar-17c2 |
| 6a-2-19 | 6a | 1 (Ar3-1a) | Ar-2a | Ar-9b |
| 6a-2-20 | 6a | 1 (Ar3-1a) | Ar-2b | Ar-9b |
| 6a-2-21 | 6a | 1 (Ar3-1a) | Ar-2b | Ar-10b |
| 6a-2-22 | 6a | 2 (Ar3-1b, Ar2-7) | | Ar-1a |
| 6a-2-23 | 6a | 2 (Ar3-1b, Ar2-7) | | Ar-2a |
| 6a-2-24 | 6a | 2 (Ar3-1b, Ar2-7) | | Ar-1a |
| 7a-1-1 | 7a | 0 | Ar-2a | Ar-2a |
| 7a-1-2 | 7a | 0 | Ar-9b | Ar-2c |
| 8a-1-1 | 8a | 0 | Ar1-16 | |
| | | | Ar1-16 | |
| 8a-1-2 | 8a | 0 | Ar1-16 | |
| | | | Ar1-17a | |
| 8a-1-3 | 8a | 0 | Ar1-16 | |
| | | | Ar1-14a | |
| 8a-1-4 | 8a | 0 | Ar1-14a | |
| | | | Ar1-14a | |
| 8a-1-5 | 8a | 0 | Ar1-14a | |
| | | | Ar1-13 | |
| 8a-1-6 | 8a | 0 | Ar1-14b | |
| | | | Ar1-14b | |
| 10a-1-1 | 10a | 0 | Ar-2a | Ar-2a |
| 10a-2-1 | 10a | 1 (Ar3-1a) | Ar-2a | Ar-9b |
| 10a-2-2 | 10a | 1 (Ar3-1a) | Ar-2b | Ar-9b |
| 10a-2-3 | 10a | 1 (Ar3-1b) | Ar-4a | Ar-9b |
| 10a-2-4 | 10a | 1 (Ar3-1a) | Ar-2a | Ar-2a |
| 10a-2-5 | 10a | 1 (Ar3-1a) | Ar-1a | Ar-2a |
| 10a-2-6 | 10a | 1 (Ar3-1a) | Ar-2b | Ar-2a |
| 10a-2-7 | 10a | 1 (Ar3-1b) | Ar-2a | Ar-2a |
| 10a-2-8 | 10a | 1 (Ar3-1b) | Ar-2a | Ar-2a |
| 10a-2-9 | 10a | 1 (Ar3-1b) | Ar-2a | Ar-3a |
| 10a-2-10 | 10a | 2 (Ar3-1b, Ar2-7) | | Ar-1a |
| 10a-2-11 | 10a | 2 (Ar3-1b, Ar2-9c) | | Ar-1a |
| 15a-1-1 | 15a | 0 | Ar-2a | Ar-2a |
| 15a-1-2 | 15a | 0 | Ar-2a | Ar-2a |
| 15a-2-1 | 15a | 1 (Ar3-1a) | Ar-2a | Ar-2a |
| 15a-2-2 | 15a | 1 (Ar3-1a) | Ar-9b | Ar-2a |
| 15a-2-3 | 15a | 1 (Ar3-1a) | Ar-1a | Ar-2a |
| 15a-2-4 | 15a | 1 (Ar3-1b) | Ar-2a | Ar-2a |
| 15a-2-5 | 15a | 1 (Ar3-1b) | Ar-2a | Ar-9b |
| 15a-2-6 | 15a | 1 (Ar3-1b) | Ar-2a | Ar-2b |
| 15a-2-7 | 15a | 2 (Ar3-1a, Ar2-7) | | Ar-1a |
| 15a-2-8 | 15a | 2 (Ar3-1b, Ar2-7) | | Ar-1a |
| 15a-2-9 | 15a | 2 (Ar3-1b, Ar2-7) | | Ar-2b |
| 21a-1-1 | 21a | 0 | Ar-2a | Ar-2a |
| 21a-1-2 | 21a | 0 | Ar-2c | Ar-9b |
| 21a-1-3 | 21a | 0 | Ar-17a1 | Ar-2a |
| 21a-1-4 | 21a | 0 | Ar-2a | Ar-2a |

Dabei können n und q in den Formeln die Werte 0 oder 1 annehmen. Bevorzugt sind die Reste R³ und R⁶ in den Formeln ausgewählt aus den Gruppen der Formeln (Ar-1) bis (Ar-33), besonders bevorzugt aus den Gruppen der Formeln (Ar-1) bis (Ar-10).

Beispiele für bevorzugte Verbindungen gemäß der oben aufgeführten Ausführungsformen, bzw. Verbindungen, wie sie bevorzugt in elektronischen Vorrichtungen eingesetzt werden können sind die Verbindungen der folgenden Strukturen (1) bis (98)

| | | |
|---|---|---|
| 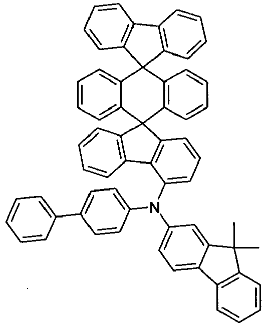 | 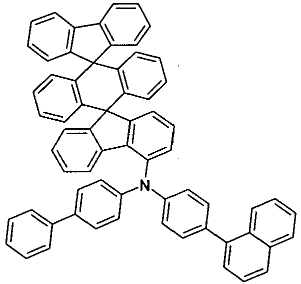 | 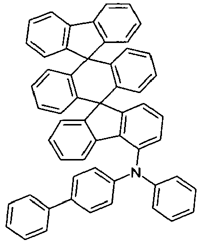 |
| 1 | 2 | 3 |
| 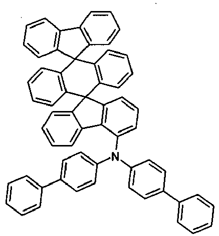 | 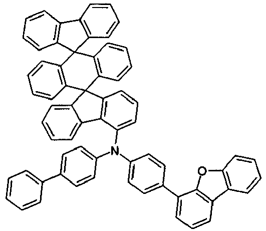 | 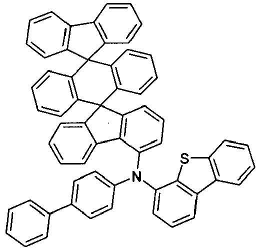 |
| 4 | 5 | 6 |
| 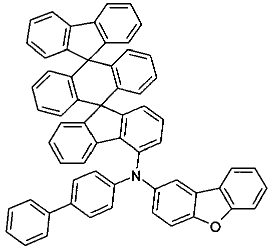 | 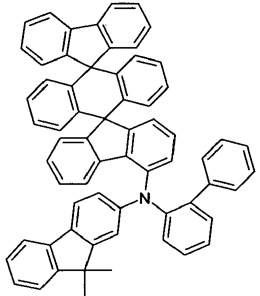 | 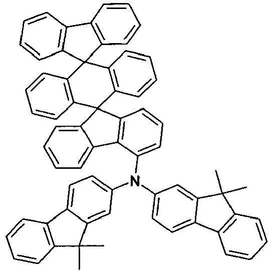 |
| 7 | 8 | 9 |
| 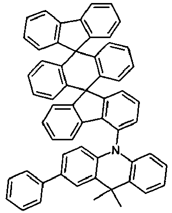 | 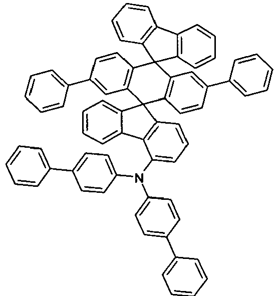 | 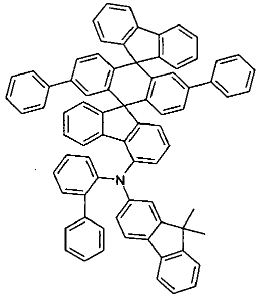 |
| 10 | 11 | 12 |
| 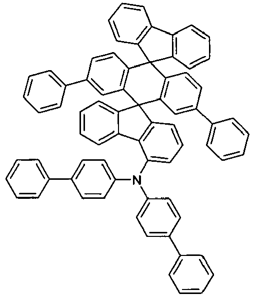 | 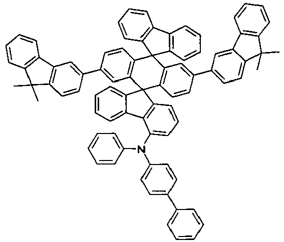 | 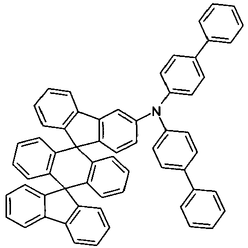 |
| 13 | 14 | 15 |
| 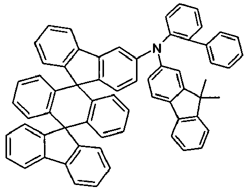 | 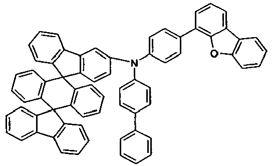 | 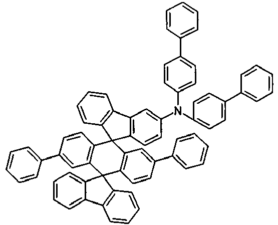 |
| 16 | 17 | 18 |
| 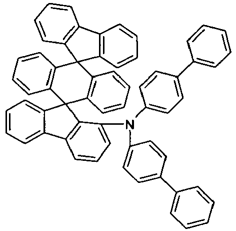 | 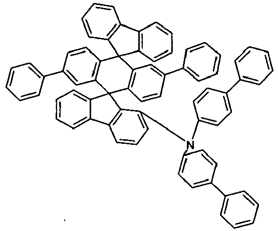 | 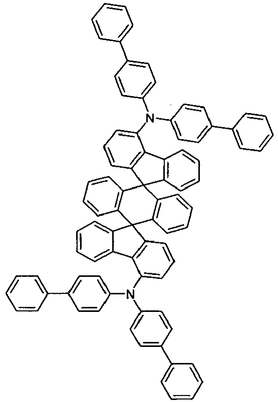 |
| 19 | 20 | 21 |
| 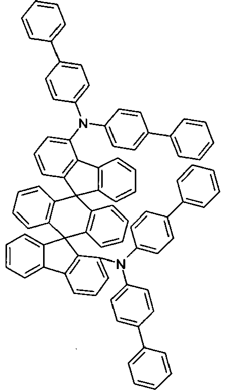 | 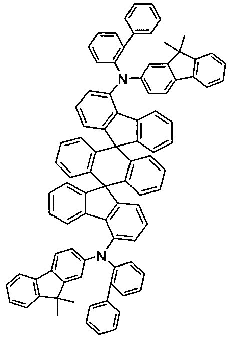 | 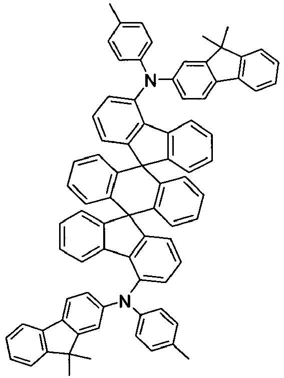 |
| 22 | 23 | 24 |
| 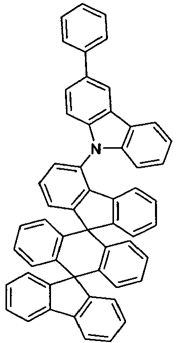 | 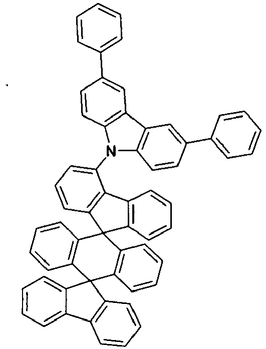 | 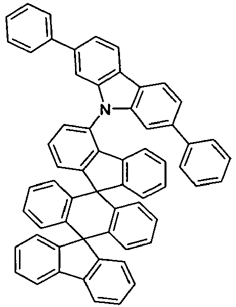 |
| 25 | 26 | 27 |
| 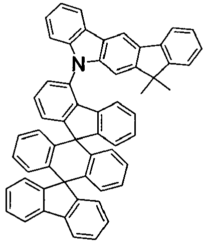 | 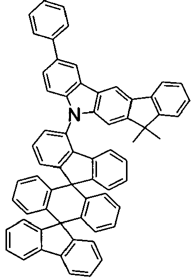 | 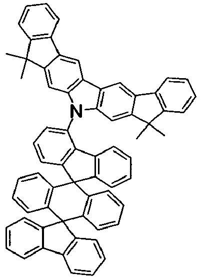 |
| 28 | 29 | 30 |
| 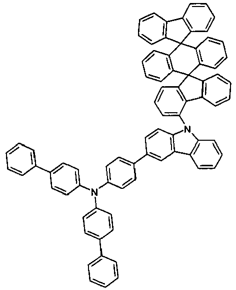 | 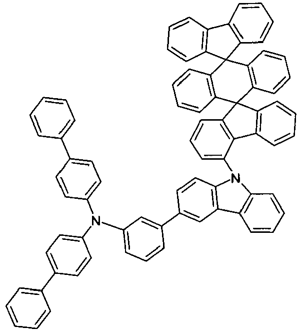 | 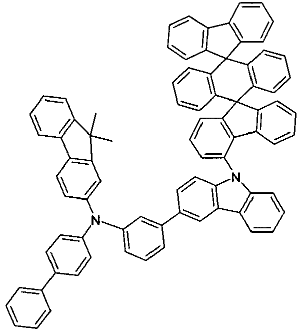 |
| 31 | 32 | 33 |
| 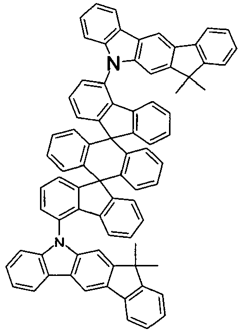 | 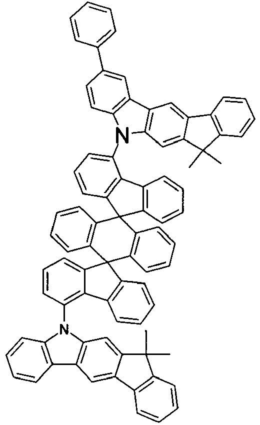 | 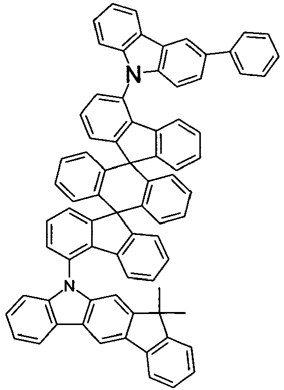 |
| 34 | 35 | 36 |
| 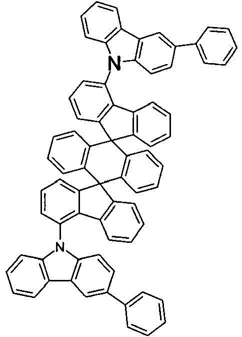 | 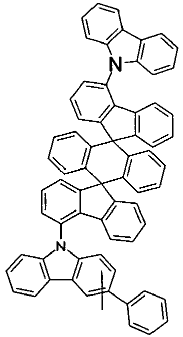 | 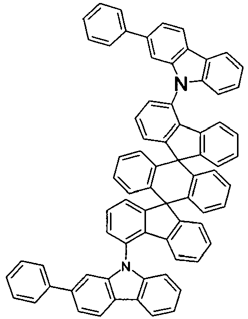 |
| 37 | 38 | 39 |
| 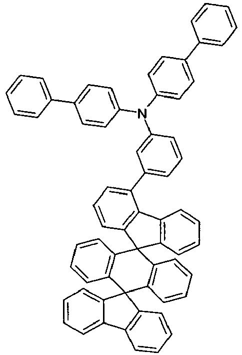 | 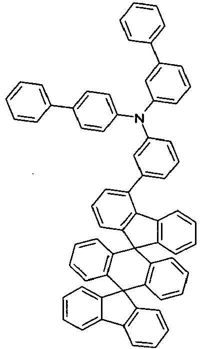 | 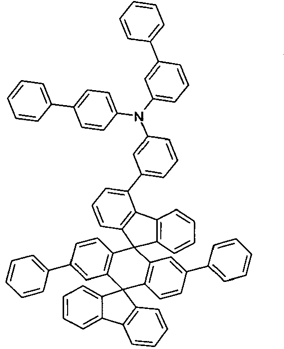 |
| 40 | 41 | 42 |
| 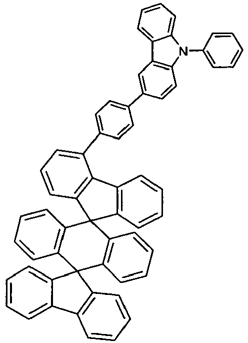 | 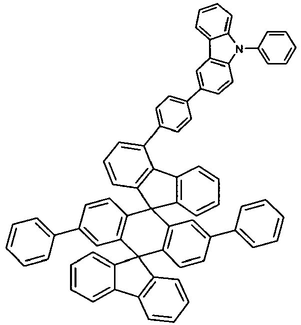 | 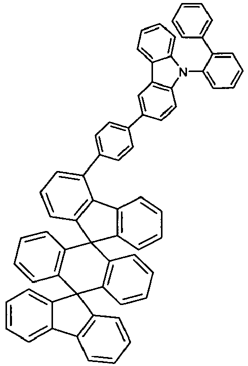 |
| 43 | 44 | 45 |
| 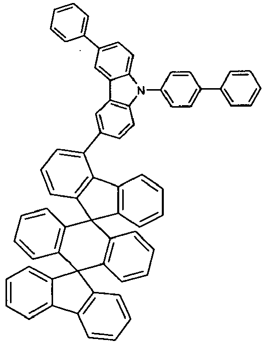 | 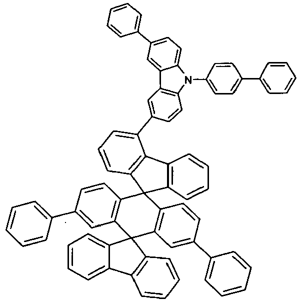 | 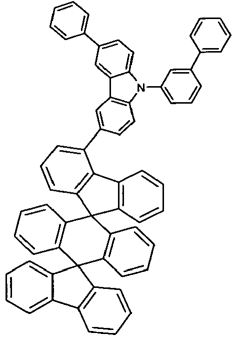 |
| 46 | 47 | 48 |
| 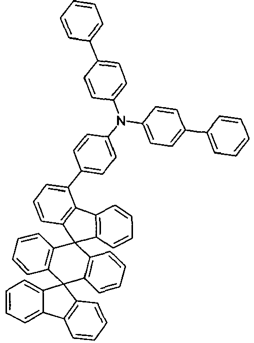 | 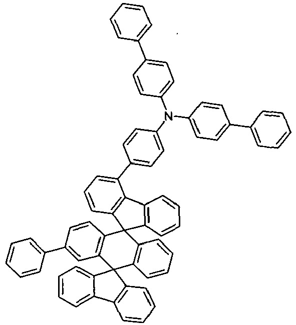 | 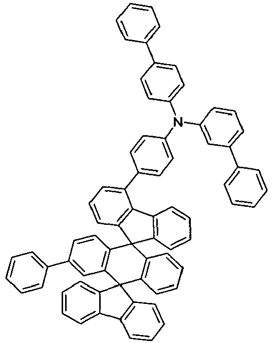 |
| 49 | 50 | 51 |
| 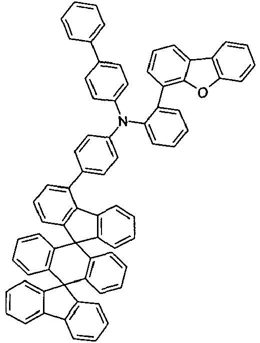 | 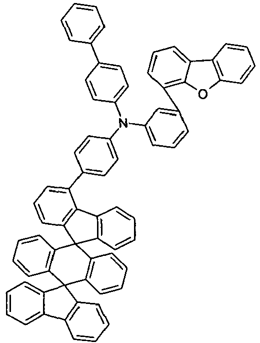 | 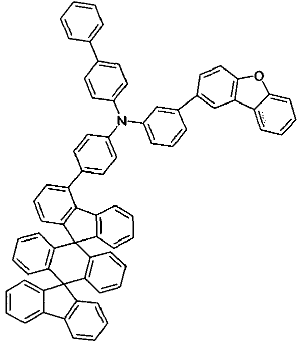 |
| 52 | 53 | 54 |
| 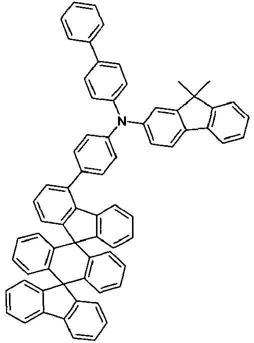 | 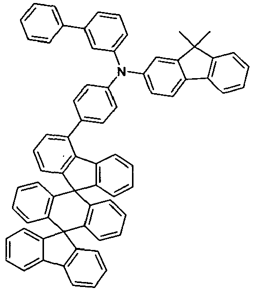 | 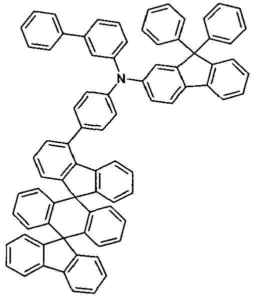 |
| 55 | 56 | 57 |
| 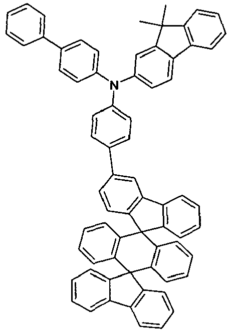 | 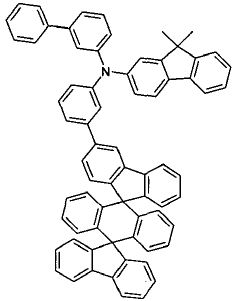 | 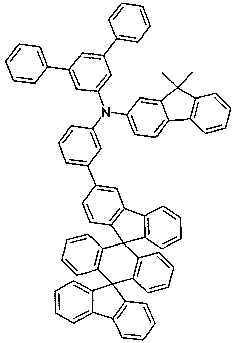 |
| 58 | 59 | 60 |
| 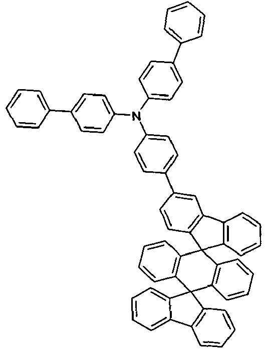 | 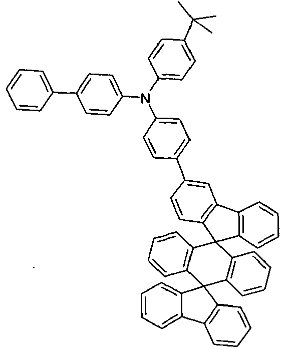 | 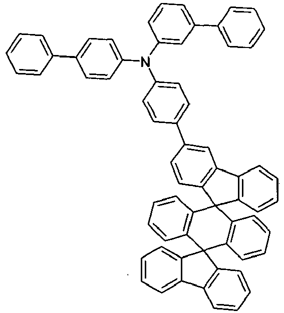 |
| 61 | 62 | 63 |
| 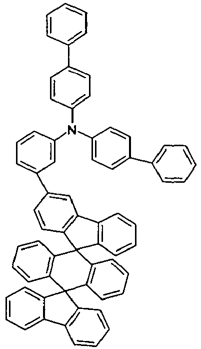 | 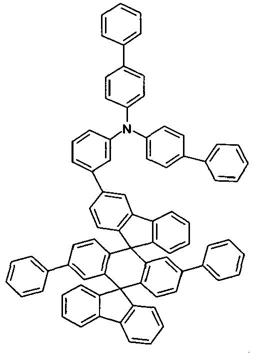 | 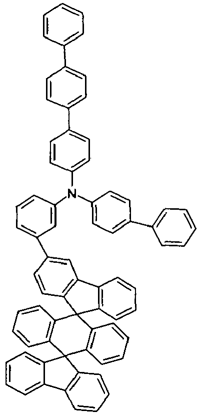 |
| 64 | 65 | 66 |
| 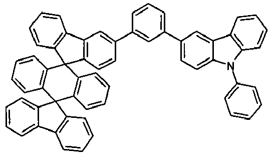 | 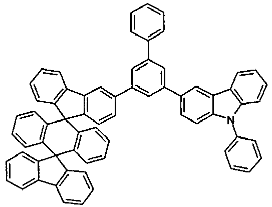 | 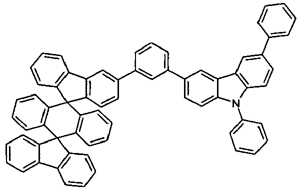 |
| 67 | 68 | 69 |
| 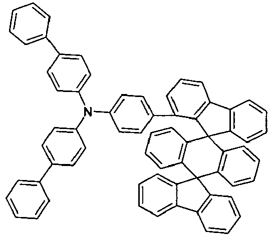 | 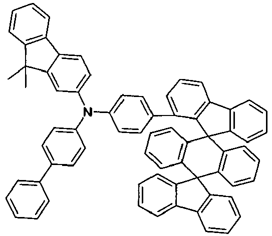 | 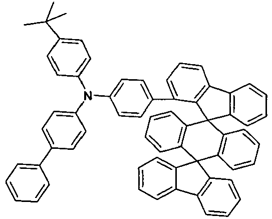 |
| 70 | 71 | 72 |
| 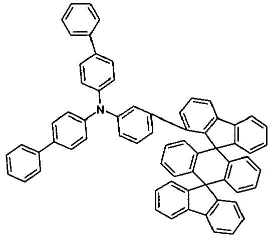 | 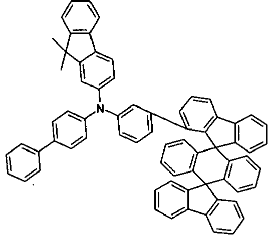 | 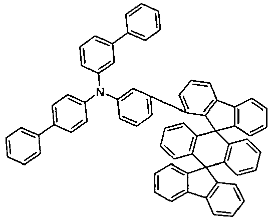 |
| 73 | 74 | 75 |
| 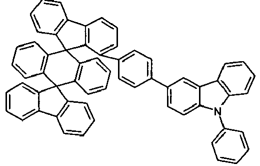 | 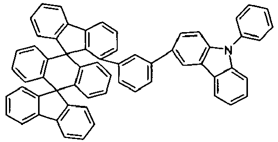 | 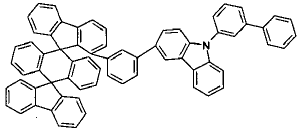 |
| 76 | 77 | 78 |
| 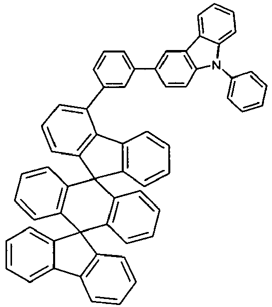 | 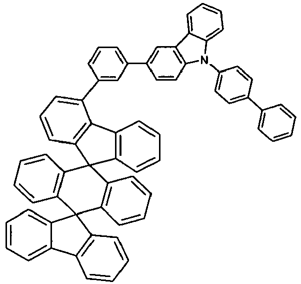 | 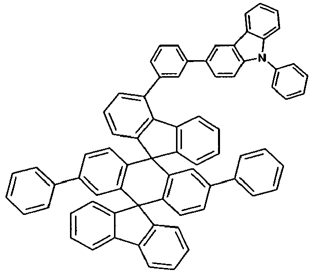 |
| 79 | 80 | 81 |
| 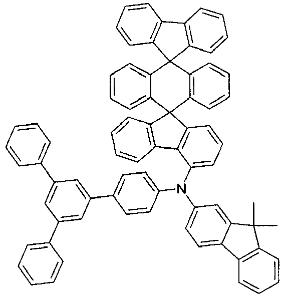 | 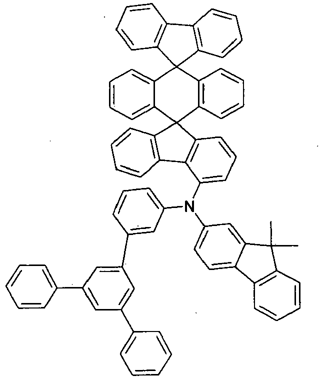 | 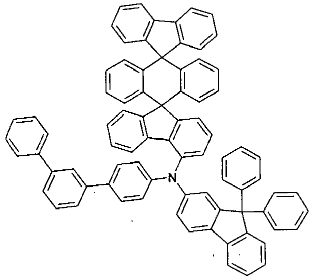 |
| 82 | 83 | 84 |
| 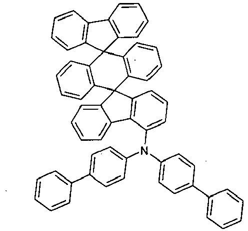 | 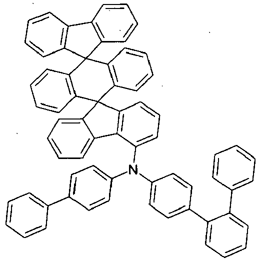 | 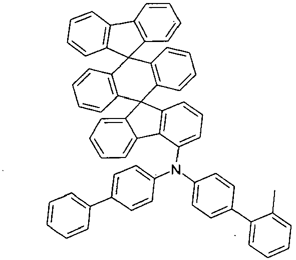 |
| 85 | 86 | 87 |
| 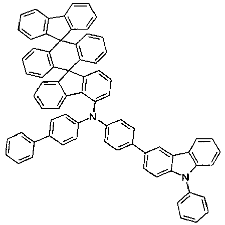 | 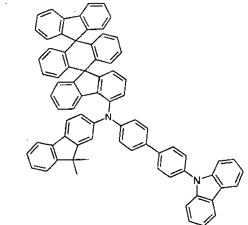 | |
| 88 | 89 | |
| 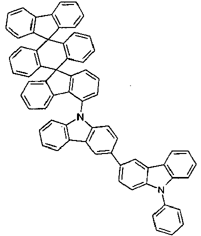 | 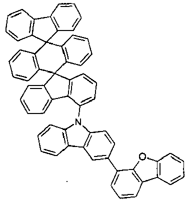 | 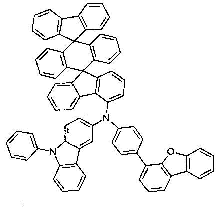 |
| 91 | 92 | 93 |
| 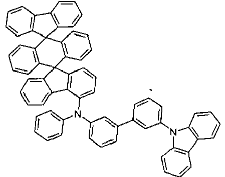 | | |
| 94 | | |
| 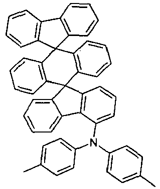 | 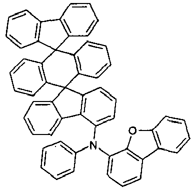 | 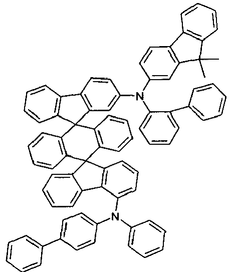 |
| 97 | 98 | 99 |
| 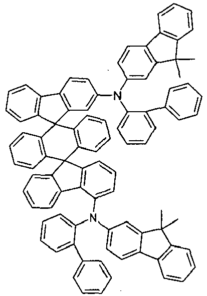 | 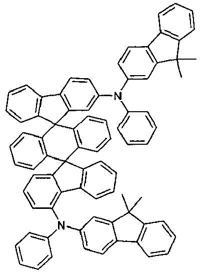 | 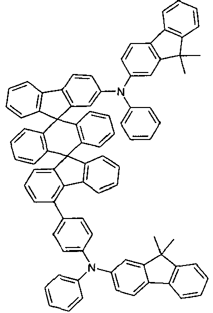 |
| 100 | 101 | 102 |
| 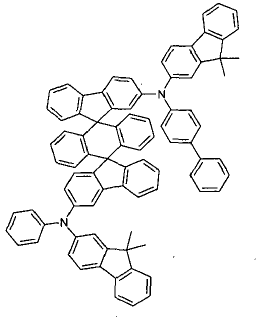 | 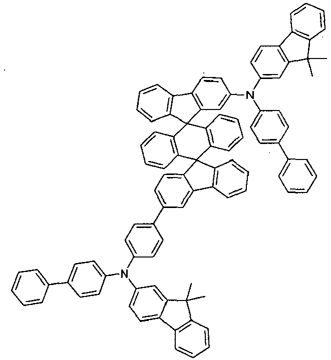 | 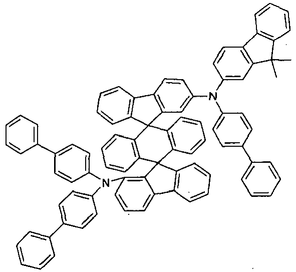 |
| 103 | 104 | 105 |
| 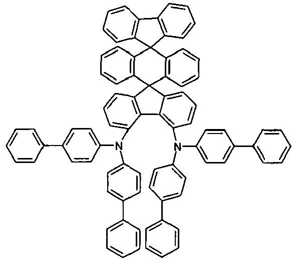 | | |
| 106 | | |

Die Synthese der erfindungsgemäßen Verbindungen kann gemäß im Stand der Technik bekannten Verfahren und Reaktionstypen, beispielsweise Bromierung, Ullmann-Arylierung, Friedel-Crafts Reaktion, Palladium-katalysierte intramolekulare C-H-Arylierung, Buchwald-Kupplung und Suzuki-Kupplung und Grignard-Reaktion erfolgen. Insbesondere lassen sich die Verbindungen aus einem entsprechend Halogensubstituierten Grundgerüst durch Einführung der Aminogruppe synthetisieren, wie in Schema 1 dargestellt. Dabei ist es entweder möglich, zunächst ein primäres Amin mit einem Substituenten Ar¹ einzuführen und die Gruppe Ar² in einer weiteren Kupplungsreaktion einzuführen, wie in Schema 1 a) gezeigt. Ebenso ist es möglich, direkt in einem Schritt das sekundäre Amin Ar¹Ar²NH einzuführen, wie in Schema 1 b) gezeigt. Als Gruppe X am Grundgerüst eignen sich reaktive Abgangsgruppen, wie beispielsweise Cl, Br, I, Triflat oder Tosylat. Als Kupplungsreaktionen eignen sich beispielsweise Kupplungsreaktionen nach Hartwig-Buchwald oder nach Ullmann. Die Reaktionsbedingungen, die für diese Kupplungsrektionen verwendet werden können, sind dem Fachmann der organischen Synthese bekannt.

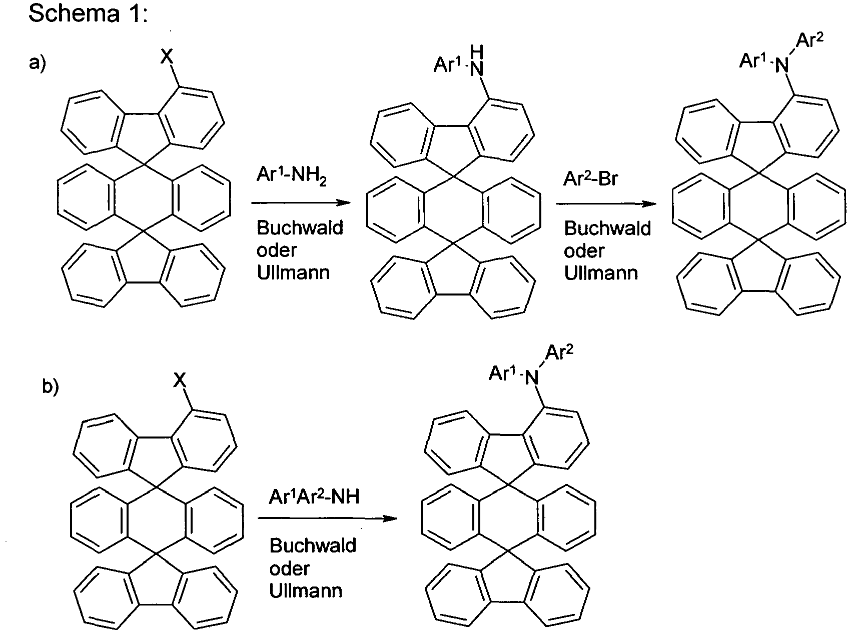

Für Verbindung mit i gleich 1 oder 2 lässt sich die Gruppe Ar^{S}-NAr¹-Ar² ebenfalls über eine metallkatalysierte Kupplungsreaktion einführen, beispielsweise über eine Suzuki-Kupplung oder eine Stille-Kupplung.

Ein weiterer Gegenstand der vorliegenden Erfindung ist daher ein Verfahren zur Herstellung einer Verbindung gemäß Formel (1) durch Kupplung eines Dispiro[fluoren-9,9'-anthracen-10',9"-fluoren]-Derivats, welches in 1, 3 oder 4-Position mit einer reaktiven Abgangsgruppe substituiert ist, mit
a) einem primären Amin, gefolgt von der Kupplung mit einer weiteren aromatischen Gruppe, die mit einer reaktiven Abgangsgruppe substituiert ist, oder
b) mit einem sekundären Amin, oder
c) mit einem Triarylaminderivat.

Dabei ist die reaktive Abgangsgruppe bevorzugt ausgewählt aus Cl, Br, I, Triflat oder Tosylat oder für eine Suzuki-Kupplung auch Boronsäure, bzw. ein Boronsäurederivat, insbesondere ein Boronsäureester.

Die Kupplungsreaktion ist bevorzugt ausgewählt aus Hartwig-Buchwald-Kupplungen, aus Ullmann-Kupplungen oder aus Suzuki-Kupplungen.

Der Aufbau des Dispiro[fluoren-9,9'-anthracen-10',9"-fluoren]-Gerüsts erfolgt bevorzugt analog zur klassischen Spirosynthese.

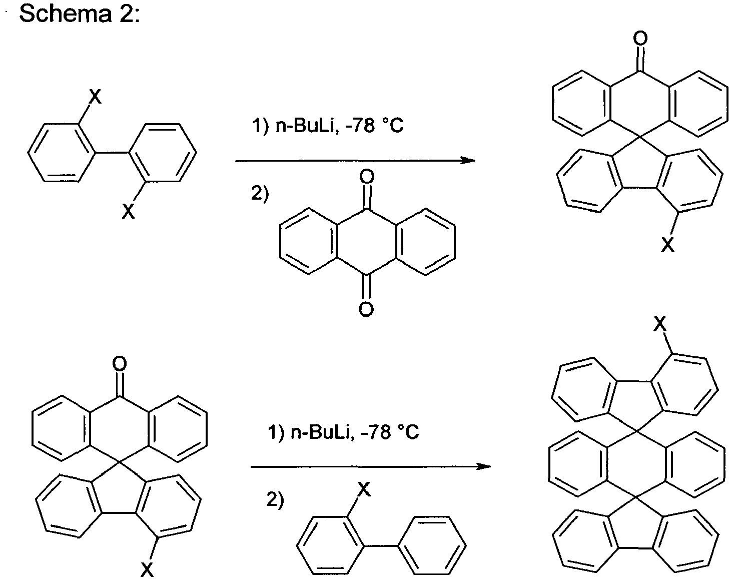

Dabei können durch die Verwendung von entsprechend substituierter Biphenyle unterschiedliche Substitutionsmuster am Grundgerüst erhalten werden.

Bei der Synthese von Grundgerüsten mit mehr als einem Substituenten, insbesondere bei der Verwendung von mehrfach substituierten Biphenylen, kann es bei der Ausbildung der Spirokohlenstoffe zur Bildung von Substitutionsisomeren kommen. Werden die Verbindungen nicht aufgereinigt, können sie auch als Gemisch eingesetzt werden.

Die oben gezeigten Syntheseverfahren haben exemplarischen Charakter und können vom Fachmann auf dem Gebiet der organischen Synthese in geeigneter Weise abgewandelt werden, wenn dies für die Synthese bestimmter Ausführungsformen von erfindungsgemäßen Verbindungen vorteilhaft ist.

Die oben beschriebenen erfindungsgemäßen Verbindungen, insbesondere Verbindungen, welche mit reaktiven Abgangsgruppen, wie Brom, Iod, Chlor, Boronsäure oder Boronsäureester, substituiert sind, können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. CC-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (1), wobei die Bindung(en) zum Polymer, Oligomer oder Dendrimer an beliebigen freien Positionen in Formel (1) lokalisiert sein können. Je nach Verknüpfung der erfindungsgemäßen Verbindung ist die Verbindung Bestandteil einer Seitenkette des Oligomers oder Polymers oder Bestandteil der Hauptkette.

Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist.

Die erfindungsgemäßen Polymere, Oligomere oder Dendrimere können konjugiert, teilkonjugiert oder nicht-konjugiert sein. Die erfindungsgemäßen Oligomere oder Polymere können linear, verzweigt oder dendritisch sein.

In den linear verknüpften Strukturen können die Einheiten gemäß Formel (1) direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein.

In verzweigten und dendritischen Strukturen können beispielsweise 3, 5 oder mehrere Einheiten gemäß Formel (1) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) in Oligomeren, Dendrimeren und Polymeren gelten dieselben Bevorzugungen wie oben für die erfindungsgemäßen Verbindungen beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1 .

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethyl-ether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-Dimethylphenyl)ethan oder Mischungen dieser Lösemittel.

Gegenstand der Erfindung ist daher weiterhin eine Formulierung, insbesondere eine Lösung, Dispersion oder Emulsion, enthaltend mindestens eine Verbindung gemäß Formel (1), oder mindestens ein Polymer, Oligomer oder Dendrimer enthaltend mindestens eine Einheit gemäß Formel (1), sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Weitere Gegenstande der Erfindung sind daher die Verwendung der erfindungsgemäßen Verbindungen in elektronischen Vorrichtungen sowie elektronische Vorrichtungen selbst, welche eine oder mehrere erfindungsgemäße Verbindungen enthalten. Dabei sind die elektronischen Vorrichtungen bevorzugt ausgewählt aus der Gruppe bestehend aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETS), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und besonders bevorzugt organischen Elektrolumineszenzvorrichtungen (OLEDs). Besonders bevorzugt sind organische Elektrolumineszenzvorrichtungen, enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine erfindungsgemäße Verbindung enthält.

Die organischen Elektrolumineszenzvorrichtungen und die lichtemittierenden elektrochemischen Zellen können für verschiedene Anwendungen eingesetzt werden, beispielsweise für einfarbige oder mehrfarbige Displays, für Beleuchtungsanwendungen oder für medizinische und/oder kosmetische Anwendungen, beispielsweise in der Phototherapie.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Exzitonenblockierschichten, Zwischenschichten (interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergangen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die organische Elektrolumineszenzvorrichtung kann auch mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues, gelbes, orangefarbenes oder rotes Licht emittieren. Insbesondere bevorzugt sind Dreischichtsysteme, also Systeme mit drei emittierenden Schichten, wobei mindestens eine dieser Schichten mindestens eine erfindungsgemäße Verbindung enthält und wobei die drei Schichten blaue, grüne und orangefarbene oder rote Emission zeigen (für den prinzipiellen Aufbau siehe z. B. WO 2005/011013). Alternativ und/oder zusätzlich können die erfindungsgemäßen Verbindungen auch in der Lochtransportschicht und/oder in einer Zwischenschicht (interlayer) vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert. Dabei ist es möglich, dass alle emittierenden Schichten fluoreszierend sind oder dass alle emittierenden Schichten phosphoreszierend sind oder dass eine oder mehrere emittierende Schichten fluoreszierend und eine oder mehrere andere Schichten phosphoreszierend sind.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die bevorzugten Ausführungsformen als Lochtransportmaterial in einer Lochtransport- oder Lochinjektions- oder Exzitonenblockierschicht oder als Matrixmaterial für fluoreszierende oder phosphoreszierende Emitter, insbesondere für phosphoreszierende Emitter. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. die bevorzugten Ausführungsformen als Lochtransport- oder Lochinjektionsmaterial in einer Lochtransport- oder Lochinjektionsschicht eingesetzt. Dabei kann die emittierende Schicht fluoreszierend oder phosphoreszierend sein. Eine Lochinjektionsschicht im Sinne der vorliegenden Erfindung ist eine Schicht, die direkt an die Anode angrenzt. Eine Lochtransportschicht im Sinne der vorliegenden Erfindung ist eine Schicht, welche zwischen einer Lochinjektionsschicht und einer emittierenden Schicht vorliegt. Die Lochtransportschicht kann direkt an die Emissionschicht angrenzen. Wenn die Verbindungen gemäß Formel (1), bzw. die bevorzugten Ausführungsformen, als Lochtransportmaterial oder als Lochinjektionsmaterial verwendet werden, kann es bevorzugt sein, wenn sie mit Elektronenakzeptor-Verbindungen dotiert sind, beispielsweise mit F4-TCNQ oder mit Verbindungen, wie sie in EP 1476881 oder EP 1596445 beschrieben werden.

In einer weiteren bevorzugten Ausführungsform der Erfindung wird eine Verbindung gemäß Formel (1), bzw. eine bevorzugte Ausführungsform, als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Wird die Verbindung gemäß Formel (1), bzw. eine bevorzugte Ausführungsform, als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In nochmals einer weiteren bevorzugten Ausführungsform der Erfindung wird die Verbindung gemäß Formel (1) bzw. eine bevorzugte Ausführungsform, in einer Elektronenblockierschicht eingesetzt. Unter einer Elektronenblockierschicht wird eine Schicht verstanden, die auf Anodenseite direkt an eine emittierende Schicht angrenzt.

Besonders bevorzugt ist der Einsatz der Verbindung gemäß Formel (1) bzw. der bevorzugten Ausführungsformen in einer Lochtransport- bzw. Elektronenblockierschicht.

In einer weiteren bevorzugten Ausführungsform der Erfindung wird die Verbindung gemäß Formel (1) bzw. eine bevorzugte Ausführungsform, als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. eine bevorzugte Ausführungsform, als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1 verstanden, insbesondere aus einem angeregten Triplettzustand. Im Sinne dieser Anmeldung sollen alle lumineszierenden Komplexe mit Übergangsmetallen oder Lanthanoiden, insbesondere alle lumineszierenden Iridium-, Platin- und Kupferkomplexe als phosphoreszierende Verbindungen angesehen werden.

Die Mischung aus der Verbindung gemäß Formel (1) bzw. eine bevorzugte Ausführungsform, und der emittierenden Verbindung enthält zwischen 99.9 und 1 Gew.-%, vorzugsweise zwischen 99 und 10 Gew.-%, besonders bevorzugt zwischen 97 und 60 Gew.-%, insbesondere zwischen 95 und 80 Gew.-% der Verbindung gemäß Formel (1) bzw. eine bevorzugte Ausführungsform, bezogen auf die Gesamtmischung aus Emitter und Matrixmaterial. Entsprechend enthält die Mischung zwischen 0.1 und 99 Gew.-%, vorzugsweise zwischen 1 und 90 Gew.-%, besonders bevorzugt zwischen 3 und 40 Gew.-%, insbesondere zwischen 5 und 20 Gew.-% des Emitters bezogen auf die Gesamtmischung aus Emitter und Matrixmaterial. Die oben angegebenen Grenzen gelten insbesondere, wenn die Schicht aus Lösung aufgebracht wird. Wird die Schicht durch Vakuumverdampfung aufgebracht, gelten dieselben Zahlenwerte, wobei in diesem Fall der Prozentanteil jeweils in Vol.-% angegeben ist.

Eine emittierende Schicht einer organischen Elektrolumineszenzvorrichtung kann auch Systeme umfassend mehrere Matrixmaterialien (Mixed-Matrix-System) und/oder mehrere Dotanden enthalten. In diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer bevorzugten Ausführungsform der Erfindung wird die Verbindung gemäß Formel (1) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Die beiden unterschiedlichen Matrixmaterialien können dabei in einem Verhältnis von 1 : 10 bis 1 : 1 vorhanden sein, bevorzugt in einem Verhältnis von 1 :4 bis 1 : 1.

Die Mixed-Matrix-Systeme können einen oder mehrere Dotanden umfassen. Die Dotandverbindung bzw. die Dotandverbindungen zusammen haben erfindungsgemäß die vorstehend für den Emitter angegebenen Anteile.

Bevorzugt werden Mixed-Matrix-Systeme in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems eingesetzt werden können, sind aromatische Ketone, aromatische Phosphinoxide oder aromatische Sulfoxide oder Sulfone, z. B. gemäß WO 04/013080, WO 04/093207, WO 06/005627 oder WO 10/006680, Triarylamine, Carbazolderivate, z. B. CBP (N,N-Biscarbazolylbiphenyl), m-CBP oder die in WO 05/039246, US 2005/0069729, JP 2004/288381, EP 1205527 oder WO 08/086851 offenbarten Carbazolderivate, Indolocarbazolderivate, z. B. gemäß WO 07/063754 oder WO 08/056746, Indenocarbazolderivate, z. B. gemäß WO 2010/136109 oder WO 2011/000455, Azacarbazolderivate, z. B. gemäß EP 1617710, EP 1617711, EP1731584, JP 2005/347160, bipolare Matrixmaterialien, z. B. gemäß WO 07/137725, Silane, z. B. gemäß WO 05/111172, Azaborole oder Boronester, z. B. gemäß WO 06/117052, Triazinderivate, z. B. gemäß WO 2010/015306, WO 2007/063754 oder WO 2008/056746, Zinkkomplexe, z. B. gemäß EP 652273 oder WO 09/062578, Fluorenderivate, z. B. gemäß WO 2010/054730, oder verbrückte Carbazolderivate, z. B. gemäß US 2009/0136779, WO 2010/050778, WO 2011/042107 oder gemäß der nicht offengelegten Anmeldung DE 10201005697.9, Diazasilol- bzw. Tetraazasilol-Derivate, z. B. gemäß WO 10/054729, oder Diazaphosphol-Derivate, z. B. gemäß WO 10/054730. Weiterhin ist es möglich, einen elektronisch neutralen Co-Host zu verwenden, der weder lochtransportierende noch elektronentransportierende Eigenschaften aufweist, wie beispielsweise in WO 2010/108579 beschrieben.

Ebenso ist es möglich, zwei oder mehrere phosphoreszierende Emitter in der Mischung zu verwenden. Dabei wirkt der Emitter, welcher kürzerwellig emittiert, als Co-Host in der Mischung.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Dotanden können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Explizite Beispiele für geeignete phosphoreszierende Emitterverbindungen können weiterhin einer im Folgenden abgebildeten Tabelle phosphoreszierender Dotanden entnommen werden.

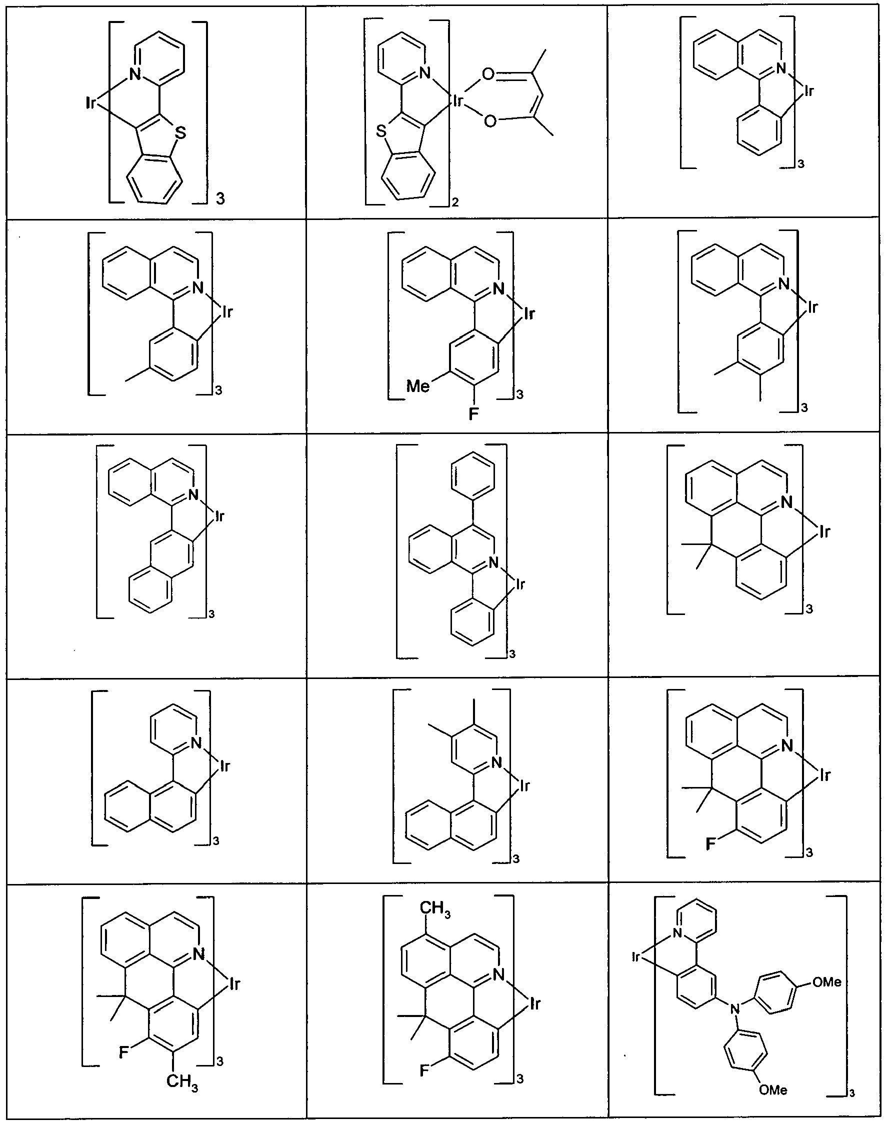

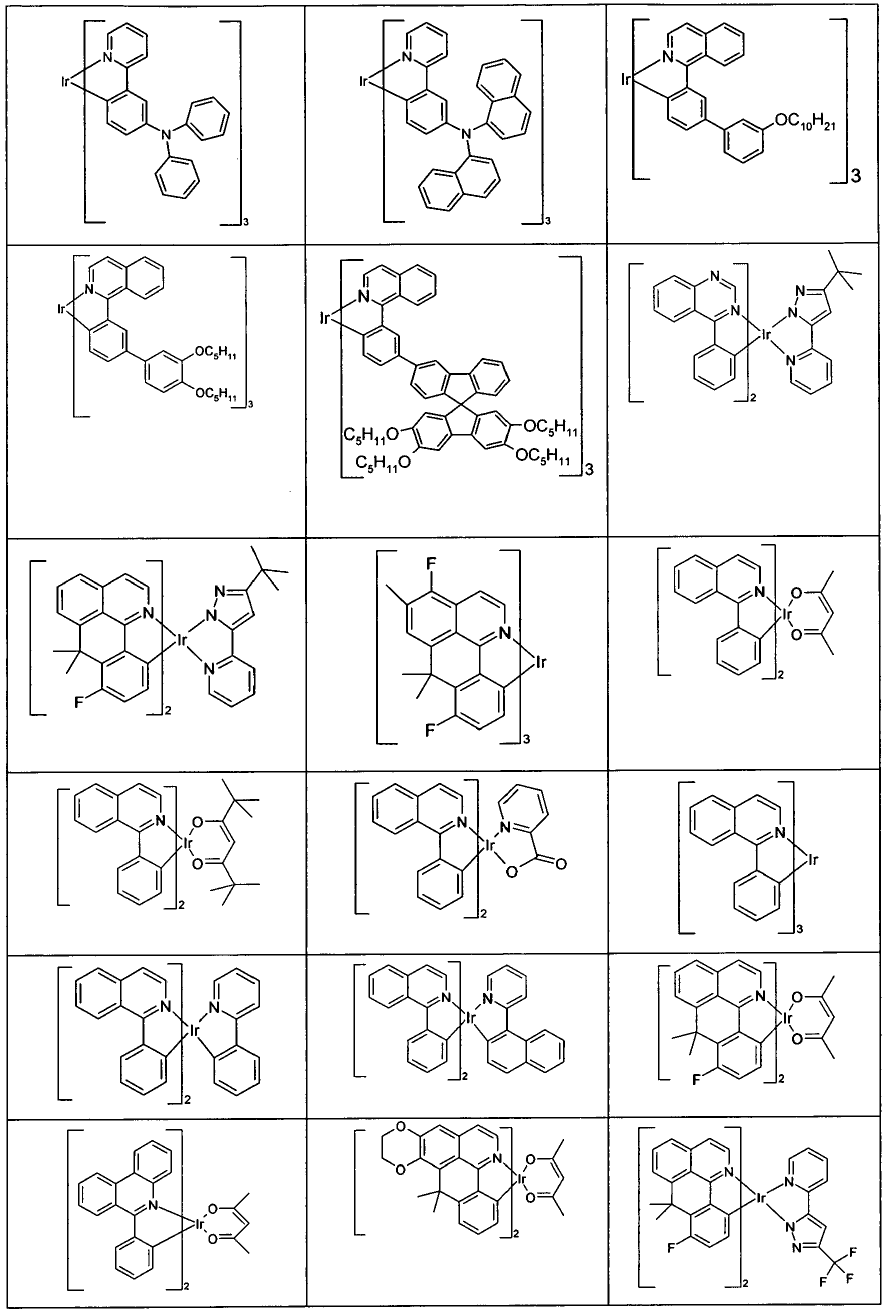

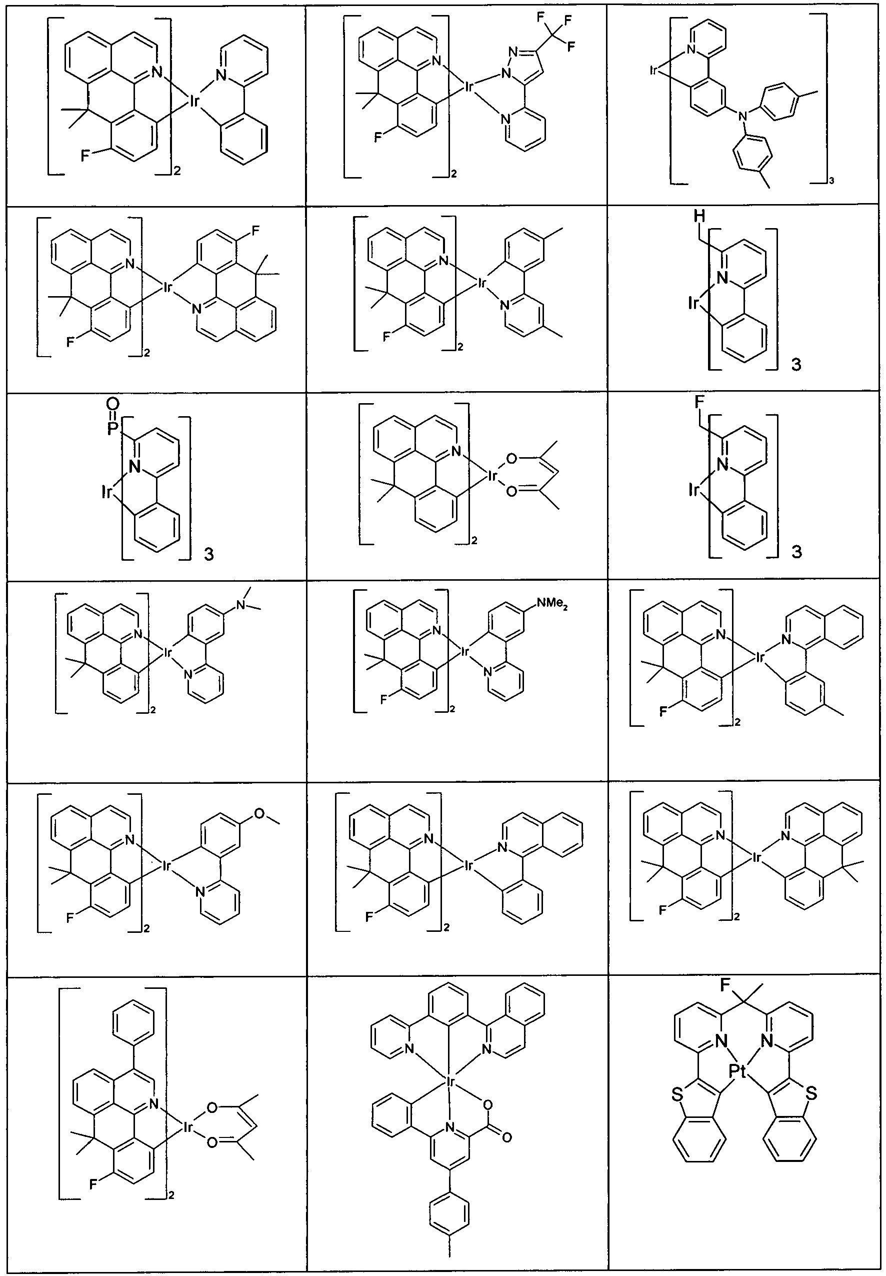

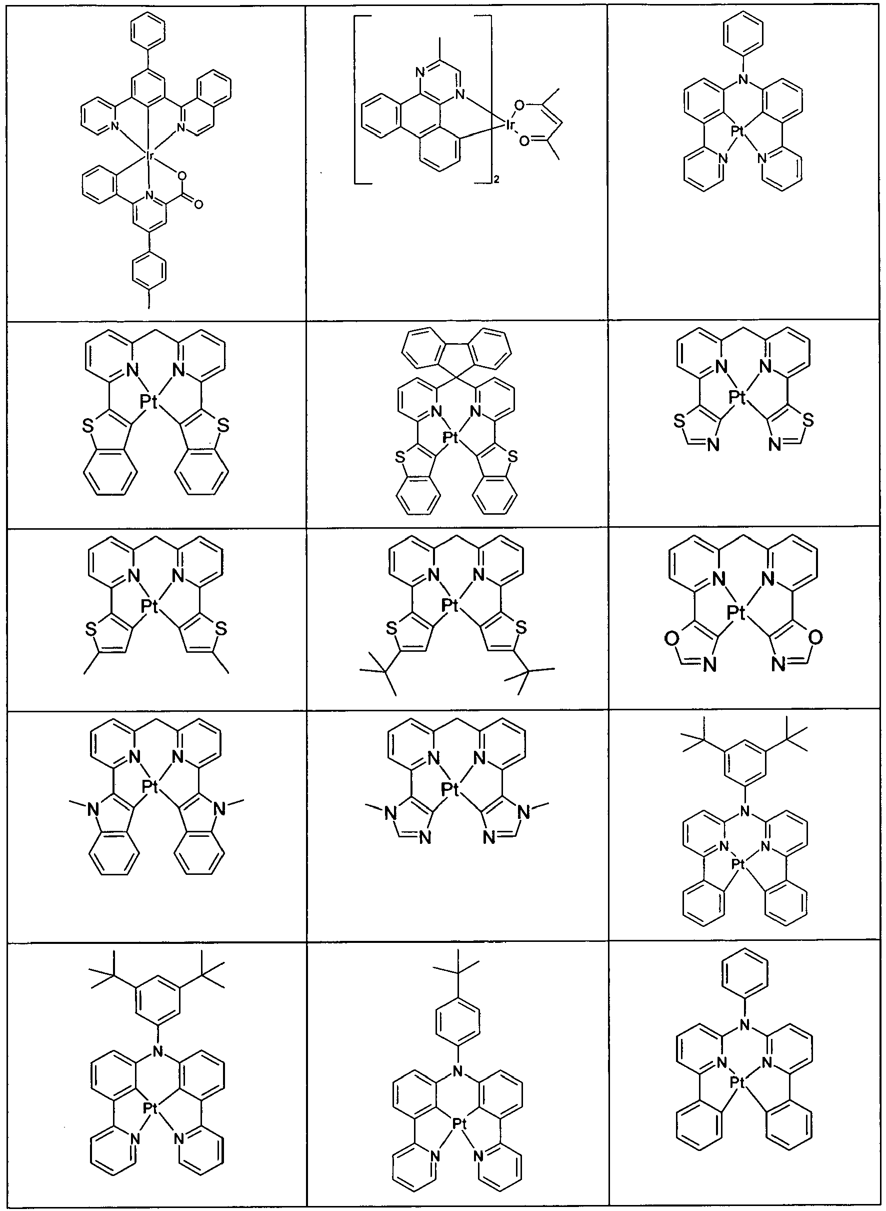

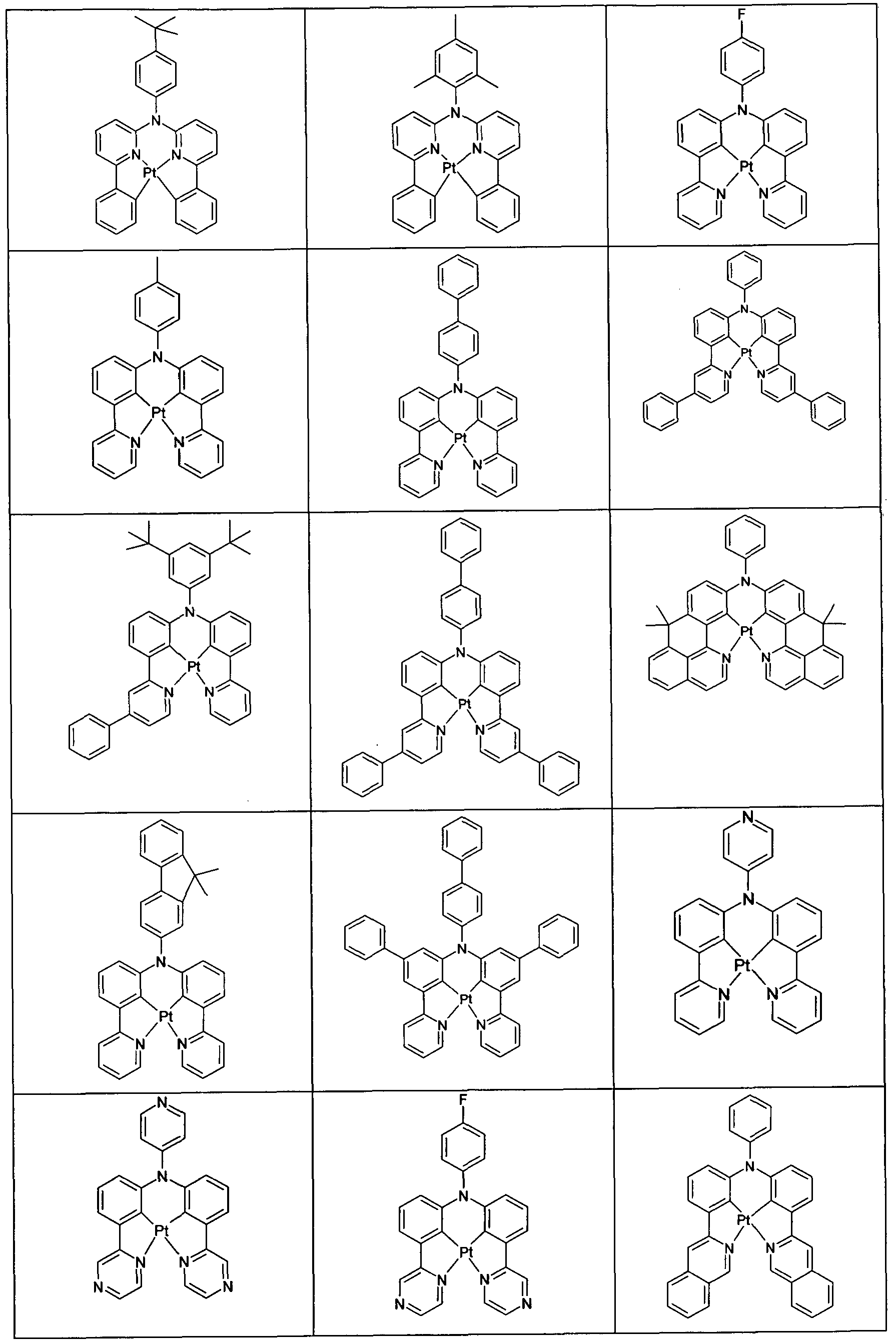

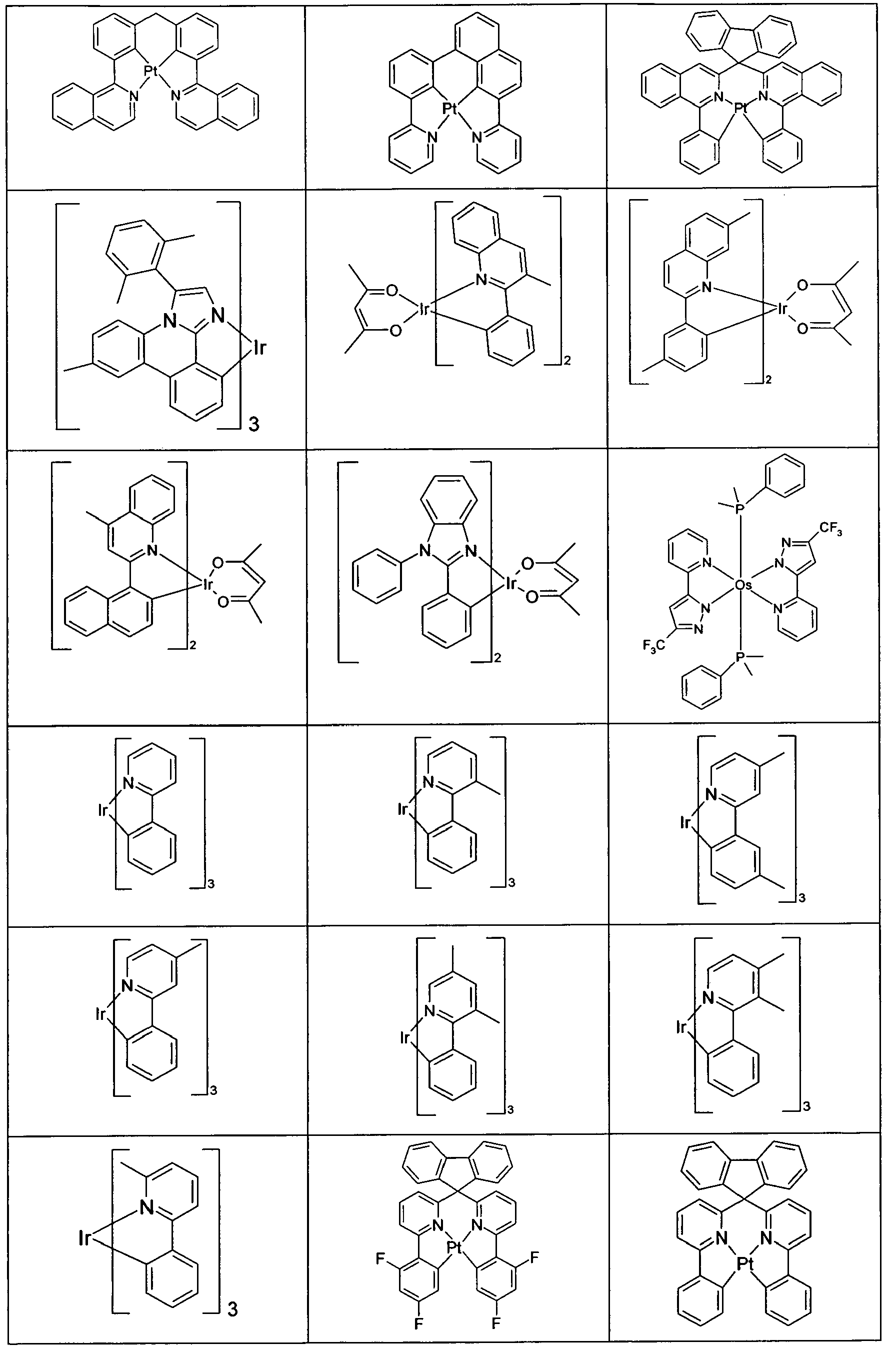

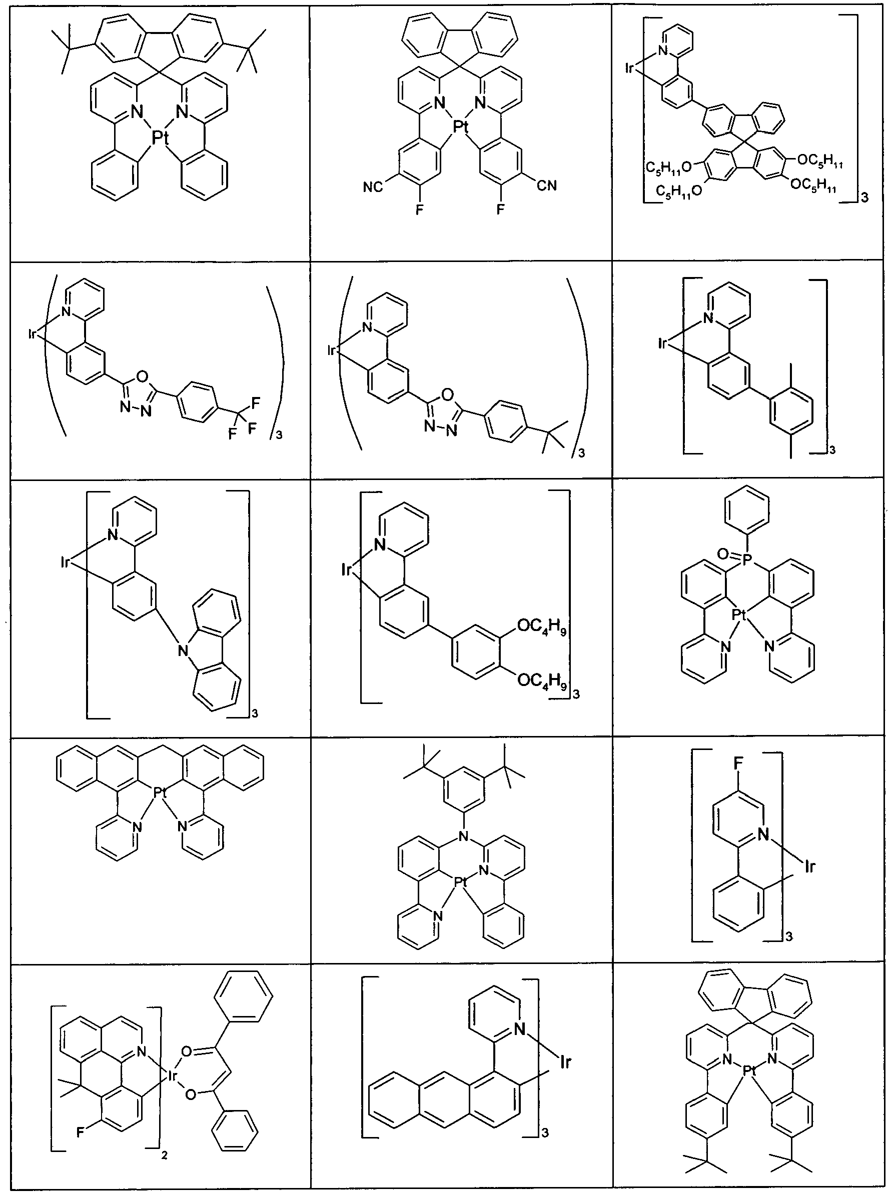

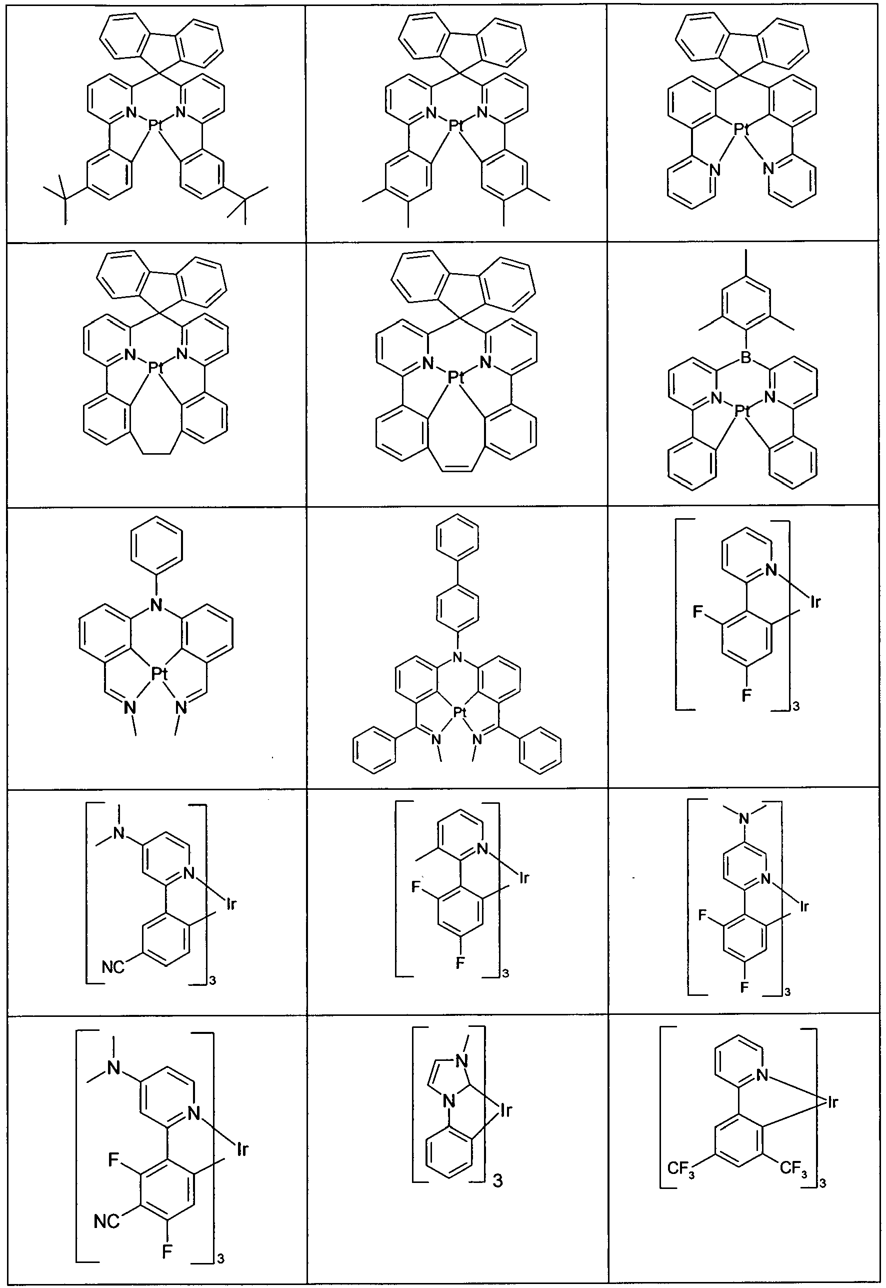

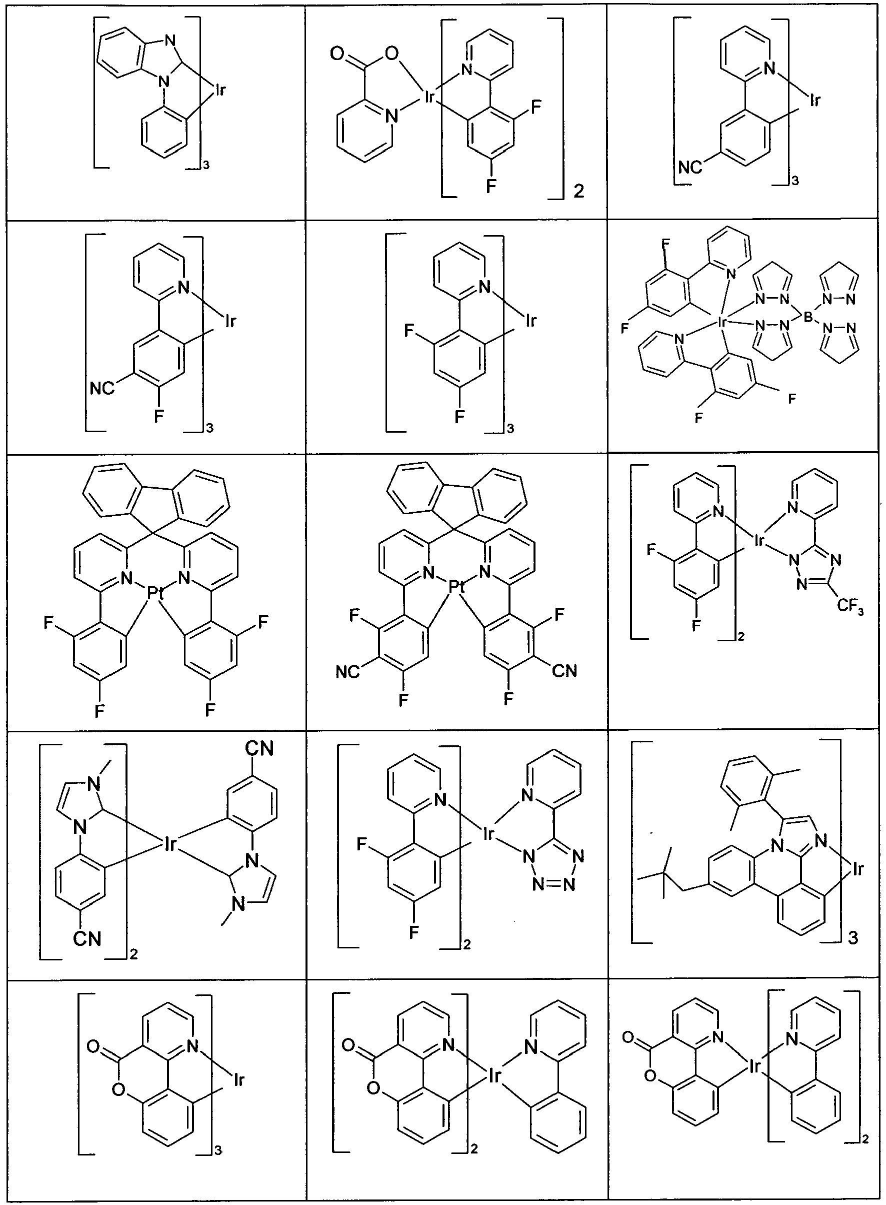

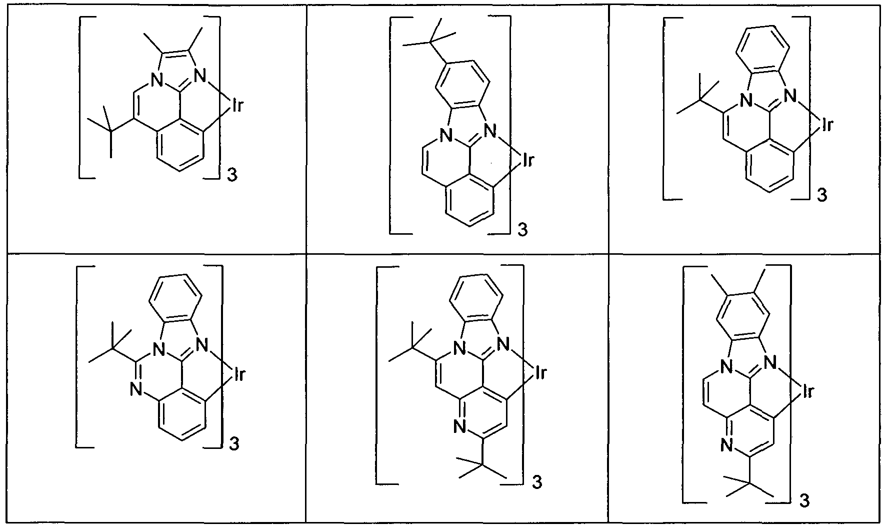

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionsschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Es ist weiterhin möglich, die Verbindung gemäß Formel (1) bzw. die bevorzugten Ausführungsformen sowohl in einer Lochtransportschicht bzw. Exzitonenblockierschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden.

Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den bevorzugten Ausführungsformen einsetzen.

Gemäß einer weiteren bevorzugten Ausführungsform der Erfindung werden die Verbindungen gemäß Formel (1), bzw. die bevorzugten Ausführungsformen in einer Zwischenschicht (interlayer) eingesetzt. Zwischenschichten werden bevorzugt in organischen Elektrolumineszenzvorrichtungen enthaltend mehrere emittierende Schichten eingesetzt, beispielsweise in weiß emittierenden OLEDs, welche jeweils eine rot emittierende, eine grün emittierende und eine blau emittierende Schicht enthalten. Besonders bevorzugt sind Zwischenschichten zwischen zwei emittierenden Schichten angeordnet. Eine Zwischenschicht enthaltend eine erfindungsgemäße Verbindung ist gemäß einer bevorzugten Ausführungsform der Erfindung zwischen der blau emittierenden Schicht und der grün emittierenden Schicht einer weißes Licht emittierenden OLED, welche eine rot emittierende, eine grün emittierende und eine blau emittierende Schicht enthält, angeordnet. Besonders bevorzugt ist die blau emittierende Schicht dabei eine fluoreszierende Schicht, und die grün emittierende Schicht ist eine phosphoreszierende Schicht.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Dotanden sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. -diaminen, beispielsweise gemäß WO 2007/140847. Weiterhin bevorzugt sind die in WO 2010/012328 offenbarten kondensierten Kohlenwasserstoffe.

Geeignete fluoreszierende Dotanden sind weiterhin die in JP 2006100 1973, WO 2004/047499, WO 2006/098080, WO 2007/065678, US 2005/0260442 und WO 2004/092111 offenbarten Derivate dieser Strukturen.

Als Matrixmaterialien, bevorzugt für fluoreszierende Dotanden, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev, 2007, 107(4), 953-1010 offenbarten Verbindungen oder andere Materialien, wie sie gemäß dem Stand der Technik in diesen Schichten eingesetzt werden.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, AI, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder AI, wobei dann in der Regel Kombinationen der Metalle, wie beispielsweise Ca/Ag, Ba/Ag oder Mg/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li2O, BaF2, MgO, NaF, CsF, CS₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4,5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOx, Al/PtOx) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink-Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et a/. ,Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1), bzw. einer bevorzugten Ausführungsform, nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Weiterhin sind Hybridverfahren möglich, bei denen beispielsweise eine oder mehrere Schichten aus Lösung aufgebracht werden und eine oder mehrere weitere Schichten aufgedampft werden. So kann beispielsweise die emittierende Schicht aus Lösung aufgebracht werden und die Elektronentransportschicht aufgedampft werden. Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Ein weiterer Gegenstand der vorliegenden Erfindung sind Mischungen enthaltend mindestens eine Verbindung gemäß Formel (1) bzw. die oben ausgeführten bevorzugten Ausführungsformen und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird. Die Mischung kann dann auch zusätzlich noch ein weiteres Material als zusätzliches Matrixmaterial enthalten.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransport- bzw. Lochinjektionsschicht in einer organischen Elektrolumineszenzvorrichtung. Dabei eignen sie sich insbesondere auch für die Verwendung in einer Schicht, die direkt an eine phosphoreszierende emittierende Schicht angrenzt, da die erfindungsgemäßen Verbindungen nicht die Lumineszenz löschen.
2. Die erfindungsgemäßen Verbindungen, eingesetzt als Matrixmaterial für fluoreszierende oder phosphoreszierende Emitter führen zu sehr hohen Effizienzen sowie zu langen Lebensdauern. Dies gilt insbesondere, wenn die Verbindungen als Matrixmaterial zusammen mit einem weiteren Matrixmaterial und einem phosphoreszierenden Emitter eingesetzt werden.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Betriebsspannungen.
4. Die erfindungsgemäßen Verbindungen weisen eine hohe thermische Stabilität auf und lassen sich unzersetzt und rückstandsfrei sublimieren.
5. Die erfindungsgemäßen Verbindungen weisen eine hohe Oxidationsstabilität auf, was sich insbesondere positiv auf die Handhabung dieser Verbindungen sowie auf die Lagerstabilität für Lösungen auswirkt.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

Die Erfindung wird durch die nachfolgenden Anwendungsbeispiele näher erläutert, wobei die Erfindung nicht auf den Umfang der Beispiele beschränkt ist.

### Ausführungsbeispiele

Die nachfolgenden Synthesen werden, sofern nicht anders angegeben, unter einer Schutzgasatmosphäre durchgeführt. Die Edukte können von den Firmen ALDRICH bzw. ABCR (Palladium(II)acetat, Tri-o-tolylphosphin, Anorganika, Lösemittel) bezogen werden.

Die in den folgenden Tabellen mit* gekennzeichneten Verbindungen können auch als Gemisch von Substituionsisomeren vorliegen. Dies kann auch bereits auf die verwendeten Edukte zutreffen.

Die Verbindungen können außerdem gegebenenfalls als Gemisch von Enantionmeren oder Diastomeren vorliegen.

### Beispiel 0

### Synthese der Verbindung (0-1)

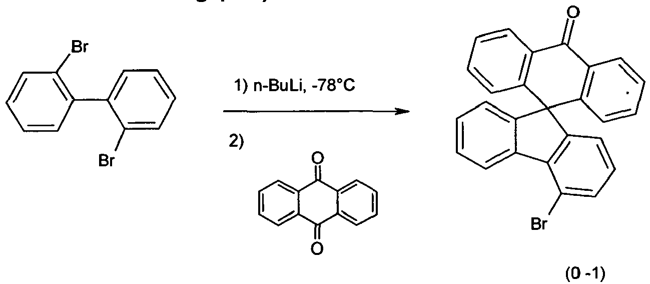

37,4 g (120 mmol) 2,2'-Dibromo-biphenyl werden in einem ausgeheizten Kolben in 300 mL getrocknetem THF gelöst. Die Reaktionsmischung wird auf -78°C gekühlt. Bei dieser Temperatur werden 75 mL einer 15%-igen Lösung n-BuLi in Hexan (119 mmol) langsam zugetropft. Der Ansatz wird 1 h bei -70°C nachgerührt. Anschließend werden 22,9 g Anthrachinon (CAS-Nr.: 84-65-1) (110 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 500 ml Essigsäure versetzt und anschließend werden 90 ml rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Ausbeute beträgt 44,4 (105 mmol) g (87,5% der Theorie).

Analog hierzu werden die folgenden Verbindungen (0-2) bis (0-3) hergestellt.

| | Edukt 1 | Edukt 2 | Produkt | Ausbeute |
|---|---|---|---|---|
| 0-2 | 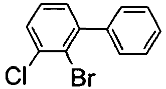 | 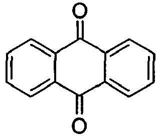 | 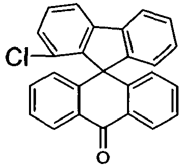 | 50 % |
| | 1195886-14-9 | 84-65-1 | | |
| 0-3 | 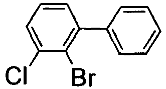 | 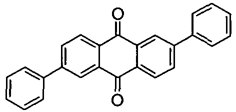 | 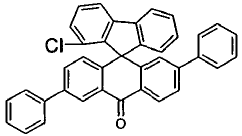 | 43 % * |
| | 1195886-14-9 | 131268-46-7 | | |

### Beispiel 1

### Synthese der Verbindung (1-1)

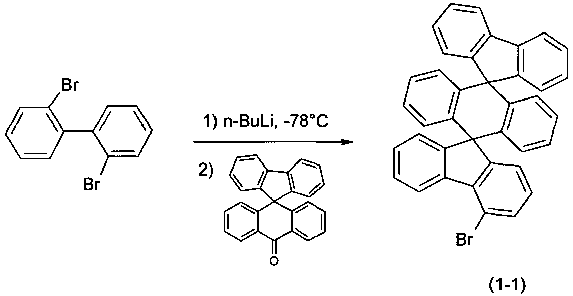

37,4 g (120 mmol) 2,2'-Dibromo-biphenyl werden in einem ausgeheizten Kolben in 300 mL getrocknetem THF gelöst. Die Reaktionsmischung wird auf -78°C gekühlt. Bei dieser Temperatur werden 75 mL einer 15%-igen Lösung n-BuLi in Hexan (119 mmol) langsam zugetropft. Der Ansatz wird 1 h bei -70°C nachgerührt. Anschließend werden 37,8 g des Keton-Derivates (CAS-Nr.: 717881-21-5) (110 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 500 ml Essigsäure versetzt und anschließend werden 90 ml rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Ausbeute beträgt 61,4 g (81% der Theorie).

Analog hierzu werden die folgenden Verbindungen (1-2) bis (1-10) hergestellt.

| | Edukt 1 | Edukt 2 | Produkt | Ausbeute |
|---|---|---|---|---|
| 1-2 | 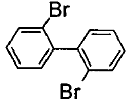 | 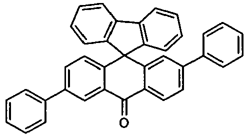 | 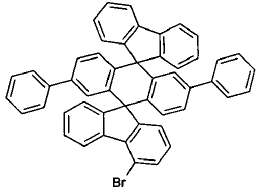 | 75% * |
| | 13029-09-9 | 1355363-51-7 | | |
| 1-3 | 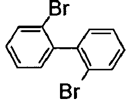 | 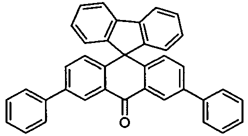 | 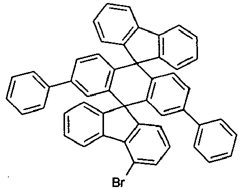 | 72 % |
| | 13029-09-9 | 1355363-60-8 | | |
| 1-4 | 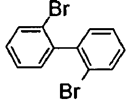 | 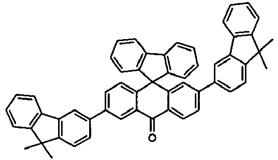 | 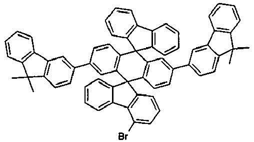 | 65% * |
| | 13029-09-9 | 1355363-56-2 | | |
| 1-5 | 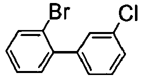 | 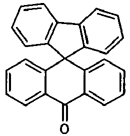 | 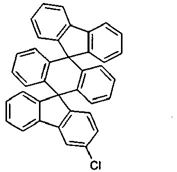 | 47% |
| | 154407-17-7 | 717881-21-5 | | |
| 1-6 | 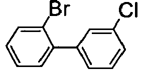 | 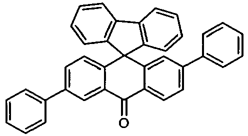 | 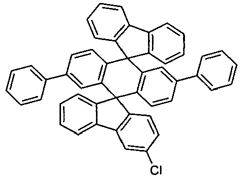 | 43% * |
| | 154407-17-7 | 1355363-51-7 | | |
| 1-7 | 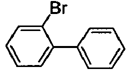 | 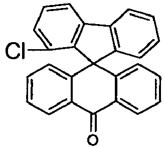 | 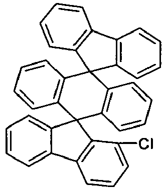 | 45% |
| | 2052-07-5 | | | |
| 1-8 | 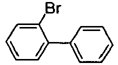 | 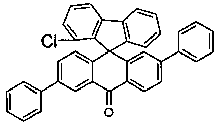 | 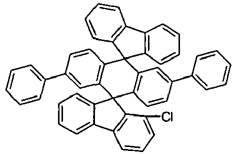 | 35% * |
| | 2052-07-5 | | | |
| 1-9 | 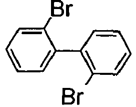 | 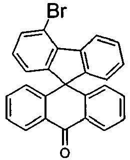 | 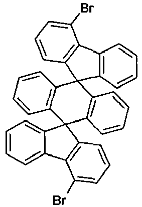 | 56%* |
| | 13029-09-9 | | | |
| 1-10 | 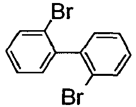 | 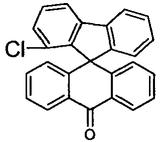 | 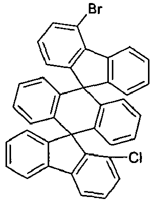 | 48%* |
| | 13029-09-9 | | | |

### Beispiel 2

### Synthese von Verbindung (2-1)

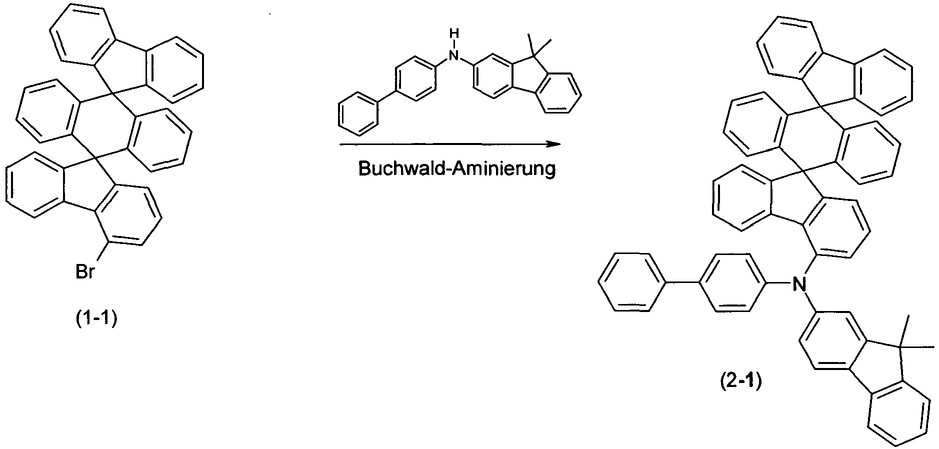

12,9 g Biphenyl-4-yl-(9,9-dimethyl-9H-fluoren-2-yl)-amine (36 mmol) und 20 g des Bromderivates (1-1) (36 mmol) werden in 600 mL Toluol gelöst: Die Lösung wird entgast und mit N₂ gesättigt. Danach wird sie mit 3,6 mL (3,6 mmol) einer 1 M Tri-tert-Butylphosphin-Lösung und 0,4 g (1,79 mmol) Palladium(II)acetat versetzt. Anschließend werden 8,59 g Natrium-tert-butylat (89,4 mmol) zugegeben. Die Reaktionsmischung wird 7h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99,9%. Die Ausbeute beträgt 25 g (85% der Theorie).

Analog hierzu werden die folgenden Verbindungen (2-2) bis (2-23) hergestellt:

| | Edukt 1 | Edukt 2 | Produkt | Ausbeute |
|---|---|---|---|---|
| 2-2 | 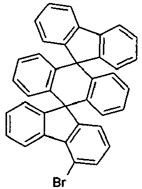 | 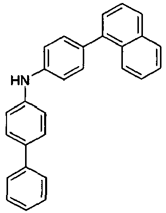 | 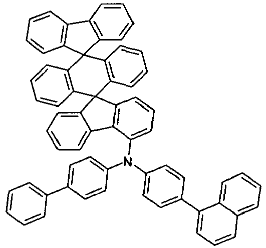 | 78% |
| | | 897921-59-4 | | |
| 2-3 | 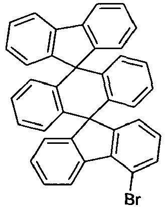 | 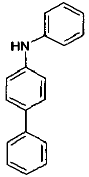 | 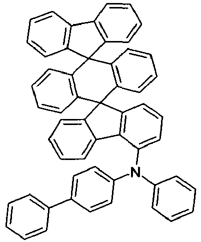 | 83% |
| | | 32228-99-2 | | |
| 2-4 | 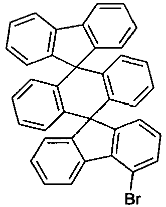 | 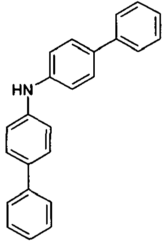 | 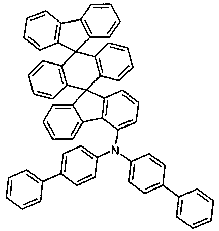 | 92% |
| | | 102113-98-4 | | |
| 2-5 | 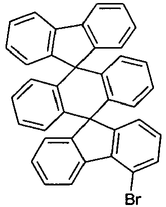 | 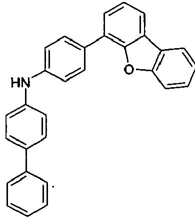 | 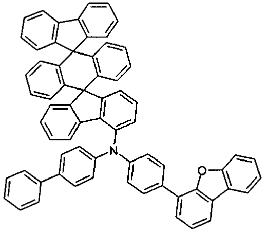 | 88% |
| | | 955959-89-4 | | |
| 2-6 | 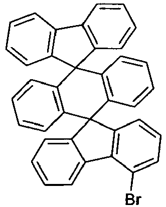 | 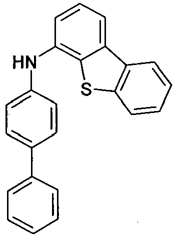 | 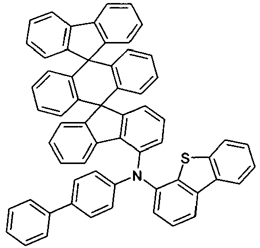 | 77% |
| | | 1448185-87-2 | | |
| 2-7 | 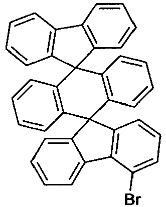 | 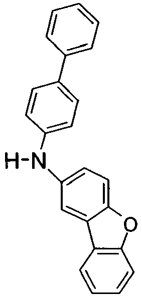 | 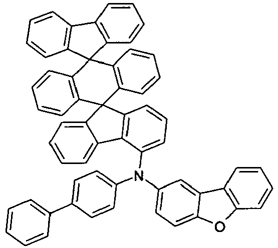 | 76% |
| | | 1300028-94-7 | | |
| 2-8 | 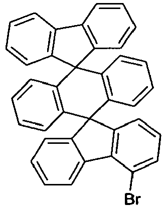 | 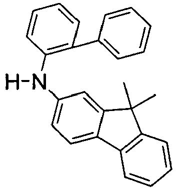 | 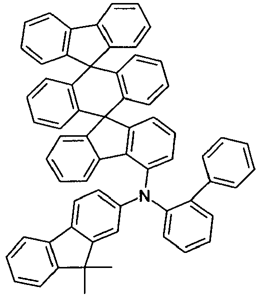 | 69% |
| | | 1198395-24-2 | | |
| 2-9 | 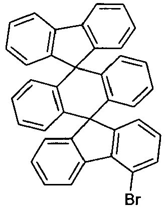 | 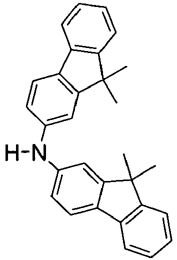 | 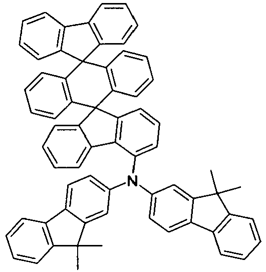 | 72% |
| | | 500717-23-7 | | |
| 2-10 | 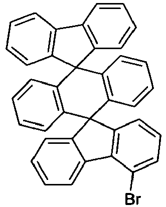 | 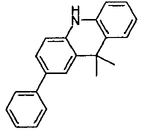 | 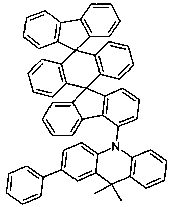 | 75% |
| 2-11 | 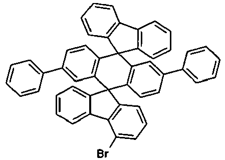 | 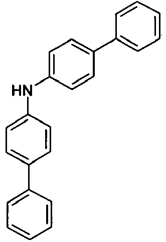 | 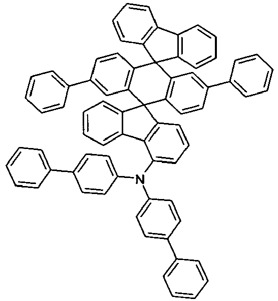 | 68%* |
| | | 102113-98-4 | | |
| 2-12 | 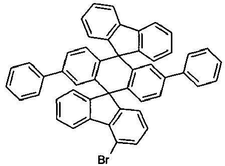 | 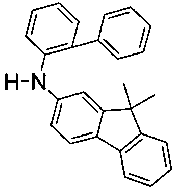 | 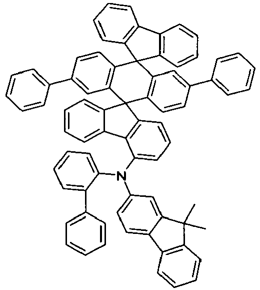 | 68%* |
| | | 1198395-24-2 | | |
| 2-13 | 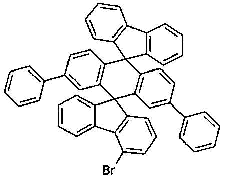 | 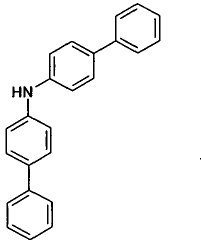 | 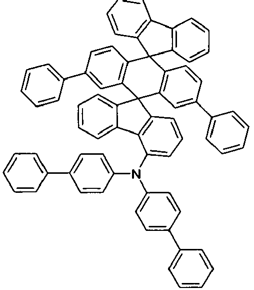 | 70% |
| | | 102113-98-4 | | |
| 2-14 | 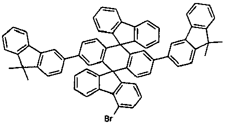 | 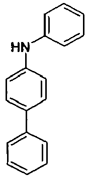 | 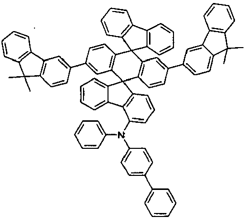 | 65%* |
| | | 32228-99-2 | | |
| 2-15 | 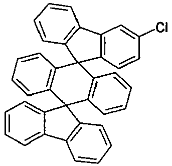 | 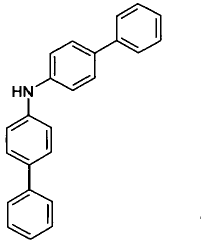 | 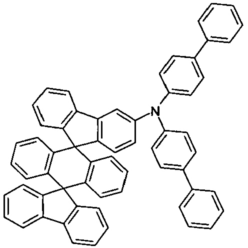 | 80% |
| | | 102113-98-4 | | |
| 2-16 | 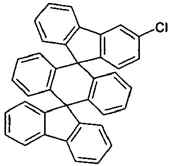 | 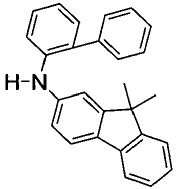 | 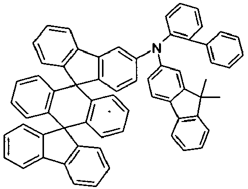 | 85% |
| | | 1198395-24-2 | | |
| 2-17 | 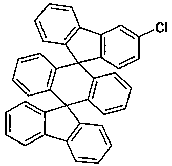 | 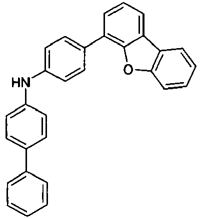 | 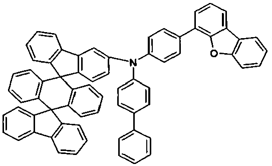 | 82% |
| | | 955959-89-4 | | |
| 2-18 | 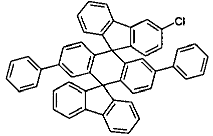 | 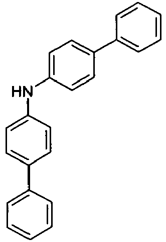 | 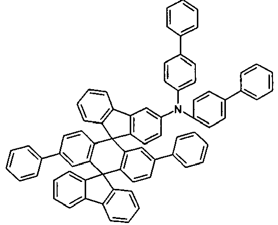 | 70%* |
| | | 102113-98-4 | | |
| 2-19 | 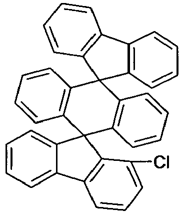 | 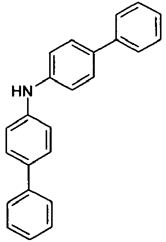 | 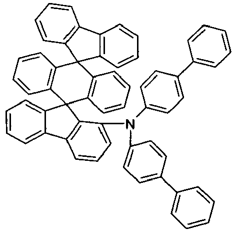 | 30% |
| | | 102113-98-4 | | |
| 2-20 | 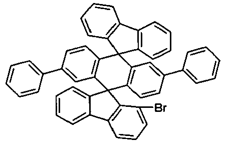 | 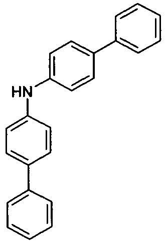 | 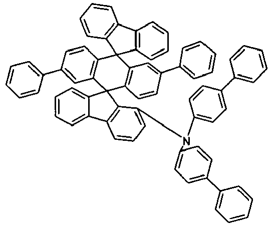 | 25%* |
| | | 102113-98-4 | | |
| 2-21 | 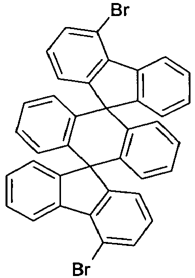 | 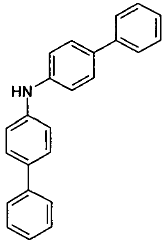 | 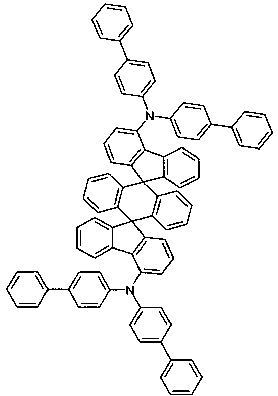 | 60%* |
| | | 102113-98-4 | | |
| | | 2 Äq. | | |
| 2-22 | 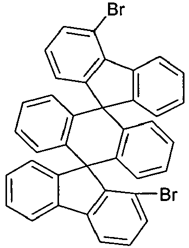 | 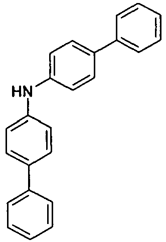 | 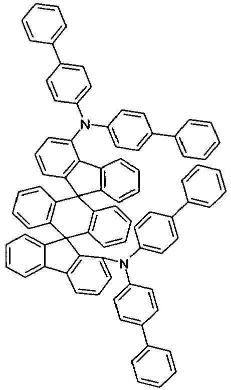 | 30%* |
| | | 102113-98-4 | | |
| | | 2 Äq. | | |
| 2-23 | 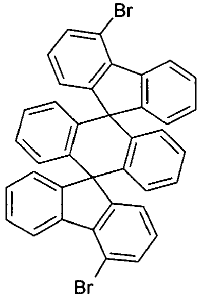 | 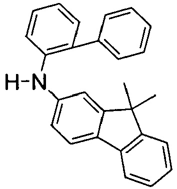 | 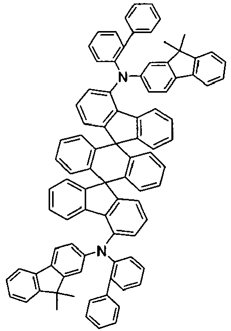 | 65%* |
| | | 1198395-24-2 | | |
| | | 2 Äq | | |

### Beispiel 3

### Synthese der Verbindung 3-1

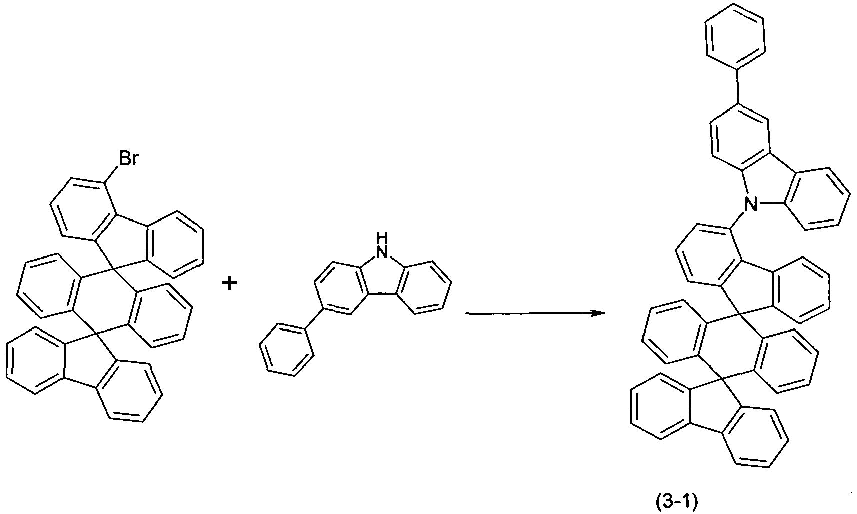

26,3 g (47 mmol) der Verbindung (1-1), 15 g (47 mmol) 3,6-Diphenyl-9-H-carbazol und 29,2 g Rb₂CO₃ werden in 250 mL p-Xylol suspendiert. Zu dieser Suspension werden 0,95 g (4,2 mmol) Pd(OAc)₂ und 12,6 ml einer 1M Tri-tert-butylphosphin Lösung gegeben. Die Reaktionsmischung wird 24 h unter Rückfluss erhitzt. Nach Erkalten wird die organische Phase abgetrennt, dreimal mit 150 mL Wasser gewaschen und anschließend zur Trockene eingeengt. Der Rückstand wird mit Toluol heiß extrahiert, aus Toluol/Heptan dreimal umkristallisiert und abschließend im Hochvakuum sublimiert, man erhält 18,5 g (25,6 mmol) entsprechend 54,5 % der Theorie. Die Reinheit beträgt 99,9 %.

Analog hierzu werden die folgenden Verbindungen (3-2) bis (3-5) hergestellt.

| | Edukt 1 | Edukt 2 | Produkt | Ausbeute |
|---|---|---|---|---|
| 3-2 | 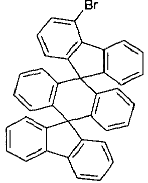 | 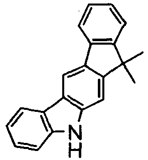 | 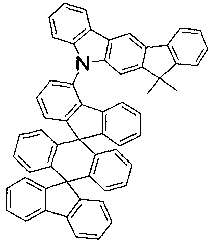 | 60% |
| | | 1257220-47-5 | | |
| 3-3* | 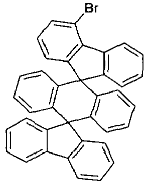 | 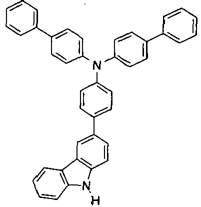 | 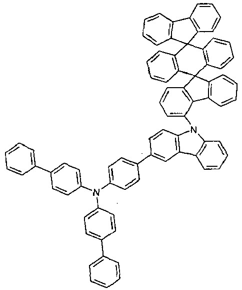 | 40% |
| | | 1221495-66-4 | * nicht anspruchsgemäß | |
| 3-4 | 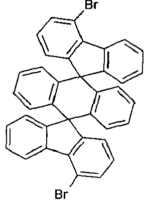 | 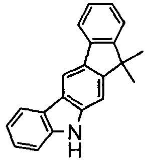 | 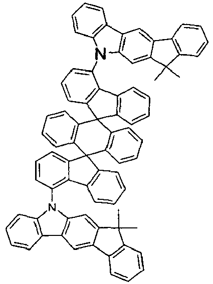 | 35%* |
| | | 1257220-47-5 2 Äq | | |
| 3-5 | 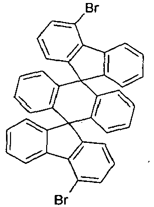 | 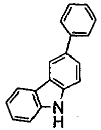 | 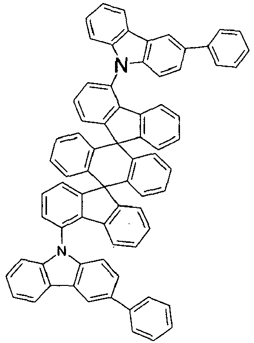 | 35%* |
| | | 103012-26-6 2 Äq | | |
| | | | | |

### Beispiel 4

### Synthese der Verbindung 4-1

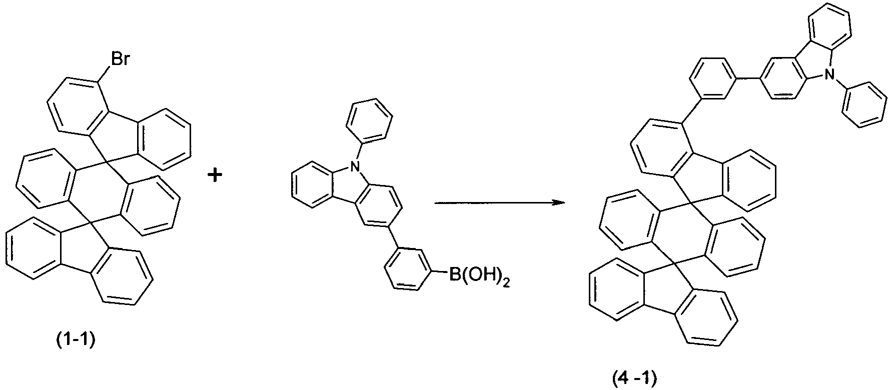

16,8 g (30 mmol) der Verbindung (1-1), 10 g (30 mmol) Carbazolboronsäure werden in 300 mL Dioxan und 9,1 g Caesiumfluorid (60 mmol) suspendiert. Zu dieser Suspension werden 2,2 g (3 mmol) Palladium dichlorid-bis(tricyclohexylphosphin) gegeben, und die Reaktionsmischung wird 18 h unter Rückfluss erhitzt. Nach Erkalten wird die organische Phase abgetrennt, über Kieselgel filtriert, dreimal mit 80 mL Wasser gewaschen und anschließend zur Trockene eingeengt. Der Rückstand wird mit Toluol heiß extrahiert, aus Toluol/Heptan dreimal umkristallisiert und abschließend im Hochvakuum sublimiert, man erhält 15,9 g entsprechend 66,4 % der Theorie. Die Reinheit beträgt 99,9 %.

Analog hierzu werden die folgenden Verbindungen (4-2) bis (4-14) hergestellt.

| | Edukt 1 | Edukt 2 | Produkt | Ausbeute |
|---|---|---|---|---|
| 4-2 | 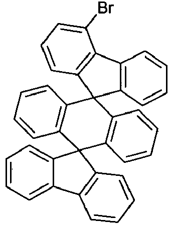 | 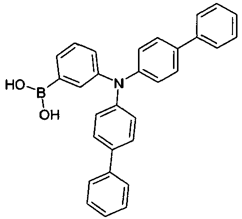 | 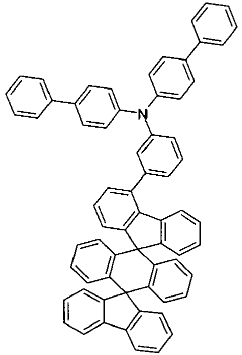 | 68% |
| 4-3 | 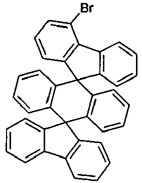 | 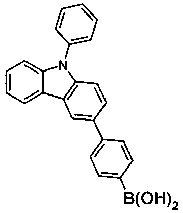 | 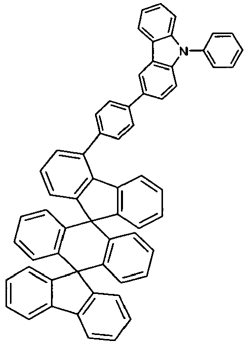 | 70% |
| | | 40963-55-6 | | |
| 4-4 | 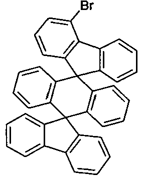 | 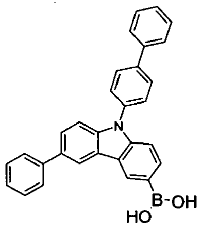 | 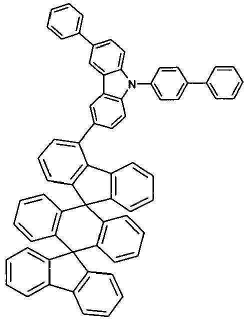 | 72% |
| | | 1316311-18-8 | | |
| 4-5 | 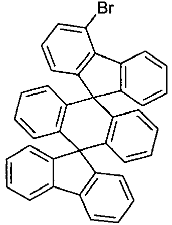 | 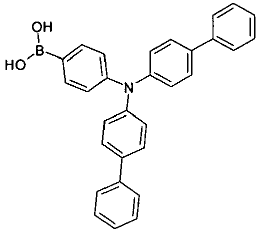 | 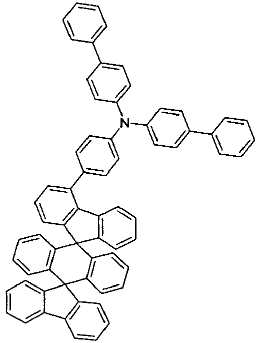 | 75% |
| | | 943836-24-6 | | |
| 4-6 | 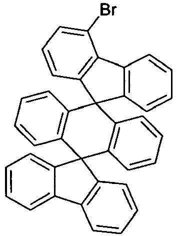 | 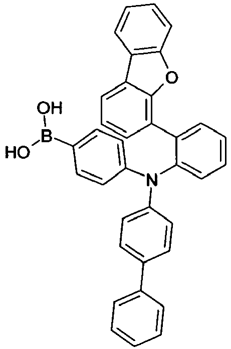 | 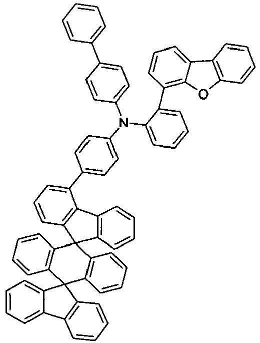 | 77% |
| 4-7 | 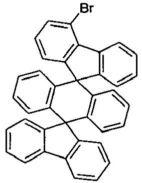 | 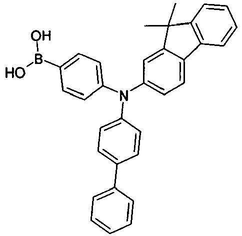 | 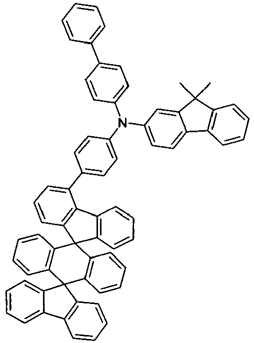 | 81% |
| | | 1265177-27-2 | | |
| 4-8 | 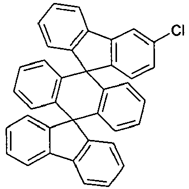 | 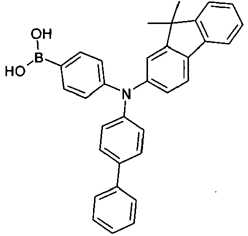 | 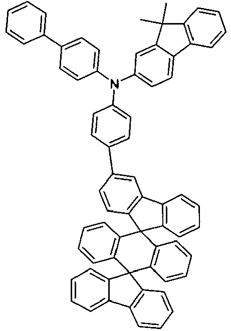 | 82% |
| | | 1265177-27-2 | | |
| 4-9 | 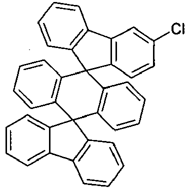 | 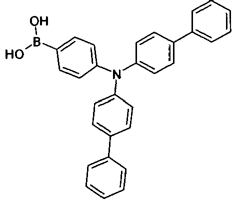 | 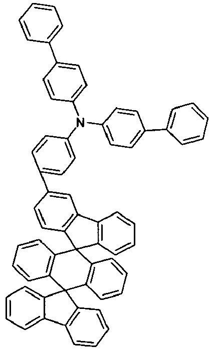 | 79% |
| | | 943836-24-6 | | |
| 4-10 | 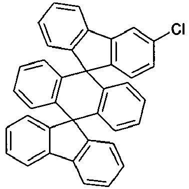 | 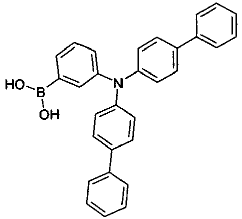 | 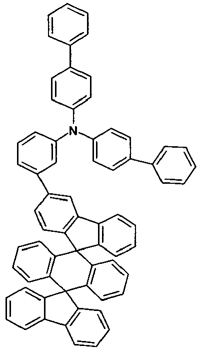 | 75% |
| 4-11 | 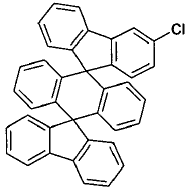 | 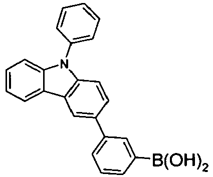 | 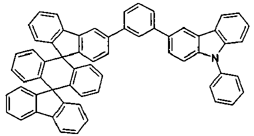 | 81% |
| | | 854952-60-6 | | |
| 4-12 | 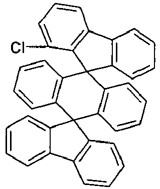 | 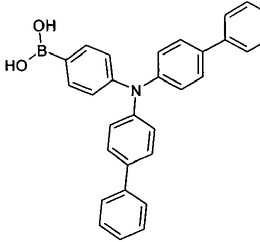 | 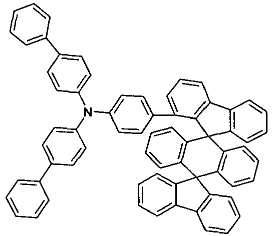 | 51% |
| | | 943836-24-6 | | |
| 4-13 | 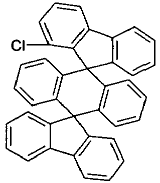 | 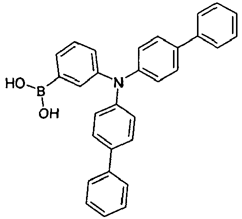 | 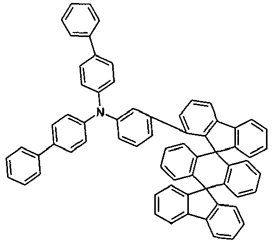 | 46% |
| 4-14 | 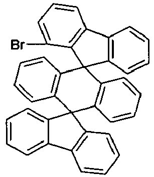 | 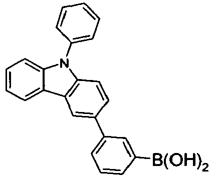 | 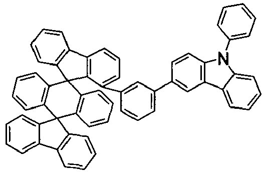 | 48% |
| | | 854952-60-6 | | |

### Teil B: Herstellung der OLEDs

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden erfinderischen Beispielen E1 bis E9 und in den Referenzbeispielen V1 werden die Daten verschiedener OLEDs vorgestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / p-dotierte Lochtransportschicht A' (HIL1) / Lochtransportschicht A (HTL) / p-dotierte Lochtransportschicht B (HIL2) / Lochtransportschicht C (EBL) / Emissionsschicht (EML) / Elektronentransportschicht (ETL) / Elektroneninjektionsschicht (EIL) und abschließend eine Kathode. Die Kathode wird durch eine 100 nm dicke Aluminiumschicht gebildet. Die zur Herstellung der OLEDs benötigten Materialien sind in Tabelle 1 gezeigt, der Aufbau der verschiedenen hergestellten elektronischen Vorrichtungen in Tabelle 2.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1 :SEB(5%) bedeutet hierbei, dass das Material H1 in einem Volumenanteil von 95% und SEB in einem Anteil von 5% in der Schicht vorliegt. Analog können auch die Elektronentransportschicht oder die Lochinjektionsschichten aus einer Mischung von zwei Materialien bestehen. Eine Angabe wie H2:H3 (60%):TEG(10%) bedeutet hierbei, dass das Material H2 in einem Volumenanteil von 30%, H3 in einem Volumenanteil von 60% und TEG in einem Anteil von 10% in der Schicht vorliegt.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 2 mA/cm² bezeichnet die externe Quanteneffizienz bei einer Stromdichte von 2 mA/cm². LD80 @ 50 mA/cm² ist die Lebensdauer, bis das OLED bei konstantem Strom auf 80 % der Anfangsintensität abgefallen ist.

| Tabelle 1: Strukturen der verwendeten Materialien | | |
|---|---|---|
| 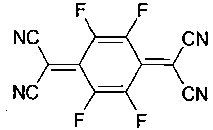 | 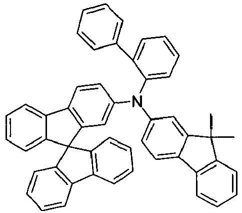 | 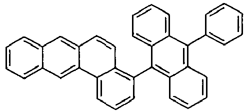 |
| **F4TCNQ** | **HIM** | **H1** |
| 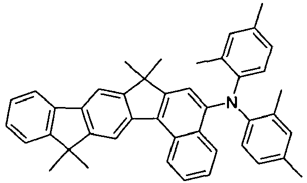 | 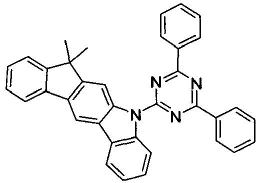 | 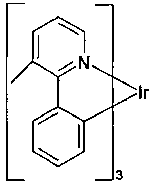 |
| **SEB** | **H2** | **TEG** |
| 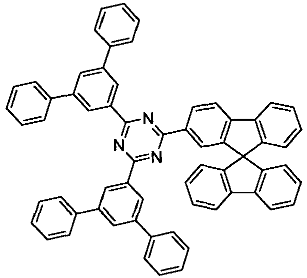 | 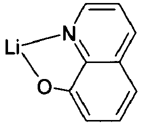 | |
| **ETM** | **LiQ** | |
| 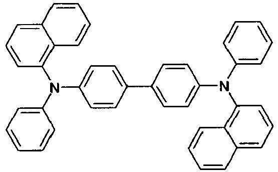 | 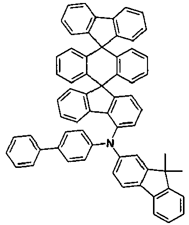 | 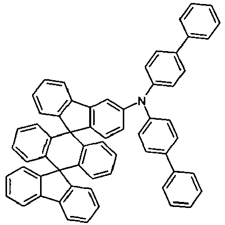 |
| **NPB** | **HTM1** | **HTM2** |
| 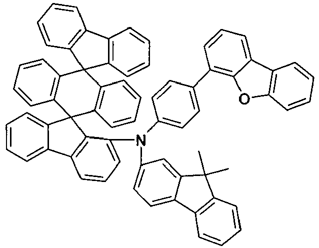 | 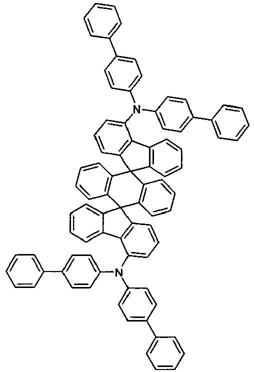 | 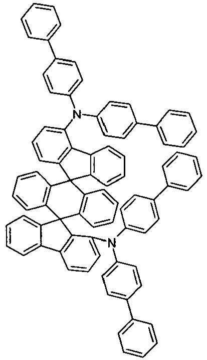 |
| **HTM3** | **HTM4** | **HTM5** |
| 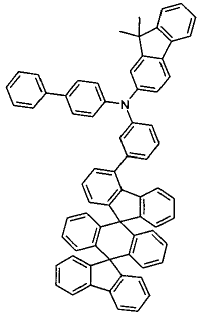 | 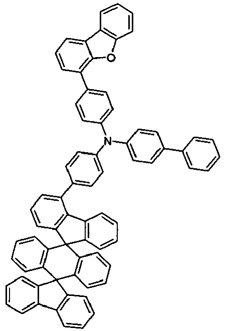 | 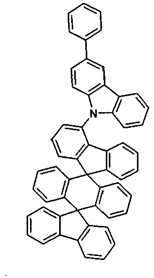 |
| **HTM6** | **HTM7** | **H3** |
| 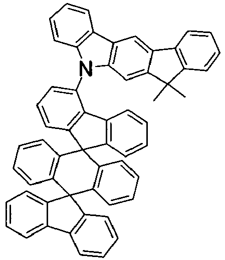 | | |
| **H4** | | |

| Tabelle 2: Aufbau der OLEDs | | | | | | | |
|---|---|---|---|---|---|---|---|
| Bsp | HIL1 | HTL | HIL2 | EBL | EML | ETL | EIL |
| | Dicke / nm | Dicke / nm | Dicke / nm | Dicke/nm | Dicke / nm | Dicke / nm | Dicke / nm |
| V1 | HIM1 :F4TCNQ (3%) 20 nm | HIM1 155 nm | NPB:F4TCNQ (3%) 20 nm | NPB 20 nm | H1:SEB(5%) 20 nm | ETM(50%):LiQ (50%) 30 nm | LiQ 1nm |
| E1 | HIM1 :F4TCNQ (3%) 20 nm | HIM1 155 nm | HTM1:F4TCNQ (3%) 20 nm | HTM1 20 nm | H1 :SEB(5%) 20 nm | ETM(50%):LiQ (50%) 30 nm | LiQ 1 nm |
| E2 | HIM1:F4TCNQ (3%) 20 nm | HIM1 155 nm | HTM2:F4TCNQ (3%) 20 nm | HTM2 20 nm | H1:SEB(5%) 20 nm | ETM(50%):LiQ (50%) 30 nm | LiQ 1 nm |
| E3 | HIM1:F4TCNQ (3%) 20 nm | HIM1 155 nm | HTM3:F4TCNQ (3%) 20 nm | HTM3 20 nm | H1:SEB(5%) 20 nm | ETM(50%):LiQ (50%) 30 nm | LiQ 1 nm |
| E4 | HIM1:F4TCNQ (3%) 20 nm | HIM1 155 nm | HTM4:F4TCNQ (3%) 20 nm | HTM4 20 nm | H1:SEB(5%) 20 nm | ETM(50%):LiQ (50%) 30 nm | LiQ 1 nm |
| E5 | HIM1:F4TCNQ (3%) 20 nm | HIM1 155 nm | HTM5:F4TCNQ (3%) 20 nm | HTM5 20 nm | H1:SEB(5%) 20 nm | ETM(50%):LiQ (50%) 30 nm | LiQ 1 nm |
| E6 | HIM1:F4TCNQ (3%) 20 nm | HIM1 155 nm | HTM6:F4TCNQ (3%) 20 nm | HTM6 20 nm | H1:SEB(5%) 20 nm | ETM(50%):LiQ (50%) 30 nm | LiQ 1 nm |
| E7 | HIM1 :F4TCNQ (3%) 20 nm | HIM1 155 nm | HTM7:F4TCNQ (3%) 20 nm | HTM7 20 nm | H1:SEB(5%) 20 nm | ETM(50%):LiQ (50%) 30 nm | LiQ 1 nm |
| V2 | HIM1:F4TCNQ (3%) 20 nm | HIM1 210 nm | HIM1:F4TCNQ (3%) 20 nm | HIM1 20 nm | H2:TEG(10% ) 30 nm | ETM(50%):LiQ (50%) 40 nm | LiQ 1 nm |
| E8 | HIM1:F4TCNQ (3%) 20 nm | HIM1 210 nm | HIM1:F4TCNQ (3%) 20 nm | HIM1 20 nm | H2:H3(60%): TEG(10%) 30 nm | ETM(50%):LiQ (50%) 40 nm | LiQ 1 nm |
| E9 | HIM1:F4TCNQ (3%) 20 nm | HIM1 210 nm | HIM1:F4TCNQ (3%) 20 nm | HIM1 20 nm | H2:H4(60%): TEG(10%) 30 nm | ETM(50%):LiQ (50%) 40 nm | LiQ 1 nm |

### Beispiel 1

In einerm OLED mit blauer Singulettemission zeigen im Vergleich zu der Referenzprobe V1 (6,2 %) die erfindungsgemäßen Proben E1 (6,6 %), E2 (7,6 %), E3 (8,5 %), E4 (7,5 %), E5 (7,5 %), E6 (7,3 %) und E7 (7,1 %) höhere Quanteneffizienzen bei 10 mA/cm². Die Lebensdauer LT80 bei 50 mA/cm² ist bei den erfindungsgemäßen Proben E1 (390 h), E2 (370 h), E3 (380 h), E4 (305 h), E5 (220 h), E6 (300 h) und E7 (440 h) auch deutlich besser bei den Referenzproben V1 (135 h).

### Beispiel 2

Es wurde eine grüne phosphoreszierende Referenzprobe V2 hergestellt und mit den erfindungsgemäßen Proben E8 und E9 verglichen. Die Referenzprobe V2 hat bei einer Stromdichte von 2 mA/cm² eine externe Quanteneffizienz von 19,8 % und eine Lebensdauer (LD80 @ 20 mA/cm²) von 135 h. Verglichen damit haben die erfindungsgemäßen Proben E8 (19,2 %) und E9 (19,3 %) vergleichbare Quanteneffizienzen und deutliche bessere Lebensdauern von 310 h (E8) und 270 h (E9).

## Patentansprüche

1. Verbindung gemäß Formel (1), 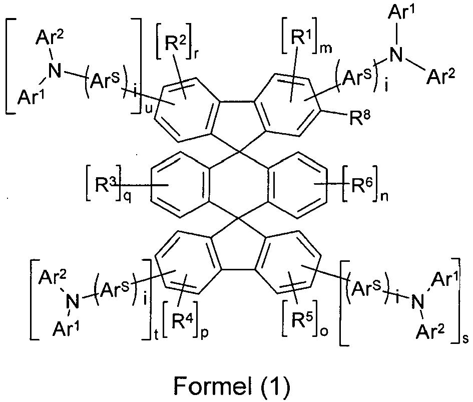 wobei für die auftretenden Symbole und Indizes gilt:
Ar^{S} ist gleich oder verschieden bei jedem Auftreten ein aromatisches oder heteroaromatisches Ringsystem mit 6 bis 60 C-Atomen, das jeweils auch mit einem oder mehreren Resten R⁷ substituiert sein kann; dabei kann Ar^{S} mit Ar¹ und/oder mit Ar² durch eine Gruppe E verbunden sein;
Ar¹, Ar² sind gleich oder verschieden bei jedem Auftreten ein aromatisches oder heteroaromatisches Ringsystem mit 6 bis 60 aromatischen Ringatomen, das jeweils auch mit einem oder mehreren Reste R⁷ substituiert sein kann, dabei können Ar¹ und Ar² miteinander und/oder Ar¹ mit Ar^{S} und/oder Ar² mit Ar^{S} durch eine Gruppe E verbunden sein;
oder die Gruppe -NAr¹Ar² weist eine Struktur gemäß einer der Formeln (Ar1-13) bis (Ar1-19) auf; 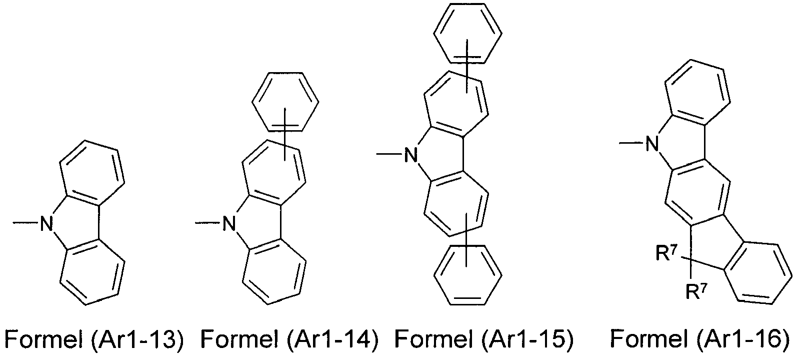 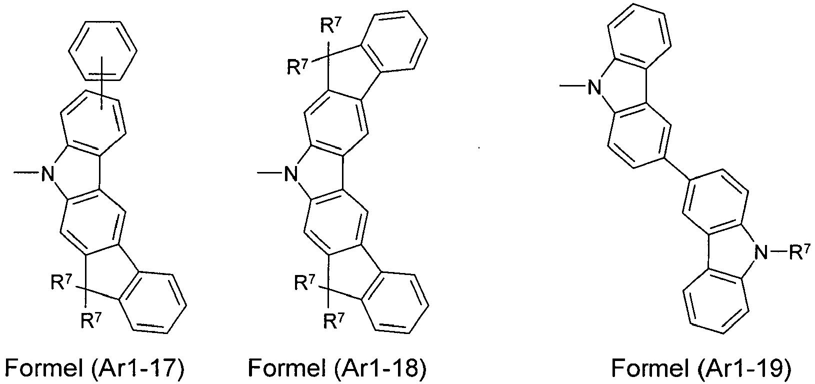 wobei die Gruppen der Formeln (Ar1-13) bis (Ar1-19) an den freien Positionen mit R⁷ substituiert sein können;
oder die Gruppe -Ar^{S}-NAr¹Ar² weist eine Struktur einer der Formeln (Ar2-5) bis (Ar2-9) auf 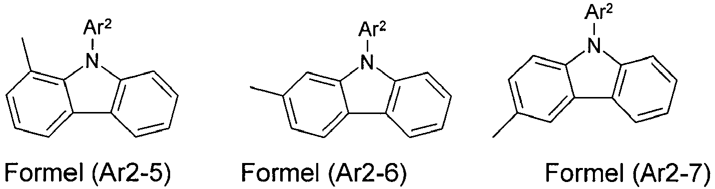 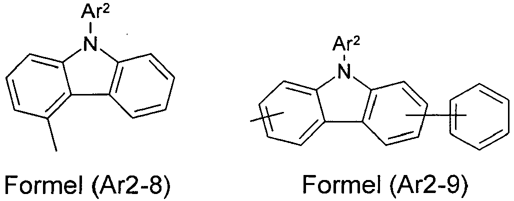 wobei die Gruppen der Formeln (Ar2-5) bis (Ar2-9) an den freien Positionen mit R⁷ substituiert sein können;
E ist gleich oder verschieden bei jedem Auftreten ausgewählt aus der Gruppe bestehend aus C(R⁷)₂, O, S und NR⁷;
R¹, R², R³, R⁴, R⁵, R⁶, sind bei jedem Auftreten gleich oder verschieden ausgewählt aus der Gruppe bestehend aus H, D, F, Cl, Br, I, CN, Si(R⁹)₃, einer geradkettigen Alkyl-, Alkoxy- oder Thioalkylgruppe mit 1 bis 40 C- Atomen oder einer verzweigten oder cyclischen Alkyl-, Alkoxy- oder Thioalkylgruppe mit 3 bis 40 C-Atomen, die jeweils mit einem oder mehreren Resten R⁹ substituiert sein kann, wobei jeweils eine oder mehrere nicht-benachbarte CH₂-Gruppen durch Si(R⁹)₂, C=NR⁹, P(=O)(R⁹), SO, SO₂, NR⁹, O, S oder CONR⁹ ersetzt sein können und wobei ein oder mehrere H-Atome durch D, F, Cl, Br oder I ersetzt sein können, einem aromatischen oder heteroaromatischen Ringsystem mit 6 bis 60 aromatischen Ringatomen, das jeweils mit einem oder mehreren Resten R⁹ substituiert sein kann, einer Aryloxygruppe oder Heteroaryloxygruppe mit 5 bis 60 aromatischen Ringatomen, die mit einem oder mehreren Resten R⁹ substituiert sein kann, oder einer Aralkylgruppe oder Heteroaralkylgruppe mit 5 bis 60 aromatischen Ringatomen, die jeweils mit einem oder mehreren Resten R⁹ substituiert sein kann, wobei optional zwei oder mehr benachbarte Substituenten R¹ bzw. zwei oder mehr benachbarte Substituenten R² bzw. zwei oder mehr benachbarte Substituenten R³ bzw. zwei oder mehr benachbarte Substituenten R⁴ bzw. zwei oder mehr benachbarte Substituenten R⁵ bzw. zwei oder mehr benachbarte Substituenten R⁶ ein mono- oder polycyclisches, aliphatisches oder aromatisches Ringsystem bilden können, das mit einem oder mehreren Resten R⁹ substituiert sein kann;
R⁷ ist bei jedem Auftreten gleich oder verschieden ausgewählt aus der Gruppe bestehend aus H, D, F, Cl, Br, I, CN, Si(R⁹)₃, einer geradkettigen Alkyl-, Alkoxy- oder Thioalkylgruppe mit 1 bis 40 C-Atomen oder einer verzweigten oder cyclischen Alkyl-, Alkoxy-oder Thioalkylgruppe mit 3 bis 40 C-Atomen, die jeweils mit einem oder mehreren Resten R⁹ substituiert sein kann, wobei jeweils eine oder mehrere nicht-benachbarte CH₂-Gruppen durch Si(R⁹)₂, C=NR⁹, P(=O)(R⁹), SO, SO₂, NR⁹, O, S oder CONR⁹ ersetzt sein können und wobei ein oder mehrere H-Atome durch D, F, Cl, Br oder I ersetzt sein können, einem aromatischen oder heteroaromatischen Ringsystem mit 6 bis 60 aromatischen Ringatomen, das jeweils mit einem oder mehreren Resten R⁹ substituiert sein kann, einer Aryloxygruppe oder Heteroaryloxygruppe mit 5 bis 60 aromatischen Ringatomen, die mit einem oder mehreren Resten R⁹ substituiert sein kann, oder einer Aralkylgruppe oder Heteroaralkylgruppe mit 5 bis 60 aromatischen Ringatomen, die jeweils mit einem oder mehreren Resten R⁹ substituiert sein kann, wobei optional zwei oder mehr benachbarte Substituenten R⁷ ein mono- oder polycyclisches, aliphatisches oder aromatisches Ringsystem bilden können, das mit einem oder mehreren Resten R⁹ substituiert sein kann;
R⁸ ist ausgewählt aus der Gruppe bestehend aus H, D, F, Cl, Br, I, CN, Si(R⁹)₃, einer geradkettigen Alkyl-, Alkoxy- oder Thioalkylgruppe mit 1 bis 40 C- Atomen oder einer verzweigten oder cyclischen Alkyl-, Alkoxy- oder Thioalkylgruppe mit 3 bis 40 C-Atomen, die jeweils mit einem oder mehreren Resten R⁹ substituiert sein kann, wobei jeweils eine oder mehrere nicht-benachbarte CH₂-Gruppen durch Si(R⁹)₂, C=NR⁹, P(=O)(R⁹), SO, SO₂, NR⁹, O, S oder CONR⁹ ersetzt sein können und wobei ein oder mehrere H-Atome durch D, F, Cl, Br oder I ersetzt sein können, einem aromatischen oder heteroaromatischen Ringsystem mit 6 bis 60 aromatischen Ringatomen, das jeweils mit einem oder mehreren Resten R⁹ substituiert sein kann, einer Aryloxygruppe oder Heteroaryloxygruppe mit 5 bis 60 aromatischen Ringatomen, die mit einem oder mehreren Resten R⁹ substituiert sein kann, oder einer Aralkylgruppe oder Heteroaralkylgruppe mit 5 bis 60 aromatischen Ringatomen, die jeweils mit einem oder mehreren Resten R⁹ substituiert sein kann, wobei optional ein Substituent R⁸ und ein benachbarter Substituent R¹ ein mono- oder polycyclisches, aliphatisches oder aromatisches Ringsystem bilden können, das mit einem oder mehreren Resten R⁹ substituiert sein kann;
R⁹ ist bei jedem Auftreten gleich oder verschieden ausgewählt aus der Gruppe bestehend aus H, D, F, Cl, Br, I, Si(R¹⁰)₃, einer geradkettigen Alkyl-, Alkoxy- oder Thioalkylgruppe mit 1 bis 40 C-Atomen oder einer verzweigten oder cyclischen Alkyl-, Alkoxy-oder Thioalkylgruppe mit 3 bis 40 C-Atomen, die jeweils mit einem oder mehreren Resten R¹⁰ substituiert sein kann, wobei eine oder mehrere nicht-benachbarte CH₂-Gruppen durch Si(R¹⁰)₂, C=NR¹⁰, P(=O)(R¹⁰), SO, SO₂, NR¹⁰, O, S oder CONR¹⁰ ersetzt sein können und wobei ein oder mehrere H-Atome durch D, F, Cl, Br oder I ersetzt sein können, einem aromatischen oder heteroaromatischen Ringsystem mit 6 bis 60 aromatischen Ringatomen, das jeweils mit einem oder mehreren Resten R¹⁰ substituiert sein kann, einer Aryloxygruppe oder Heteroaryloxygruppe mit 5 bis 60 aromatischen Ringatomen, die mit einem oder mehreren Resten R¹⁰ substituiert sein kann, oder einer Aralkylgruppe oder Heteroaralkylgruppe mit 5 bis 60 aromatischen oder heteroaromatischen Ringatomen, die mit einem oder mehreren Resten R¹⁰ substituiert sein kann, wobei optional zwei oder mehr benachbarte Substituenten R⁹ ein mono- oder polycyclisches, aliphatisches Ringsystem bilden können, das mit einem oder mehreren Resten R¹⁰ substituiert sein kann;
R¹⁰ ist ausgewählt aus der Gruppe bestehend aus H, D, F, einem aliphatischem Kohlenwasserstoffrest mit 1 bis 20 C-Atomen, einem aromatischem Ringsystem mit 6 bis 30 C-Atomen, in dem ein oder mehrere H-Atome durch D oder F ersetzt sein können, wobei zwei oder mehr benachbarte Substituenten R¹⁰ miteinander ein mono-oder polycyclisches, aliphatisches, Ringsystem bilden können;
i ist bei jedem Auftreten 0, 1 oder 2;
m ist 0, 1 oder 2;
n, o, p, q, r sind bei jedem Auftreten gleich oder verschieden 0, 1, 2, 3 oder 4;
s, t, u sind bei jedem Auftreten gleich oder verschieden 0, 1 oder 2;
dabei gilt s+o ≤ 4, p+t ≤ 4 und, r+u ≤ 4;
und weiterhin gilt u+t+s ≤ 2.

2. Verbindung nach Anspruch 1 gemäß der Formel (2), (3), (4), (5), 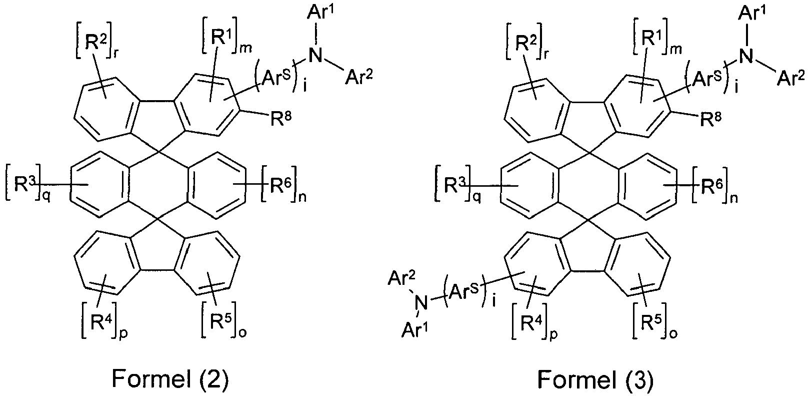 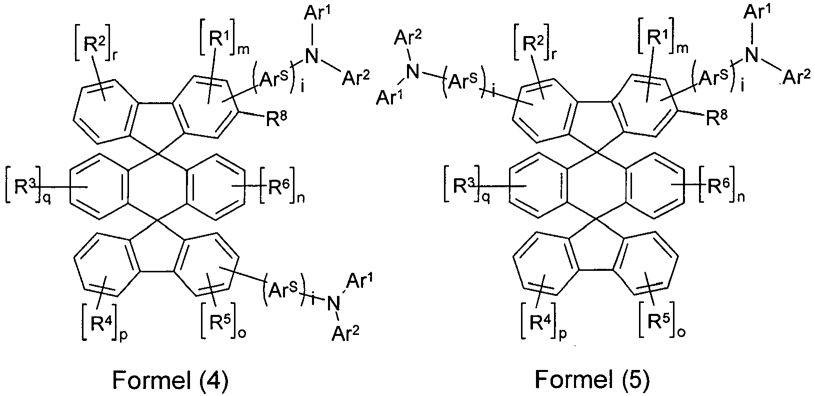 wobei die Symbole und Indizes die in Anspruch 1 genannten Bedeutungen haben.

3. Verbindung nach Anspruch 1 oder 2, **dadurch gekennzeichnet, dass** die Gruppen Ar¹ und Ar² gleich oder verschieden bei jedem Auftreten ausgewählt sind aus den Gruppen der Formeln (Ar-1) bis (Ar-33) 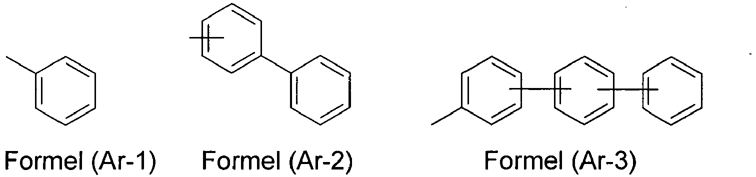 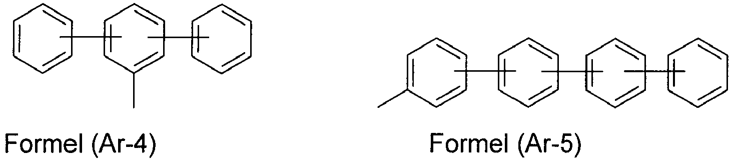 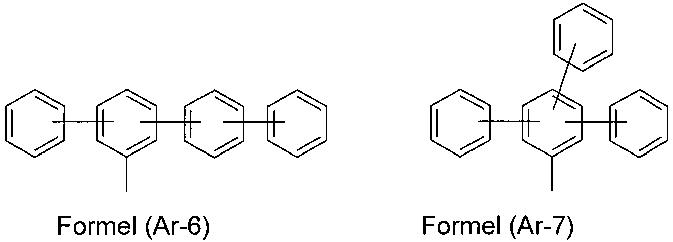 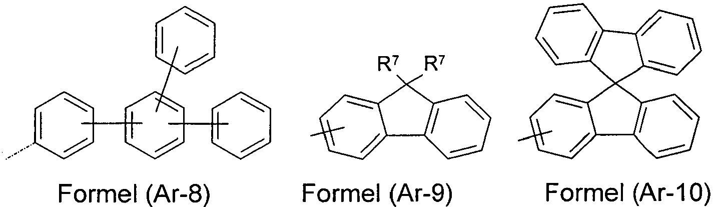 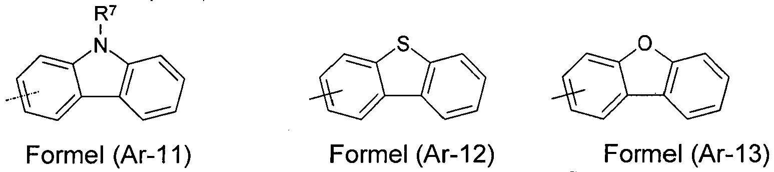 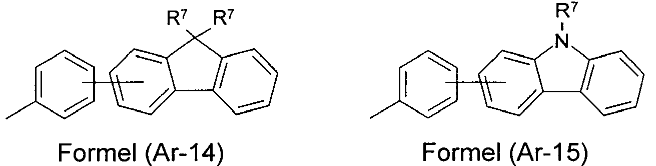 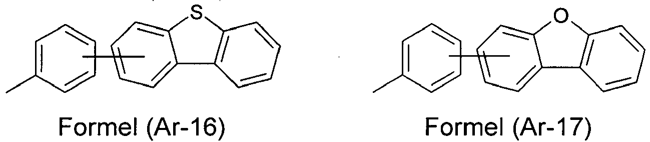 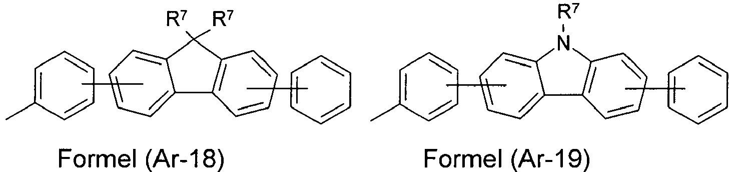 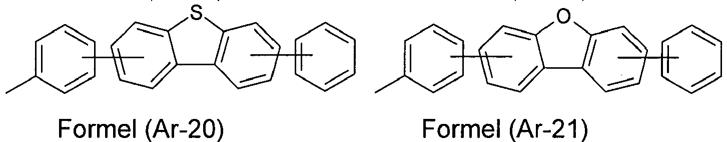 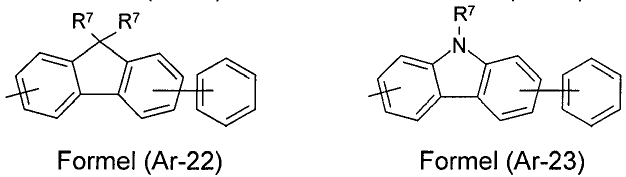 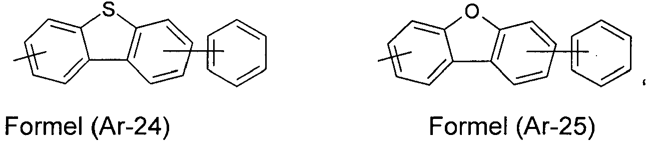 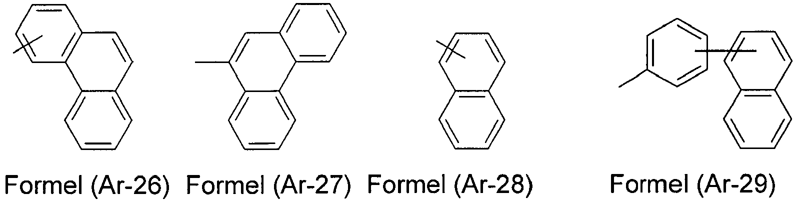 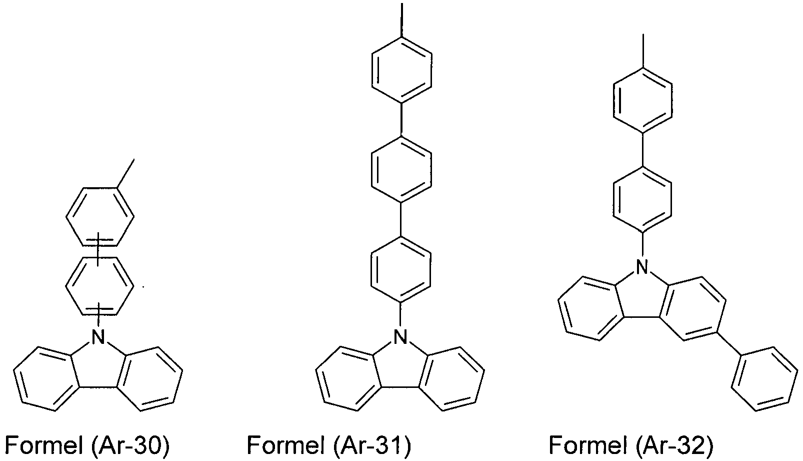 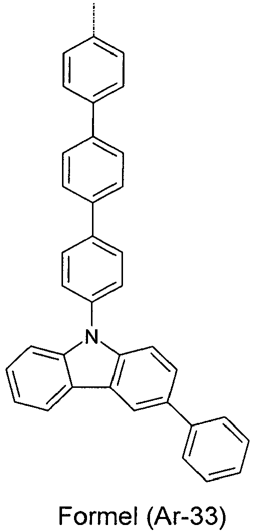 wobei die verwendeten Symbole die in Anspruch 1 genannten Bedeutungen haben und die Gruppen an den freien Positionen mit R⁷ substituiert sein können.

4. Verbindung nach einem oder mehreren der Ansprüche 1 bis 3, **dadurch gekennzeichnet, dass** die Gruppe -NAr¹Ar² eine Struktur gemäß einer der folgenden Formeln (Ar1-1) bis (Ar1-19) aufweist 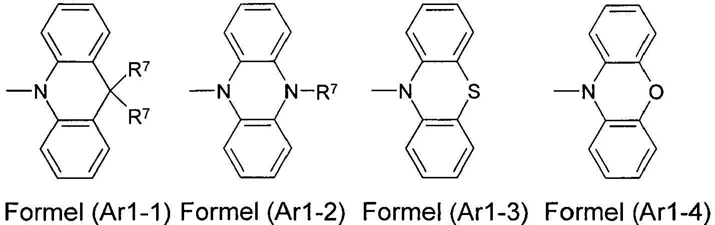 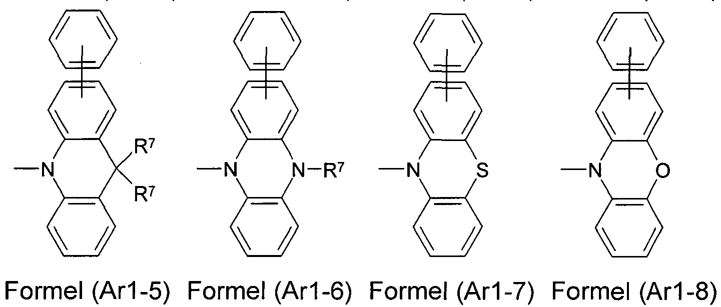 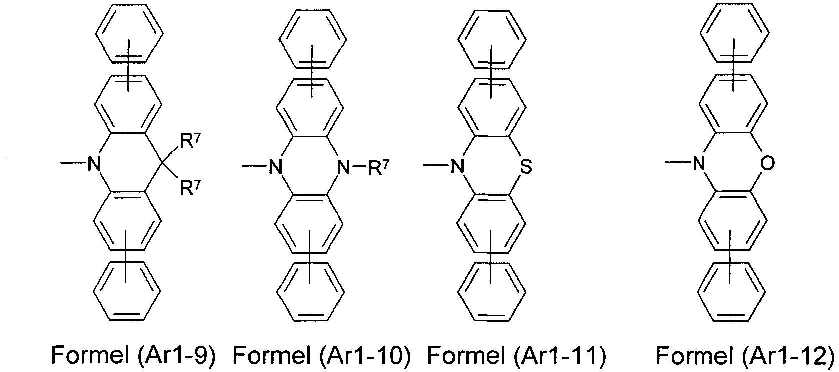 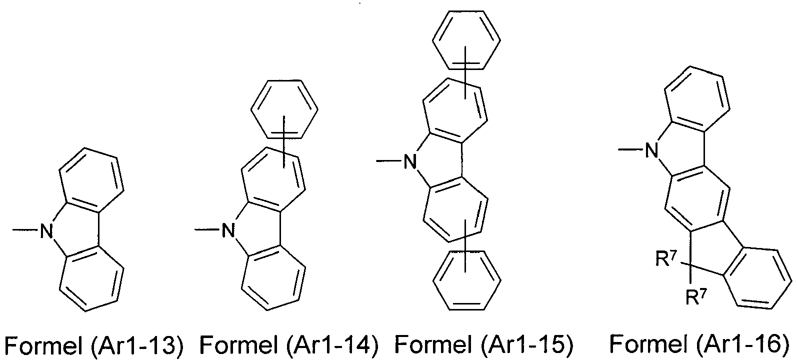 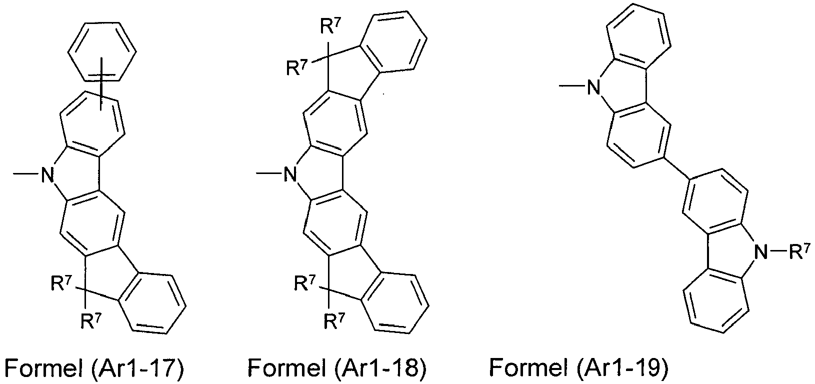 wobei die verwendeten Symbole die in Anspruch 1 genannten Bedeutungen haben und die Gruppen an den freien Positionen mit R⁷ substituiert sein können
oder dass die Gruppe -Ar^{S}-NAr¹Ar² die Struktur einer der folgenden Formeln (Ar2-1) bis (Ar2-9) aufweist 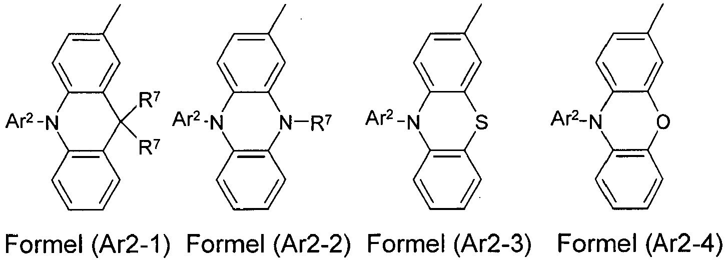 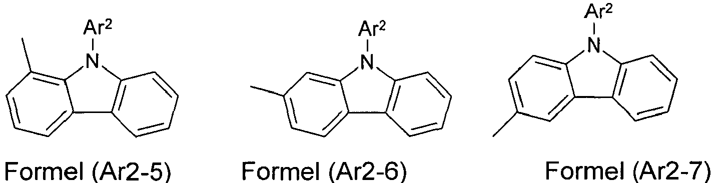 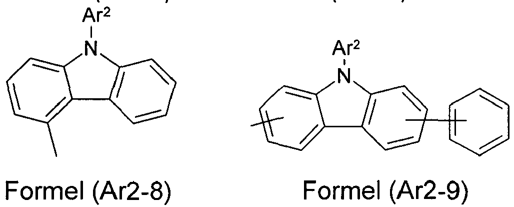 wobei die verwendeten Symbole die in Anspruch 1 genannten Bedeutungen aufweisen und die Gruppen an den freien Positionen mit R⁷ substituiert sein können.

5. Verbindung nach einem oder mehreren der Ansprüche 1 bis 4, **dadurch gekennzeichnet, dass** die Gruppe Ar^{S} für eine Gruppe gemäß einer der folgenden Formeln (Ar3-1) bis (Ar3-12) steht, 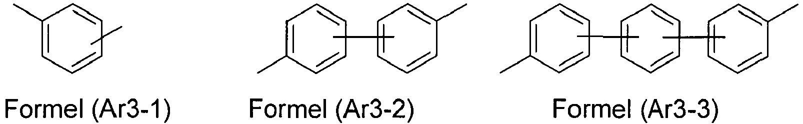 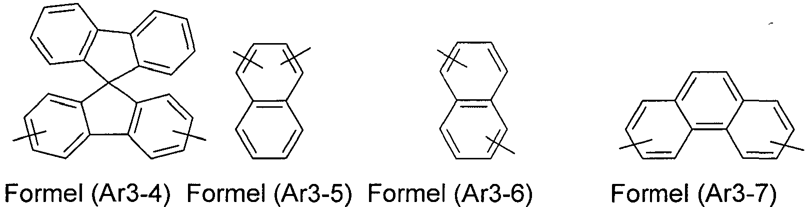 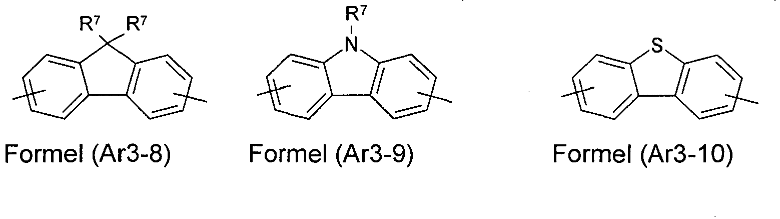 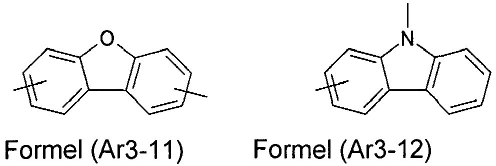 wobei die verwendeten Symbole die in Anspruch 1 genannten Bedeutungen aufweisen und die Gruppen an den freien Positionen mit R⁷ substituiert sein können.

6. Verbindung nach einem oder mehreren der Ansprüche 1 bis 5, **dadurch gekennzeichnet, dass** R¹, R², R³, R⁴, R⁵, R⁶, R⁷, R⁸ gleich oder verschieden sind und bei jedem Auftreten ausgewählt aus der Gruppe bestehend aus H, D, F, Si(R⁹)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R⁹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 aromatischen Ringatomen, das jeweils mit einem oder mehreren Resten R⁹ substituiert sein kann, wobei optional zwei oder mehr benachbarte Substituenten R¹ bis R⁷ ein mono- oder polycyclisches, aliphatisches Ringsystem bilden können, das mit einem oder mehreren Resten R⁹ substituiert sein kann.

7. Verbindung nach einem oder mehreren der Ansprüche 1 bis 6 für die gilt:
Ar^{S} ist gleich oder verschieden bei jedem Auftreten ein aromatisches oder heteroaromatisches Ringsystem, wobei für i=1 oder 2 Ar^{S} ausgewählt ist aus den Gruppen der Formel (Ar3-1) bis (Ar3-12), wobei Ar^{S} mit Ar¹ durch eine Gruppe E verbunden sein kann;
Ar¹, Ar² sind gleich oder verschieden bei jedem Auftreten ein aromatisches oder heteroaromatisches Ringsystem ausgewählt aus den Gruppen der Formel (Ar-1) bis (Ar-33);
oder -NAr¹Ar² steht für eine Gruppe gemäß einer der Formeln (Ar1-1) bis (Ar1-19);
oder Ar^{S}-NAr¹-Ar² steht für eine Gruppe gemäß einer der Formeln (Ar2-1) bis (Ar2-9);
E ist gleich oder verschieden bei jedem Auftreten ausgewählt aus der Gruppe bestehend aus C(R⁷)₂, O, S und NR⁷;
R¹, R², R³, R⁴, R⁵, R⁶, R⁸ sind gleich oder verschieden bei jedem Auftreten ausgewählt aus der Gruppe bestehend aus H, D, F, Si(R⁹)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R⁹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 aromatischen Ringatomen, das jeweils mit einem oder mehreren Resten R⁹ substituiert sein kann, wobei optional zwei oder mehr benachbarte Substituenten R¹ bis R⁶, bzw. R¹ und R⁸, ein mono-oder polycyclisches, aliphatisches Ringsystem bilden können, das mit einem oder mehreren Resten R⁹ substituiert sein kann;
R⁷ ist gleich oder verschieden bei jedem Auftreten ausgewählt aus der Gruppe bestehend aus H, D, F, Si(R⁹)₃, CN, einer geradkettigen Alkyl- oder Alkoxygruppe mit 1 bis 10 C-Atomen oder einer verzweigten oder cyclischen Alkyl- oder Alkoxygruppe mit 3 bis 10 C- Atomen, die jeweils mit einem oder mehreren Resten R⁹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen Ringsystem mit 6 bis 60 aromatischen Ringatomen, das jeweils mit einem oder mehreren Resten R⁹ substituiert sein kann, wobei optional zwei oder mehr benachbarte Substituenten R⁷ ein mono- oder polycyclisches, aliphatisches Ringsystem bilden können, das mit einem oder mehreren Resten R⁹ substituiert sein kann;
oder R⁷, welcher in Formel (Ar1-1), (Ar1-5), (Ar1-9), (Ar1-16), (Ar1-17), (Ar1-5a), (Ar1-5b), (Ar1-9a), (Ar1-9b), (Ar1-17a), (Ar1-17b), (Ar2-1) (Ar3-8), (Ar3-8a), (Ar3-8b) an die Kohlenstoffbrücke bindet, ist gleich oder verschieden bei jedem Auftreten ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R⁹ substituiert sein kann; dabei können die beiden Gruppen R⁷ auch miteinander ein Ringsystem bilden, welches aliphatisch oder aromatisch sein kann;
oder R⁷, welcher in Formel (Ar1-2), (Ar1-6), (Ar1-10), (Ar1-19), (Ar1-6a), (Ar1-6b), (Ar1-10a), (Ar1-10b), (Ar2-2), (Ar3-9), (Ar3-9a) an die Stickstoffbrücke bindet, ist ausgewählt aus der Gruppe bestehend aus einer geradkettigen Alkylgruppe mit 1 bis 10 C-Atomen, einer verzweigten oder cyclischen Alkylgruppe mit 3 bis 10 C-Atomen oder einem aromatischen Ringsystem mit 6 bis 30 C-Atomen, das mit einem oder mehreren Resten R⁹ substituiert sein kann;
R⁹ ist gleich oder verschieden bei jedem Auftreten ausgewählt aus der Gruppe bestehend aus H, D, F, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 30 C-Atomen, das jeweils mit einem oder mehreren Resten R¹⁰ substituiert sein kann, wobei zwei oder mehr benachbarte Substituenten R⁹ miteinander ein mono- oder polycyclisches, aliphatisches, Ringsystem bilden können;
R¹⁰ ist ausgewählt aus der Gruppe bestehend aus H, D, F, einem aliphatischem Kohlenwasserstoffrest mit 1 bis 20 C-Atomen, einem aromatischem Ringsystem mit 6 bis 30 C-Atomen, in dem ein oder mehrere H-Atome durch D oder F ersetzt sein können, wobei zwei oder mehr benachbarte Substituenten R¹⁰ miteinander ein mono-oder polycyclisches, aliphatisches, Ringsystem bilden können;
i ist bei jedem Auftreten 0, 1 oder 2;
m ist 0 oder 1;
o, p, r sind bei jedem Auftreten gleich oder verschieden 0, 1 oder 2;
n, q sind bei jedem Auftreten gleich oder verschieden 0, 1 oder 2;
s, t, u sind bei jedem Auftreten gleich oder verschieden 0 oder 1;
dabei gilt u+t+s ≤ 2;

8. Verfahren zur Herstellung einer Verbindung nach einem oder mehreren der Ansprüche 1 bis 7 durch Kupplung eines Dispiro[fluoren-9,9'-anthracen-10',9"-fluoren]-Derivats, welches in 1,3 oder 4-Position mit einer reaktiven Abgangsgruppe substituiert ist, mit
a) einem primären Amin, gefolgt von der Kupplung mit einer weiteren aromatischen Gruppe, die mit einer reaktiven Abgangsgruppe substituiert ist, oder
b) mit einem sekundären Amin, oder
c) mit einem Triarylaminderivat.

9. Formulierung enthaltend mindestens eine Verbindung nach einem oder mehreren der Ansprüche 1 bis 7 und mindestens ein Lösungsmittel.

10. Verwendung einer Verbindung nach einem oder mehreren der Ansprüche 1 bis 7 oder einer Formulierung nach Anspruch 9 in einer elektronischen Vorrichtung, insbesondere in einer elektronischen Elektrolumineszenzvorrichtung.

11. Elektronische Vorrichtung enthaltend mindestens eine Verbindung gemäß einem oder mehreren der Ansprüche 1 bis 7 oder mindestens einer Formulierung gemäß Anspruch 9.

12. Elektronische Vorrichtung nach Anspruch 11, **dadurch gekennzeichnet, dass** sie aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs) ausgewählt ist.

13. Elektronische Vorrichtung nach Anspruch 12, gewählt aus organischen Elektrolumineszenzvorrichtungen (OLEDs), **dadurch gekennzeichnet, dass** die Verbindung nach einem oder mehreren der Ansprüche 1 bis 7 oder mindestens eine Formulierung nach Anspruch 9 als Lochtransportmaterial in einer Lochtransport- oder Lochinjektionsschicht, oder als Elektronenblockierschicht, oder als Exzitonenblockierschicht oder als Emitter für fluoreszierende Emissionsschichten oder als Matrixmaterial in einer emittierenden Schicht eingesetzt wird.

## Claims

1. Compound of the formula (1), 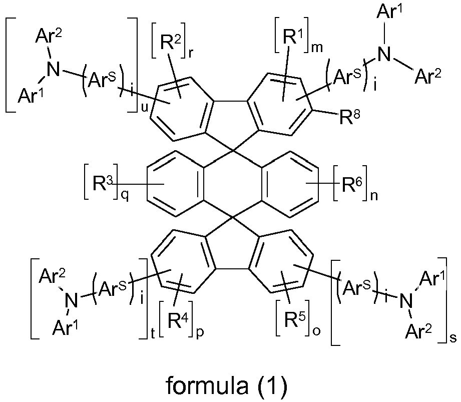 where the following applies to the symbols and indices occurring:
Ar^{S} is, identically or differently on each occurrence, an aromatic or heteroaromatic ring system having 6 to 60 C atoms, which may in each case also be substituted by one or more radicals R⁷; Ar^{S} here may be connected to Ar¹ and/or to Ar² by a group E;
Ar¹, Ar² are, identically or differently on each occurrence, an aromatic or heteroaromatic ring system having 6 to 60 aromatic ring atoms, which may in each case also be substituted by one or more radicals R⁷; Ar¹ and Ar² here may be connected to one another and/or Ar¹ may be connected to Ar^{S} and/or Ar² may be connected to Ar^{S} by a group E;
or the group -NAr¹Ar² has a structure of one of the formulae (Ar1-13) to (Ar1-19); 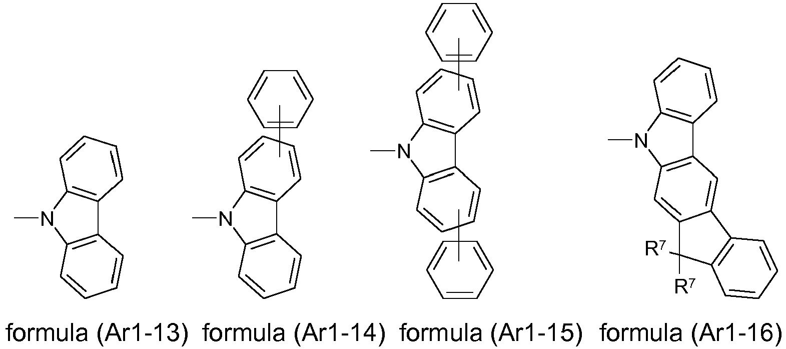 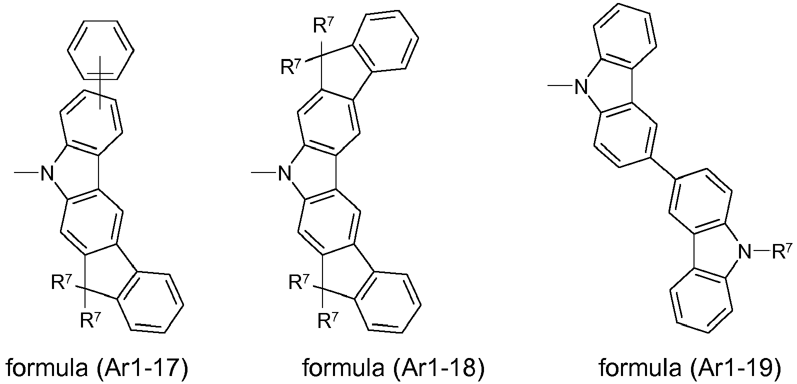 where the groups of the formulae (Ar1-13) to (Ar1-19) may be substituted at the free positions by R⁷;
or the group -Ar^{S}-NAr¹Ar² has a structure of one of the formulae (Ar2-5) to (Ar2-9); 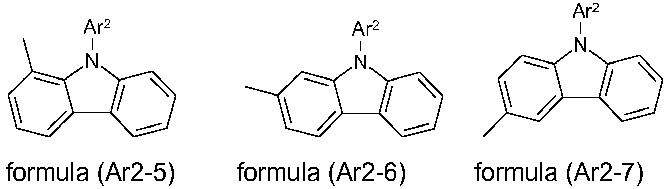 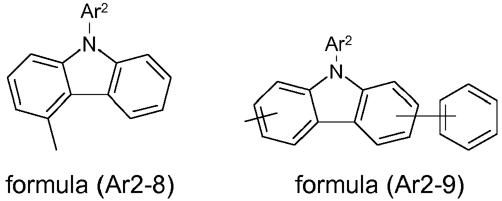 where the groups of the formulae (Ar2-5) to (Ar2-9) may be substituted at the free positions by R⁷;
E is selected, identically or differently on each occurrence, from the group consisting of C(R⁷)₂, O, S and NR⁷;
R¹, R², R³, R⁴, R⁵, R⁶ are selected on each occurrence, identically or differently, from the group consisting of H, D, F, Cl, Br, I, CN, Si(R⁹)₃, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁹, where in each case one or more non-adjacent CH₂ groups may be replaced by Si(R⁹)₂, C=NR⁹, P(=O)(R⁹), SO, SO₂, NR⁹, O, S or CONR⁹ and where one or more H atoms may be replaced by D, F, Cl, Br or I, an aromatic or heteroaromatic ring system having 6 to 60 aromatic ring atoms, which may in each case be substituted by one or more radicals R⁹, an aryloxy group or heteroaryloxy group having 5 to 60 aromatic ring atoms, which may be substituted by one or more radicals R⁹, or an aralkyl group or heteroaralkyl group having 5 to 60 aromatic ring atoms, which may in each case be substituted by one or more radicals R⁹, where two or more adjacent substituents R¹ or two or more adjacent substituents R² or two or more adjacent substituents R³ or two or more adjacent substituents R⁴ or two or more adjacent substituents R⁵ or two or more adjacent substituents R⁶ may optionally form a mono- or polycyclic, aliphatic or aromatic ring system, which may be substituted by one or more radicals R⁹;
R⁷ is selected on each occurrence, identically or differently, from the group consisting of H, D, F, Cl, Br, I, CN, Si(R⁹)₃, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁹, where in each case one or more non-adjacent CH₂ groups may be replaced by Si(R⁹)₂, C=NR⁹, P(=O)(R⁹), SO, SO₂, NR⁹, O, S or CONR⁹ and where one or more H atoms may be replaced by D, F, Cl, Br or I, an aromatic or heteroaromatic ring system having 6 to 60 aromatic ring atoms, which may in each case be substituted by one or more radicals R⁹, an aryloxy group or heteroaryloxy group having 5 to 60 aromatic ring atoms, which may be substituted by one or more radicals R⁹, or an aralkyl group or heteroaralkyl group having 5 to 60 aromatic ring atoms, which may in each case be substituted by one or more radicals R⁹, where two or more adjacent substituents R⁷ may optionally form a mono- or polycyclic, aliphatic or aromatic ring system, which may be substituted by one or more radicals R⁹;
R⁸ is selected from the group consisting of H, D, F, Cl, Br, I, CN, Si(R⁹)₃, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R⁹, where in each case one or more non-adjacent CH₂ groups may be replaced by Si(R⁹)₂, C=NR⁹, P(=O)(R⁹), SO, SO₂, NR⁹, O, S or CONR⁹ and where one or more H atoms may be replaced by D, F, Cl, Br or I, an aromatic or heteroaromatic ring system having 6 to 60 aromatic ring atoms, which may in each case be substituted by one or more radicals R⁹, an aryloxy group or heteroaryloxy group having 5 to 60 aromatic ring atoms, which may be substituted by one or more radicals R⁹, or an aralkyl group or heteroaralkyl group having 5 to 60 aromatic ring atoms, which may in each case be substituted by one or more radicals R⁹, where a substituent R⁸ and an adjacent substituent R¹ may optionally form a mono- or polycyclic, aliphatic or aromatic ring system, which may be substituted by one or more radicals R⁹;
R⁹ is selected on each occurrence, identically or differently, from the group consisting of H, D, F, Cl, Br, I, Si(R¹⁰)₃, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R¹⁰, where one or more non-adjacent CH₂ groups may be replaced by Si(R¹⁰)₂, C=NR¹⁰, P(=O)(R¹⁰), SO, SO₂, NR¹⁰, O, S or CONR¹⁰ and where one or more H atoms may be replaced by D, F, Cl, Br or I, an aromatic or heteroaromatic ring system having 6 to 60 aromatic ring atoms, which may in each case be substituted by one or more radicals R¹⁰, an aryloxy group or heteroaryloxy group having 5 to 60 aromatic ring atoms, which may be substituted by one or more radicals R¹⁰, or an aralkyl group or heteroaralkyl group having 5 to 60 aromatic or heteroaromatic ring atoms, which may be substituted by one or more radicals R¹⁰, where two or more adjacent substituents R⁹ may optionally form a mono- or polycyclic, aliphatic ring system, which may be substituted by one or more radicals R¹⁰;
R¹⁰ is selected from the group consisting of H, D, F, an aliphatic hydrocarbon radical having 1 to 20 C atoms, an aromatic ring system having 6 to 30 C atoms, in which one or more H atoms may be replaced by D or F, where two or more adjacent substituents R¹⁰ may form a mono- or polycyclic, aliphatic ring system with one another;
i is on each occurrence 0, 1 or 2;
m is 0, 1 or 2;
n, o, p, q, r are on each occurrence, identically or differently, 0, 1, 2, 3 or 4;
s, t, u are on each occurrence, identically or differently, 0, 1 or 2;
where s+o ≤ 4, p+t ≤ 4 and r+u ≤ 4;
and furthermore u+t+s ≤ 2.

2. Compound according to Claim 1 of the formula (2), (3), (4) or (5), 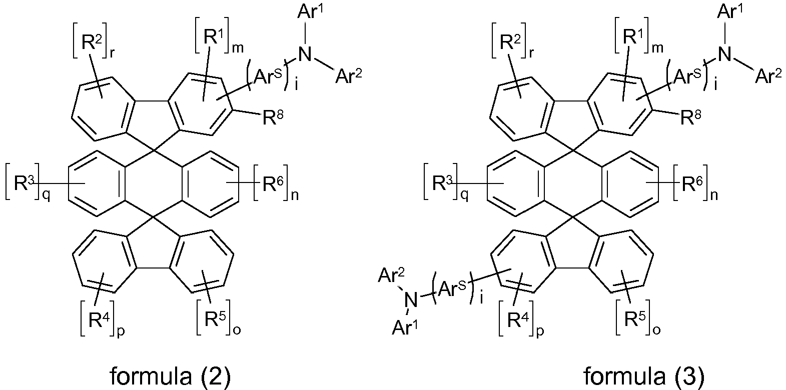 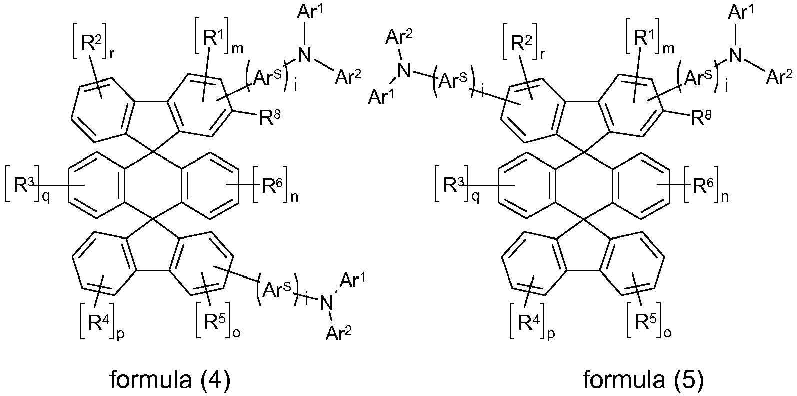 where the symbols and indices have the meanings given in Claim 1.

3. Compound according to Claim 1 or 2, **characterised in that** the groups Ar¹ and Ar² are selected, identically or differently on each occurrence, from the groups of the formulae (Ar-1) to (Ar-33) 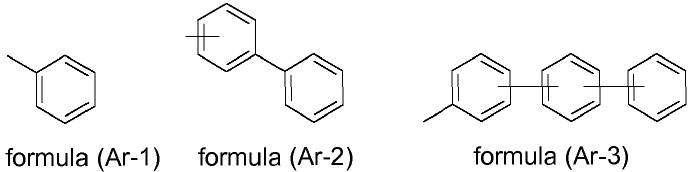 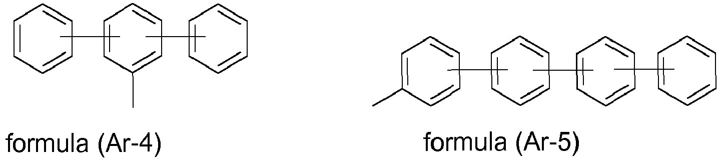 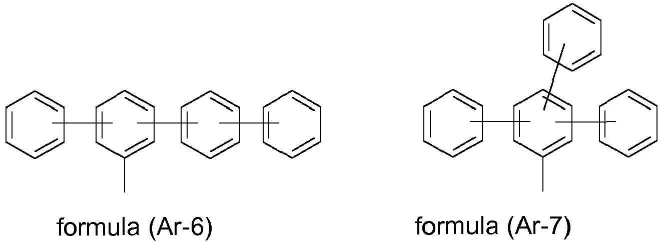 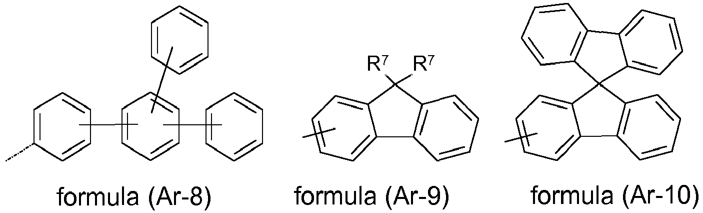 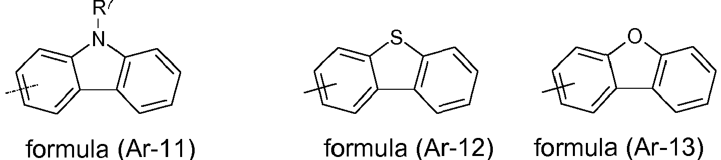 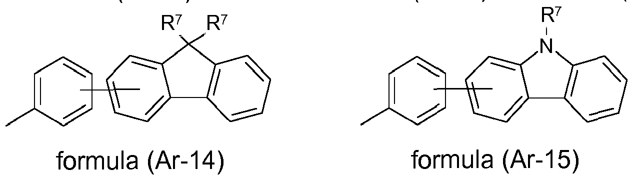 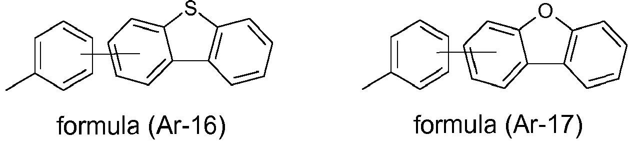 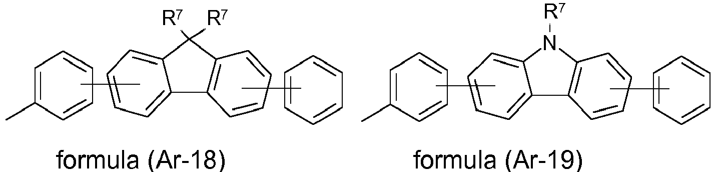 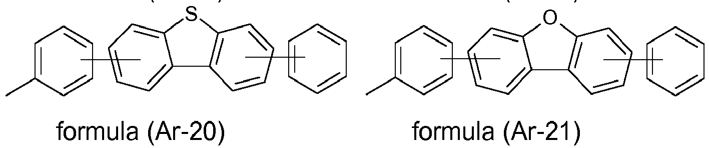 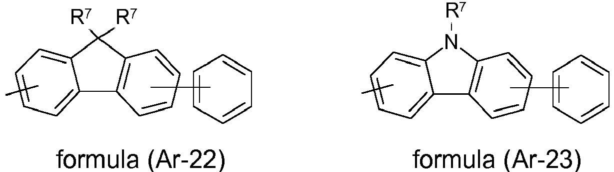 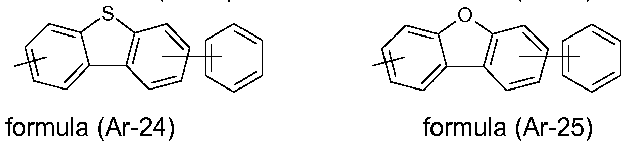 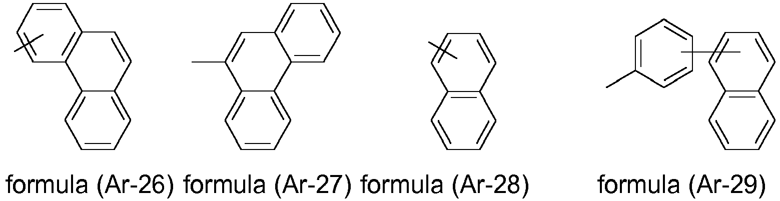 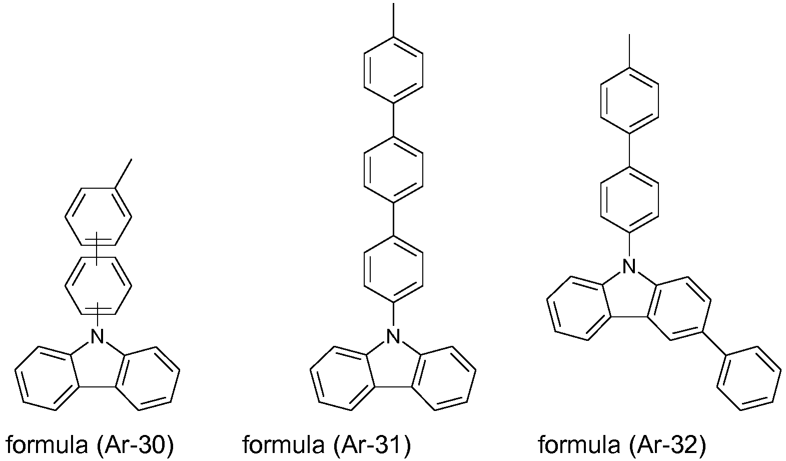 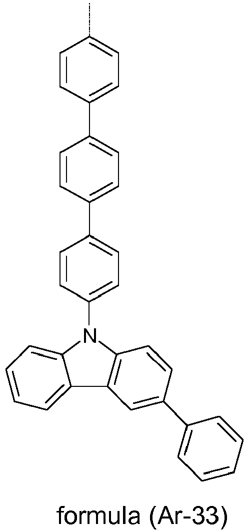 where the symbols used have the meanings given in Claim 1 and the groups may be substituted at the free positions by R⁷.

4. Compound according to one or more of Claims 1 to 3, **characterised in that** the group -NAr¹Ar² has a structure of one of the following formulae (Ar1-1) to (Ar1-19): 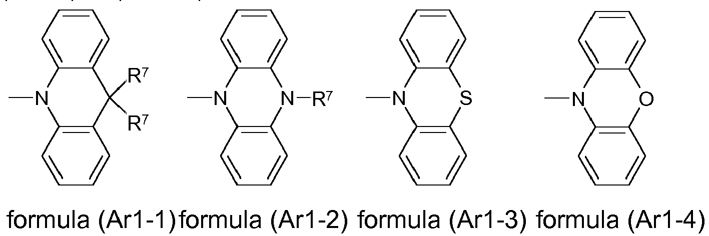 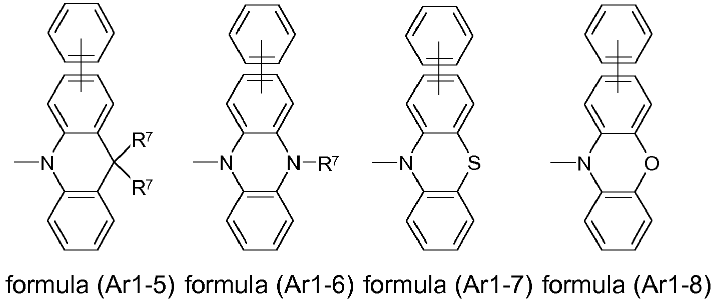 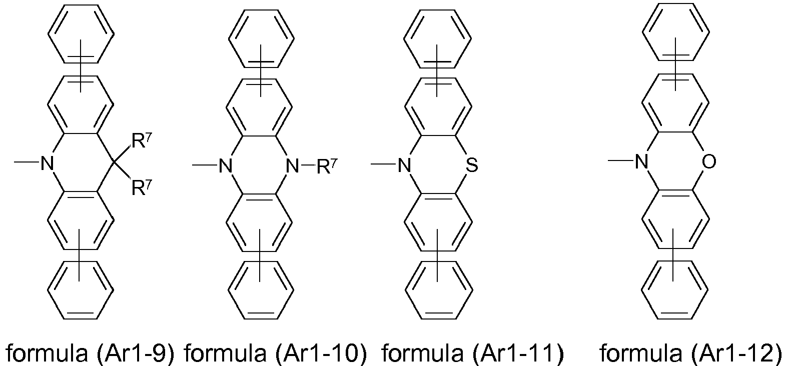 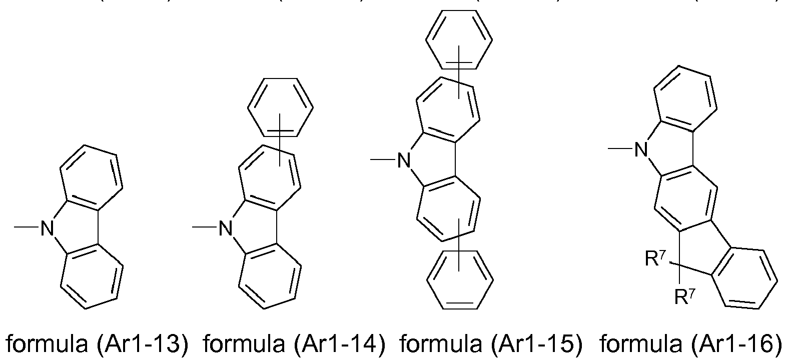 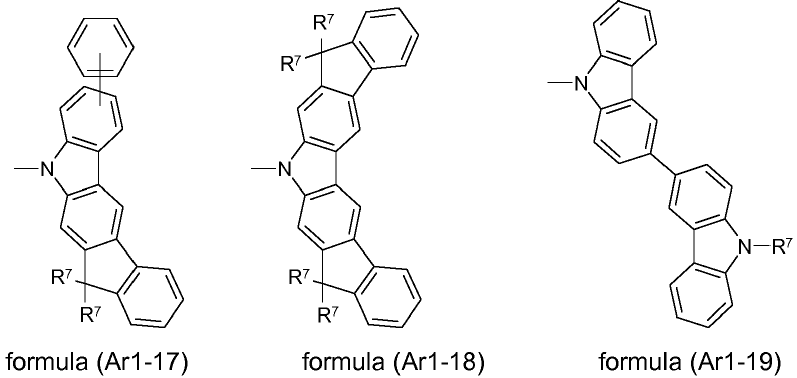 where the symbols used have the meanings given in Claim 1 and the groups may be substituted at the free positions by R⁷,
or **in that** the group -Ar^{S}-NAr¹Ar² has the structure of one of the following formulae (Ar2-1) to (Ar2-9): 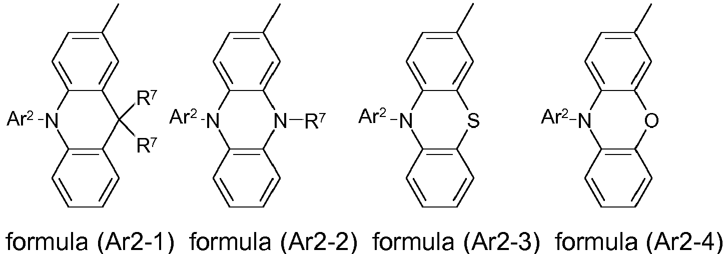 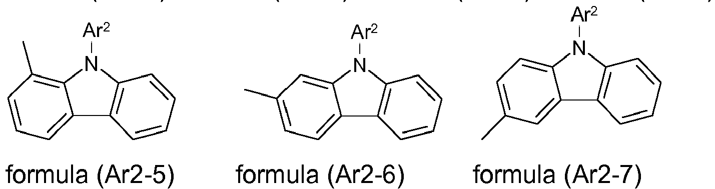 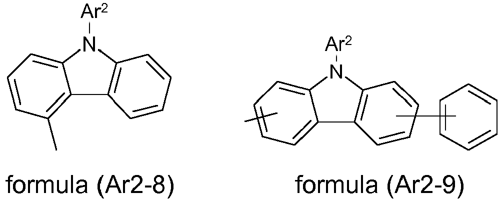 where the symbols used have the meanings given in Claim 1 and the groups may be substituted at the free positions by R⁷.

5. Compound according to one or more of Claims 1 to 4, **characterised in that** the group Ar^{S} stands for a group of one of the following formulae (Ar3-1) to (Ar3-12): 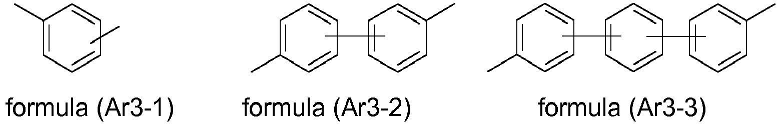 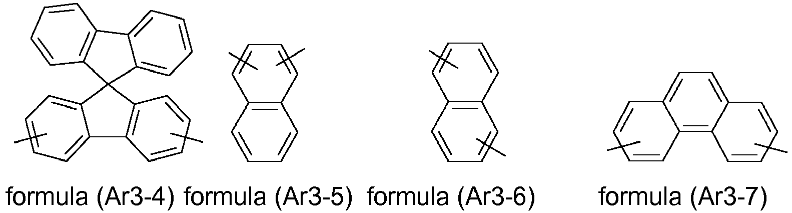 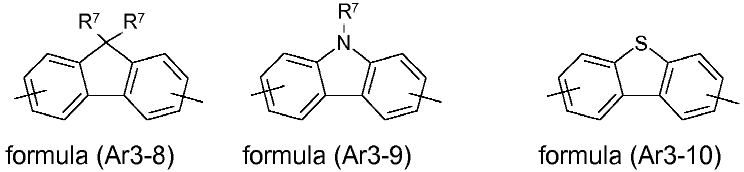 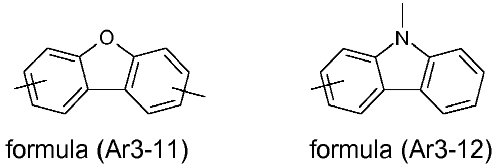 where the symbols used have the meanings given in Claim 1 and the groups may be substituted at the free positions by R⁷.

6. Compound according to one or more of Claims 1 to 5, **characterised in that** R¹, R², R³, R⁴, R⁵, R⁶, R⁷, R⁸ are identical or different and are selected on each occurrence from the group consisting of H, D, F, Si(R⁹)₃, CN, a straight-chain alkyl or alkoxy group having 1 to 10 C atoms or a branched or cyclic alkyl or alkoxy group having 3 to 10 C atoms, each of which may be substituted by one or more radicals R⁹, where one or more non-adjacent CH₂ groups may be replaced by O and where one or more H atoms may be replaced by D or F, an aromatic ring system having 6 to 60 aromatic ring atoms, which may in each case be substituted by one or more radicals R⁹, where two or more adjacent substituents R¹ to R⁷ may optionally form a mono- or polycyclic, aliphatic ring system, which may be substituted by one or more radicals R⁹.

7. Compound according to one or more of Claims 1 to 6 for which the following applies:
Ar^{S} is, identically or differently on each occurrence, an aromatic or heteroaromatic ring system, where, for i=1 or 2, Ar^{S} is selected from the groups of the formulae (Ar3-1) to (Ar3-12), where Ar^{S} may be connected to Ar¹ by a group E;
Ar¹, Ar² are, identically or differently on each occurrence, an aromatic or heteroaromatic ring system selected from the groups of the formulae (Ar-1) to (Ar-33);
or -NAr¹Ar² stands for a group of one of the formulae (Ar1-1) to (Ar1-19);
or Ar^{S}-NAr¹Ar² stands for a group of one of the formulae (Ar2-1) to (Ar2-9);
E is selected, identically or differently on each occurrence, from the group consisting of C(R⁷)₂, O, S and NR⁷;
R¹, R², R³, R⁴, R⁵, R⁶, R⁸ are selected, identically or differently on each occurrence, from the group consisting of H, D, F, Si(R⁹)₃, CN, a straight-chain alkyl or alkoxy group having 1 to 10 C atoms or a branched or cyclic alkyl or alkoxy group having 3 to 10 C atoms, each of which may be substituted by one or more radicals R⁹, where one or more non-adjacent CH₂ groups may be replaced by O and where one or more H atoms may be replaced by D or F, an aromatic ring system having 6 to 60 aromatic ring atoms, which may in each case be substituted by one or more radicals R⁹, where two or more adjacent substituents R¹ to R⁶, or R¹ and R⁸, may optionally form a mono- or polycyclic, aliphatic ring system, which may be substituted by one or more radicals R⁹;
R⁷ is selected, identically or differently on each occurrence, from the group consisting of H, D, F, Si(R⁹)₃, CN, a straight-chain alkyl or alkoxy group having 1 to 10 C atoms or a branched or cyclic alkyl or alkoxy group having 3 to 10 C atoms, each of which may be substituted by one or more radicals R⁹, where one or more non-adjacent CH₂ groups may be replaced by O and where one or more H atoms may be replaced by D or F, an aromatic ring system having 6 to 60 aromatic ring atoms, which may in each case be substituted by one or more radicals R⁹, where two or more adjacent substituents R⁷ may optionally form a mono- or polycyclic, aliphatic ring system, which may be substituted by one or more radicals R⁹;
or R⁷ which is bonded to the carbon bridge in formulae (Ar1-1), (Ar1-5), (Ar1-9), (Ar1-16), (Ar1-17), (Ar1-5a), (Ar1-5b), (Ar1-9a), (Ar1-9b), (Ar1-17a), (Ar1-17b), (Ar2-1), (Ar3-8), (Ar3-8a) and (Ar3-8b) is selected, identically or differently on each occurrence, from the group consisting of a straight-chain alkyl group having 1 to 10 C atoms, a branched or cyclic alkyl group having 3 to 10 C atoms or an aromatic ring system having 6 to 30 C atoms, which may be substituted by one or more radicals R⁹; the two groups R⁷ here may also form a ring system with one another, which may be aliphatic or aromatic;
or R⁷ which is bonded to the nitrogen bridge in formulae (Ar1-2), (Ar1-6), (Ar1-10), (Ar1-19), (Ar1-6a), (Ar1-6b), (Ar1-10a), (Ar1-10b), (Ar2-2), (Ar3-9) and (Ar3-9a) is selected from the group consisting of a straight-chain alkyl group having 1 to 10 C atoms, a branched or cyclic alkyl group having 3 to 10 C atoms or an aromatic ring system having 6 to 30 C atoms, which may be substituted by one or more radicals R⁹;
R⁹ is selected, identically or differently on each occurrence, from the group consisting of H, D, F, a straight-chain alkyl group having 1 to 10 C atoms or a branched or cyclic alkyl group having 3 to 10 C atoms or an aromatic ring system having 6 to 30 C atoms, each of which may be substituted by one or more radicals R¹⁰, where two or more adjacent substituents R⁹ may form a mono- or polycyclic, aliphatic ring system with one another;
R¹⁰ is selected from the group consisting of H, D, F, an aliphatic hydrocarbon radical having 1 to 20 C atoms, an aromatic ring system having 6 to 30 C atoms, in which one or more H atoms may be replaced by D or F, where two or more adjacent substituents R¹⁰ may form a mono- or polycyclic, aliphatic ring system with one another;
i is on each occurrence 0, 1 or 2;
m is 0 or 1;
o, p, r are on each occurrence, identically or differently, 0, 1 or 2;
n, q are on each occurrence, identically or differently, 0, 1 or 2;
s, t, u are on each occurrence, identically or differently, 0 or 1;
where u+t+s ≤ 2.

8. Process for the preparation of a compound according to one or more of Claims 1 to 7 by coupling a dispiro[fluoren-9,9'-anthracene-10',9"-fluorene] derivative which is substituted in the 1, 3 or 4 position by a reactive leaving group to
a) a primary amine, followed by coupling to a further aromatic group which is substituted by a reactive leaving group, or
b) to a secondary amine, or
c) to a triarylamine derivative.

9. Formulation comprising at least one compound according to one or more of Claims 1 to 7 and at least one solvent.

10. Use of a compound according to one or more of Claims 1 to 7 or a formulation according to Claim 9 in an electronic device, in particular in an electronic electroluminescent device.

11. Electronic device comprising at least one compound according to one or more of Claims 1 to 7 or at least one formulation according to Claim 9.

12. Electronic device according to Claim 11, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

13. Electronic device according to Claim 12, selected from organic electroluminescent devices (OLEDs), **characterised in that** the compound according to one or more of Claims 1 to 7 or at least one formulation according to Claim 9 is employed as hole-transport material in a hole-transport or hole-injection layer, or as electron-blocking layer, or as exciton-blocking layer or as emitter for fluorescent emission layers or as matrix material in an emitting layer.

## Revendications

1. Composé de la formule (1) : 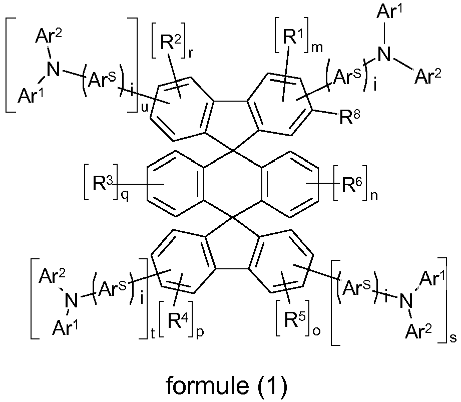 dans laquelle ce qui suit s'appliques aux symboles et indices qui sont rencontrés :
Ar^{S} est, de manière identique ou différente pour chaque occurrence, un système de cycle aromatique ou hétéroaromatique qui comporte 6 à 60 atomes de C, lequel système de cycle peut dans chaque cas être également substitué par un radical ou par plusieurs radicaux R⁷ ; Ar^{S} peut ici être connecté à Ar¹ et/ou à Ar² au moyen d'un groupe E ;
Ar¹, Ar² sont, de manière identique ou différente pour chaque occurrence, un système de cycle aromatique ou hétéroaromatique qui comporte 6 à 60 atomes de cycle aromatique, lequel système de cycle peut dans chaque cas être également substitué par un radical ou par plusieurs radicaux R⁷; Ar¹ et Ar² peuvent ici être connectés l'un à l'autre et/ou Ar¹ peut être connecté à Ar^{S} et/ou Ar² peut être connecté à Ar^{S} au moyen d'un groupe E ;
ou le groupe -NAr¹Ar² présente une structure de l'une des formules (Ar1-13) à (Ar1-19) : 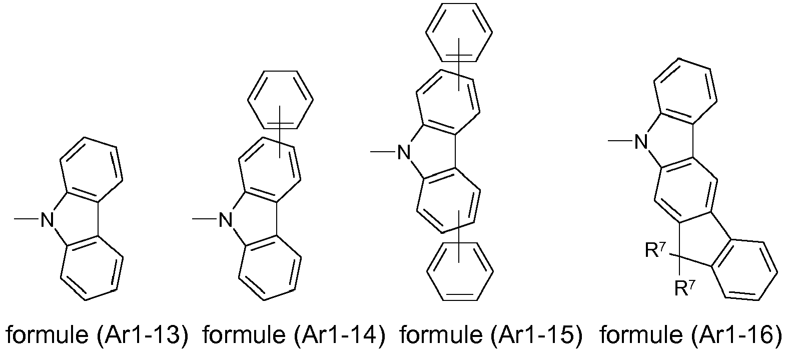 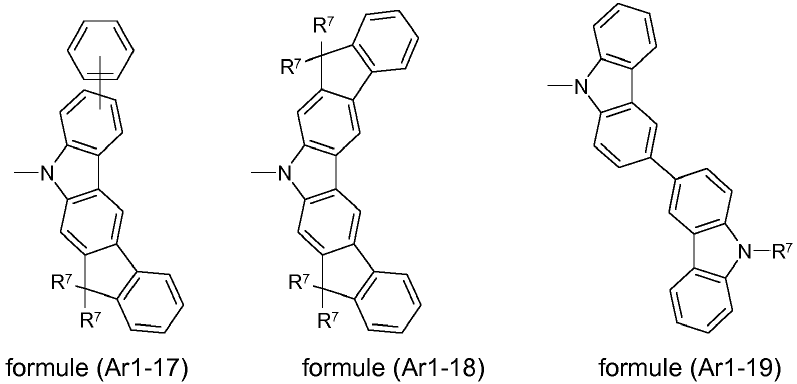 dans lesquelles les groupes des formules (Ar1-13) à (Ar1-19) peuvent être substitués au niveau des positions libres par R⁷ ;
ou le groupe -Ar^{S}-NAr¹Ar² présente une structure de l'une des formules (Ar2-5) à (Ar2-9) : 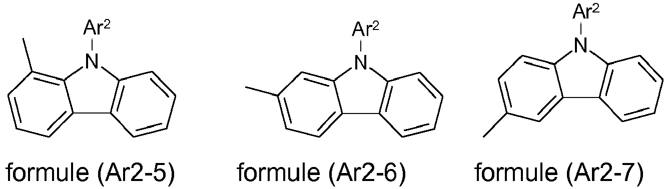 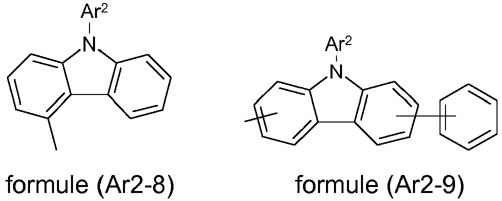 dans lesquelles les groupes des formules (Ar2-5) à (Ar2-9) peuvent être substitués au niveau des positions libres par R⁷ ;
E est sélectionné, de manière identique ou différente pour chaque occurrence, parmi le groupe qui est constitué par C(R⁷)₂, O, S et NR⁷;
R¹, R², R³, R⁴, R⁵, R⁶ sont sélectionnés pour chaque occurrence, de manière identique ou différente, parmi le groupe qui est constitué par H, D, F, Cl, Br, I, CN, Si(R⁹)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C, dont chacun peut être substitué par un radical ou par plusieurs radicaux R⁹, où dans chaque cas, un ou plusieurs groupe(s) CH₂ non adjacents peut/peuvent être remplacé(s) par Si(R⁹)₂, C=NR⁹, P(=O)(R⁹), SO, SO₂, NR⁹, O, S ou CONR⁹ et où un ou plusieurs atome(s) de H peut/peuvent être remplacé(s) par D, F, Cl, Br ou I, un système de cycle aromatique ou hétéroaromatique qui comporte 6 à 60 atomes de cycle aromatique, lequel système de cycle peut dans chaque cas être substitué par un radical ou par plusieurs radicaux R⁹, un groupe aryloxy ou hétéroaryloxy qui comporte 5 à 60 atomes de cycle aromatique, lequel groupe peut être substitué par un radical ou par plusieurs radicaux R⁹, ou un groupe aralkyle ou un groupe hétéroaralkyle qui comporte 5 à 60 atomes de cycle aromatique, lequel groupe peut dans chaque cas être substitué par un radical ou par plusieurs radicaux R⁹, où deux substituants R¹ adjacents ou plus ou deux substituants R² adjacents ou plus ou deux substituants R³ adjacents ou plus ou deux substituants R⁴ adjacents ou plus ou deux substituants R⁵ adjacents ou plus ou deux substituants R⁶ adjacents ou plus peuvent en option former un système de cycle aliphatique ou aromatique monocyclique ou polycyclique, lequel système de cycle peut être substitué par un radical ou par plusieurs radicaux R⁹ ;
R⁷ est sélectionné pour chaque occurrence, de manière identique ou différente, parmi le groupe qui est constitué par H, D, F, Cl, Br, I, CN, Si(R⁹)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C, dont chacun peut être substitué par un radical ou par plusieurs radicaux R⁹, où, dans chaque cas, un ou plusieurs groupe(s) CH₂ non adjacents peut/peuvent être remplacé(s) par Si(R⁹)₂, C=NR⁹, P(=O)(R⁹), SO, SO₂, NR⁹, O, S ou CONR⁹ et où un ou plusieurs atome(s) de H peut/peuvent être remplacé(s) par D, F, Cl, Br ou I, un système de cycle aromatique ou hétéroaromatique qui comporte 6 à 60 atomes de cycle aromatique, lequel système de cycle peut dans chaque cas être substitué par un radical ou par plusieurs radicaux R⁹, un groupe aryloxy ou un groupe hétéroaryloxy qui comporte 5 à 60 atomes de cycle aromatique, lequel groupe peut être substitué par un radical ou par plusieurs radicaux R⁹, ou un groupe aralkyle ou un groupe hétéroaralkyle qui comporte 5 à 60 atomes de cycle aromatique, lequel groupe peut dans chaque cas être substitué par un radical ou par plusieurs radicaux R⁹, où deux substituants R⁷ adjacents ou plus peuvent en option former un système de cycle aliphatique ou aromatique monocyclique ou polycyclique, lequel système de cycle peut être substitué par un radical ou par plusieurs radicaux R⁹;
R⁸ est sélectionné parmi le groupe qui est constitué par H, D, F, Cl, Br, I, CN, Si(R⁹)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C, dont chacun peut être substitué par un radical ou par plusieurs radicaux R⁹, où, dans chaque cas, un ou plusieurs groupe(s) CH₂ non adjacents peut/peuvent être remplacé(s) par Si(R⁹)₂, C=NR⁹, P(=O)(R⁹), SO, SO₂, NR⁹, O, S ou CONR⁹ et où un ou plusieurs atome(s) de H peut/peuvent être remplacé(s) par D, F, Cl, Brou I, un système de cycle aromatique ou hétéroaromatique qui comporte 6 à 60 atomes de cycle aromatique, lequel système de cycle peut dans chaque cas être substitué par un radical ou par plusieurs radicaux R⁹, un groupe aryloxy ou un groupe hétéroaryloxy qui comporte 5 à 60 atomes de cycle aromatique, lequel groupe peut être substitué par un radical ou par plusieurs radicaux R⁹, ou un groupe aralkyle ou un groupe hétéroaralkyle qui comporte 5 à 60 atomes de cycle aromatique, lequel groupe peut dans chaque cas être substitué par un radical ou par plusieurs radicaux R⁹, où un substituant R⁸ et un substituant R¹ adjacent peuvent en option former un système de cycle aliphatique ou aromatique monocyclique ou polycyclique, lequel système de cycle peut être substitué par un radical ou par plusieurs radicaux R⁹;
R⁹ est sélectionné pour chaque occurrence, de manière identique ou différente, parmi le groupe qui est constitué par H, D, F, Cl, Br, I, Si(R¹⁰)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C, dont chacun peut être substitué par un radical ou par plusieurs radicaux R¹⁰, où un ou plusieurs groupe(s) CH₂ non adjacents peut/peuvent être remplacé(s) par Si(R¹⁰)₂, C=NR¹⁰, P(=O)(R¹⁰), SO, SO₂, NR¹⁰, O, S ou CONR¹⁰ et où un ou plusieurs atome(s) de H peut/peuvent être remplacé(s) par D, F, Cl, Br ou I, un système de cycle aromatique ou hétéroaromatique qui comporte 6 à 60 atomes de cycle aromatique, lequel système de cycle peut dans chaque cas être substitué par un radical ou par plusieurs radicaux R¹⁰, un groupe aryloxy ou un groupe hétéroaryloxy qui comporte 5 à 60 atomes de cycle aromatique, lequel groupe peut être substitué par un radical ou par plusieurs radicaux R¹⁰, ou un groupe aralkyle ou un groupe hétéroaralkyle qui comporte 5 à 60 atomes de cycle aromatique ou hétéroaromatique, lequel groupe peut être substitué par un radical ou par plusieurs radicaux R¹⁰, où deux substituants R⁹ adjacents ou plus peuvent en option former un système de cycle aliphatique monocyclique ou polycyclique, lequel système de cycle peut être substitué par un radical ou par plusieurs radicaux R¹⁰ ;
R¹⁰ est sélectionné parmi le groupe qui est constitué par H, D, F, un radical hydrocarbone aliphatique qui comporte 1 à 20 atome(s) de C, un système de cycle aromatique qui comporte 6 à 30 atomes de C, système de cycle dans lequel un ou plusieurs atome(s) de H peut/peuvent être remplacé(s) par D ou par F, où deux substituants R¹⁰ adjacents ou plus peuvent former un système de cycle aliphatique monocyclique ou polycyclique l'un avec l'autre ou les uns avec les autres ;
i est pour chaque occurrence 0, 1 ou 2 ;
m est 0, 1 ou 2 ;
n, o, p, q, r sont pour chaque occurrence, de manière identique ou différente, 0, 1, 2, 3 ou 4 ;
s, t, u sont pour chaque occurrence, de manière identique ou différente, 0, 1 ou 2 ;
où s + o ≤ 4, p + t ≤ 4 et r + u ≤ 4;
et en outre, u + t + s ≤ 2.

2. Composé selon la revendication 1 de la formule (2), (3), (4) ou (5) : 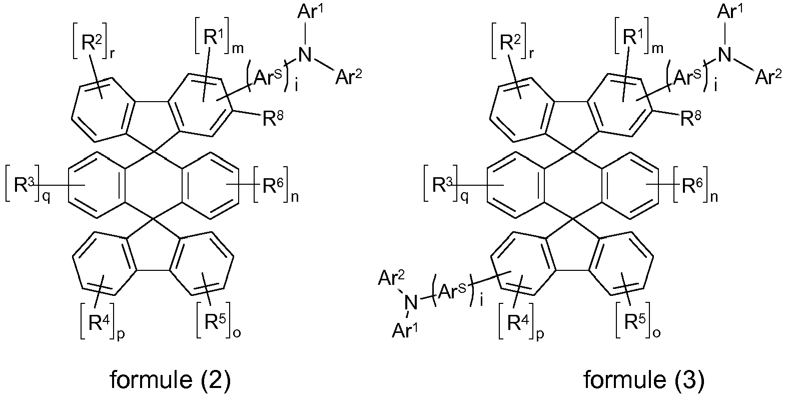 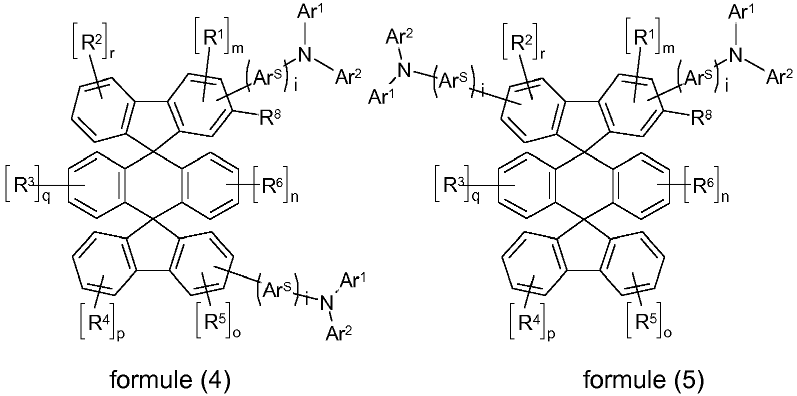 dans lesquelles les symboles et indices présentent les significations qui ont été données selon la revendication 1.

3. Composé selon la revendication 1 ou 2, **caractérisé en ce que** les groupes Ar¹ et Ar² sont sélectionnés, de manière identique ou différente pour chaque occurrence, parmi les groupes des formules (Ar-1) à (Ar-33) : 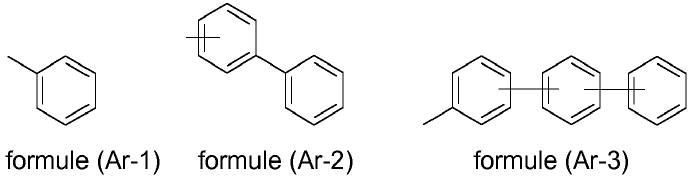 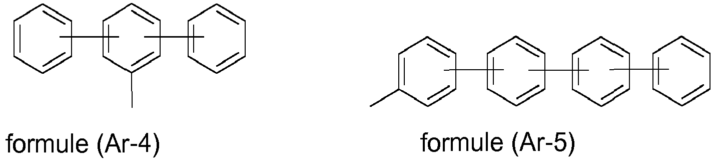 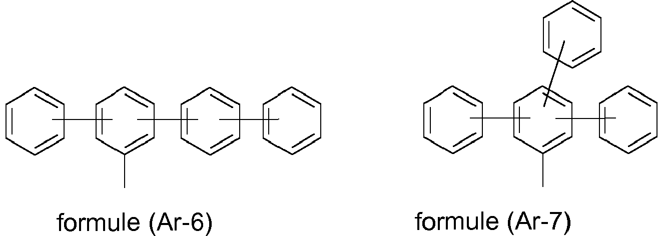 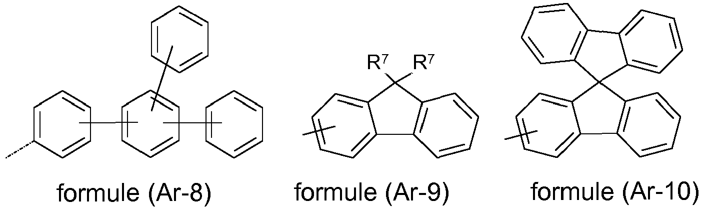 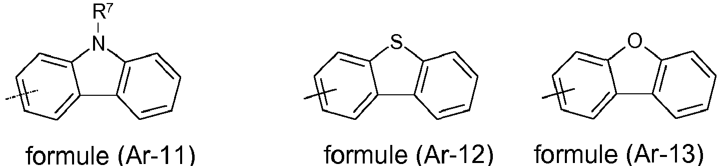 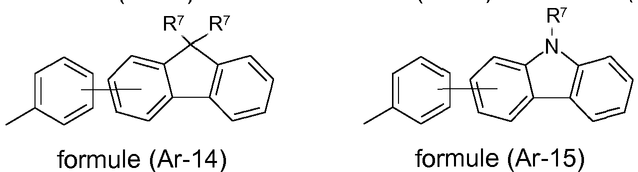 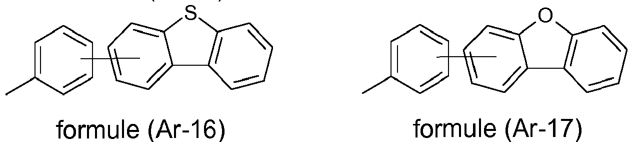 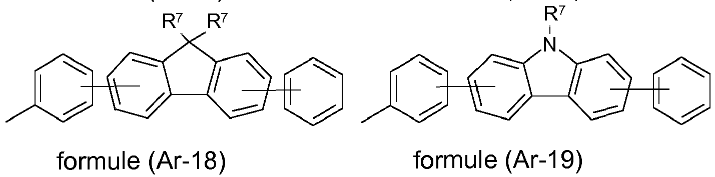 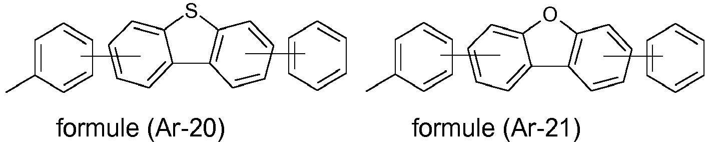 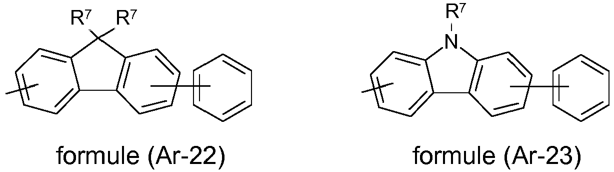 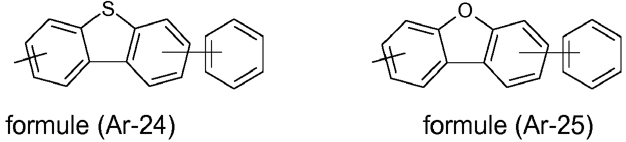 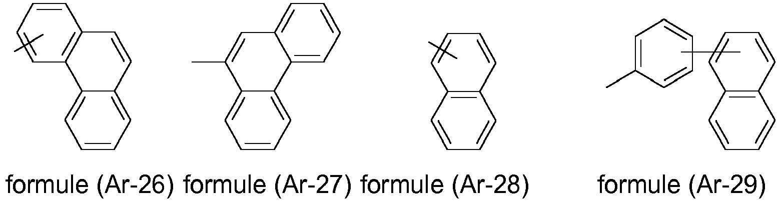 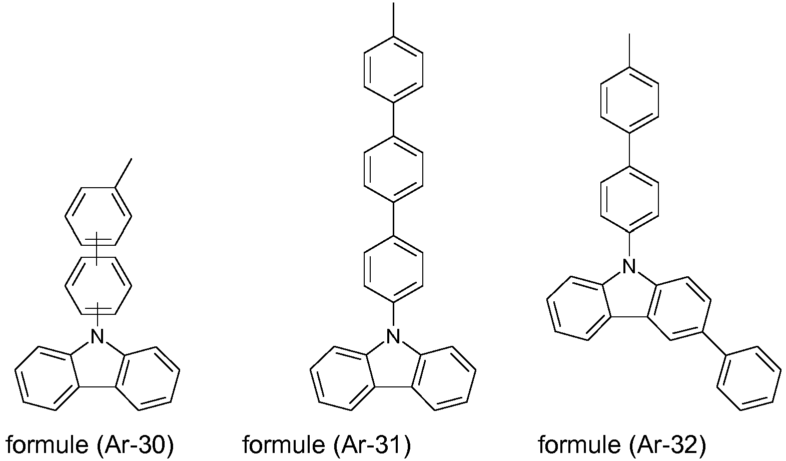 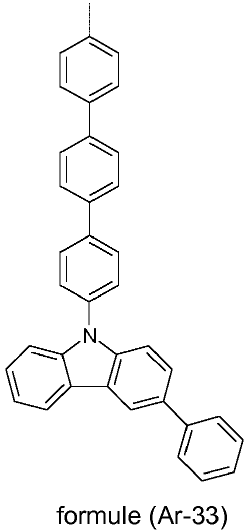 dans lesquelles les symboles qui sont utilisés présentent les significations qui ont été données selon la revendication 1 et les groupes peuvent être substitués au niveau des positions libres par R⁷.

4. Composé selon une ou plusieurs des revendications 1 à 3, **caractérisé en ce que** le groupe -NAr¹Ar² présente une structure de l'une des formules (Ar1-1) à (Ar1-19) qui suivent : 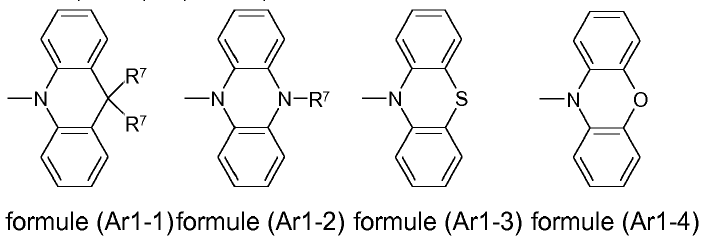 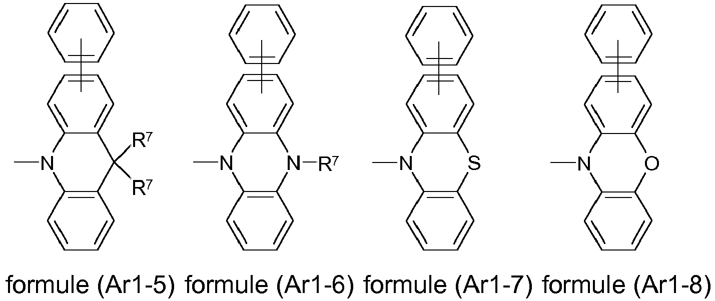 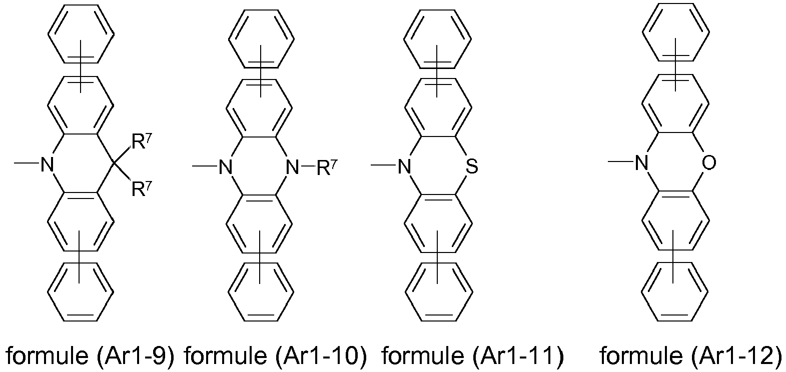 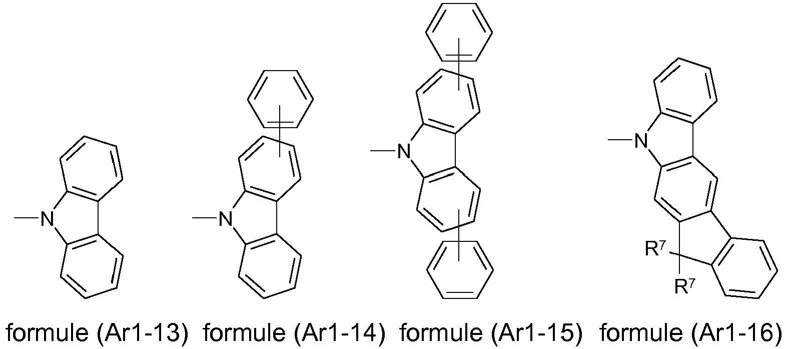 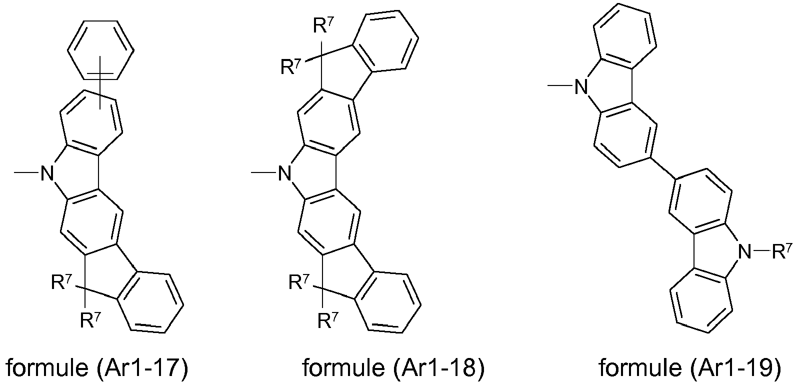 dans lesquelles les symboles qui sont utilisés présentent les significations qui ont été données selon la revendication 1 et les groupes peuvent être substitués au niveau des positions libres par R⁷,
ou **en ce que** le groupe -Ar^{S}-NAr¹Ar² présente la structure de l'une des formules (Ar2-1) à (Ar2-9) qui suivent : 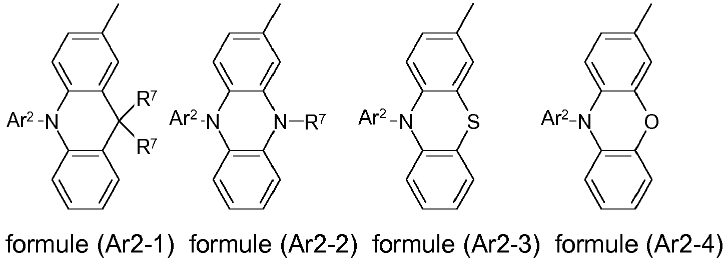 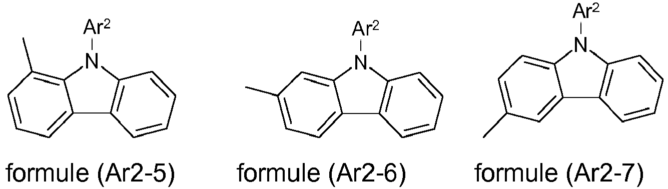 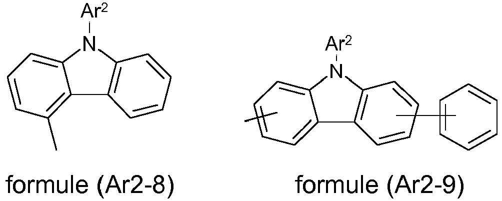 dans lesquelles les symboles qui sont utilisés présentent les significations qui ont été données selon la revendication 1 et les groupes peuvent être substitués au niveau des positions libres par R⁷.

5. Composé selon une ou plusieurs des revendications 1 à 4, **caractérisé en ce que** le groupe Ar^{S} représente un groupe de l'une des formules (Ar3-1) à (Ar3-12) qui suivent : 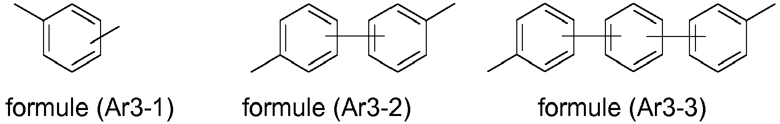 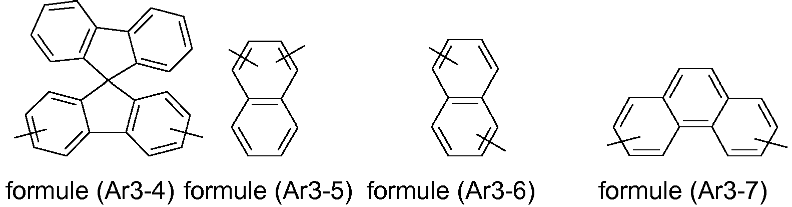 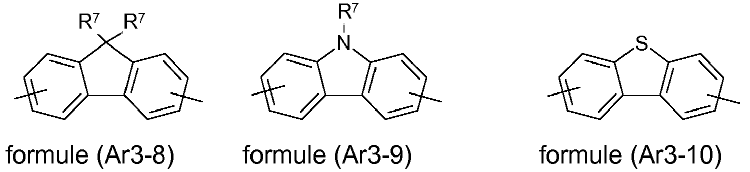 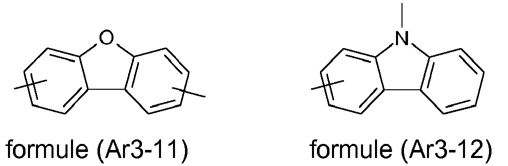 dans lesquelles les symboles qui sont utilisés présentent les significations qui ont été données selon la revendication 1 et les groupes peuvent être substitués au niveau des positions libres par R⁷.

6. Composé selon une ou plusieurs des revendications 1 à 5, **caractérisé en ce que** R¹, R², R³, R⁴, R⁵, R⁶, R⁷, R⁸ sont identiques ou différents et sont sélectionnés pour chaque occurrence parmi le groupe qui est constitué par H, D, F, Si(R⁹)₃, CN, un groupe alkyle ou alcoxy en chaîne droite qui comporte 1 à 10 atome(s) de C ou un groupe alkyle ou alcoxy ramifié ou cyclique qui comporte 3 à 10 atomes de C, dont chacun peut être substitué par un radical ou par plusieurs radicaux R⁹, où un ou plusieurs groupe(s) CH₂ non adjacents peut/peuvent être remplacé(s) par O et où un ou plusieurs atome(s) de H peut/peuvent être remplacé(s) par D ou F, un système de cycle aromatique qui comporte 6 à 60 atomes de cycle aromatique, lequel système de cycle peut dans chaque cas être substitué par un radical ou par plusieurs radicaux R⁹, où deux substituants R¹ à R⁷ adjacents ou plus peuvent en option former un système de cycle aliphatique monocyclique ou polycyclique, lequel système de cycle peut être substitué par un radical ou par plusieurs radicaux R⁹.

7. Composé selon une ou plusieurs des revendications 1 à 6 pour lequel ce qui suit s'applique :
Ar^{S} est, de manière identique ou différente pour chaque occurrence, un système de cycle aromatique ou hétéroaromatique, où, pour i = 1 ou 2, Ar^{S} est sélectionné parmi les groupes des formules (Ar3-1) à (Ar3-12), où Ar^{S} peut être connecté à Ar¹ au moyen d'un groupe E ;
Ar¹, Ar² sont, de manière identique ou différente pour chaque occurrence, un système de cycle aromatique ou hétéroaromatique qui est sélectionné parmi les groupes des formules (Ar-1) à (Ar-33) ;
ou -NAr¹Ar² représente un groupe de l'une des formules (Ar1-1) à (Ar1-19) ;
ou Ar^{S}-NAr¹Ar² représente un groupe de l'une des formules (Ar2-1) à (Ar2-9) ;
E est sélectionné, de manière identique ou différente pour chaque occurrence, parmi le groupe qui est constitué par C(R⁷)₂, O, S et NR⁷;
R¹, R², R³, R⁴, R⁵, R⁶, R⁸ sont sélectionnés, de manière identique ou différente pour chaque occurrence, parmi le groupe qui est constitué par H, D, F, Si(R⁹)₃, CN, un groupe alkyle ou alcoxy en chaîne droite qui comporte 1 à 10 atome(s) de C ou un groupe alkyle ou alcoxy ramifié ou cyclique qui comporte 3 à 10 atomes de C, dont chacun peut être substitué par un radical ou par plusieurs radicaux R⁹, où un ou plusieurs groupe(s) CH₂ non adjacents peut/peuvent être remplacé(s) par O et où un ou plusieurs atome(s) de H peut/ peuvent être remplacé(s) par D ou F, un système de cycle aromatique qui comporte 6 à 60 atomes de cycle aromatique, lequel système de cycle peut dans chaque cas être substitué par un radical ou par plusieurs radicaux R⁹, où deux substituants R¹ à R⁶ ou R¹ et R⁸ adjacents ou plus peuvent en option former un système de cycle aliphatique monocyclique ou polycyclique, lequel système de cycle peut être substitué par un radical ou par plusieurs radicaux R⁹;
R⁷ est sélectionné, de manière identique ou différente pour chaque occurrence, parmi le groupe qui est constitué par H, D, F, Si(R⁹)₃, CN, un groupe alkyle ou alcoxy en chaîne droite qui comporte 1 à 10 atome(s) de C ou un groupe alkyle ou alcoxy ramifié ou cyclique qui comporte 3 à 10 atomes de C, dont chacun peut être substitué par un radical ou par plusieurs radicaux R⁹, où un ou plusieurs groupe(s) CH₂ non adjacents peut/peuvent être remplacé(s) par O et où un ou plusieurs atome(s) de H peut/peuvent être remplacé(s) par D ou par F, un système de cycle aromatique qui comporte 6 à 60 atomes de cycle aromatique, lequel système de cycle peut dans chaque cas être substitué par un radical ou par plusieurs radicaux R⁹, où deux substituants R⁷ adjacents ou plus peuvent en option former un système de cycle aliphatique monocyclique ou polycyclique, lequel système de cycle peut être substitué par un radical ou par plusieurs radicaux R⁹;
ou R⁷, lequel est lié au pont de carbone dans les formules (Ar1-1), (Ar1-5), (Ar1-9), (Ar1-16), (Ar1-17), (Ar1-5a), (Ar1-5b), (Ar1-9a), (Ar1-9b), (Ar1-17a), (Ar1-17b), (Ar2-1), (Ar3-8), (Ar3-8a) et (Ar3-8b), est sélectionné, de manière identique ou différente pour chaque occurrence, parmi le groupe qui est constitué par un groupe alkyle en chaîne droite qui comporte 1 à 10 atome(s) de C, un groupe alkyle ramifié ou cyclique qui comporte 3 à 10 atomes de C ou un système de cycle aromatique qui comporte 6 à 30 atomes de C, lequel système de cycle peut être substitué par un radical ou par plusieurs radicaux R⁹; les deux groupes R⁷ peuvent ici également former un système de cycle l'un avec l'autre, lequel système de cycle peut être aliphatique ou aromatique ;
ou R⁷, lequel est lié au pont d'azote dans les formules (Ar1-2), (Ar1-6), (Ar1-10), (Ar1-19), (Ar1-6a), (Ar1-6b), (Ar1-10a), (Ar1-10b), (Ar2-2), (Ar3-9) et (Ar3-9a), est sélectionné parmi le groupe qui est constitué par un groupe alkyle en chaîne droite qui comporte 1 à 10 atome(s) de C, un groupe alkyle ramifié ou cyclique qui comporte 3 à 10 atomes de C ou un système de cycle aromatique qui comporte 6 à 30 atomes de C, lequel système de cycle peut être substitué par un radical ou par plusieurs radicaux R⁹;
R⁹ est sélectionné, de manière identique ou différente pour chaque occurrence, parmi le groupe qui est constitué par H, D, F, un groupe alkyle en chaîne droite qui comporte 1 à 10 atome(s) de C ou un groupe alkyle ramifié ou cyclique qui comporte 3 à 10 atomes de C ou un système de cycle aromatique qui comporte 6 à 30 atomes de C, dont chacun peut être substitué par un radical ou par plusieurs radicaux R¹⁰, où deux substituants R⁹ adjacents ou plus peuvent former un système de cycle aliphatique monocyclique ou polycyclique l'un avec l'autre ou les uns avec les autres ;
R¹⁰ est sélectionné parmi le groupe qui est constitué par H, D, F, un radical hydrocarbone aliphatique qui comporte 1 à 20 atome(s) de C, un système de cycle aromatique qui comporte 6 à 30 atomes de C, système de cycle dans lequel un ou plusieurs atome(s) de H peut/peuvent être remplacé(s) par D ou par F, où deux substituants R¹⁰ adjacents ou plus peuvent former un système de cycle aliphatique monocyclique ou polycyclique l'un avec l'autre ou les uns avec les autres ;
i est pour chaque occurrence 0, 1 ou 2 ;
m est 0 ou 1 ;
o, p, r sont pour chaque occurrence, de manière identique ou différente, 0, 1 ou 2 ;
n, q sont pour chaque occurrence, de manière identique ou différente, 0, 1 ou 2 ;
s, t, u sont pour chaque occurrence, de manière identique ou différente, 0 ou 1 ;
où u + t + s ≤ 2.

8. Procédé pour la préparation d'un composé selon une ou plusieurs des revendications 1 à 7 en couplant un dérivé de dispiro[fluorèn-9,9'-anthracène-10',9"-fluorène] qui est substitué au niveau de la position 1, 3 ou 4 par un groupe partant réactif sur
a) une amine primaire, ce couplage étant suivi par un couplage sur un autre groupe aromatique, lequel est substitué par un groupe partant réactif, ou
b) sur une amine secondaire, ou
c) sur un dérivé de triarylamine.

9. Formulation comprenant au moins un composé selon une ou plusieurs des revendications 1 à 7 et au moins un solvant.

10. Utilisation d'un composé selon une ou plusieurs des revendications 1 à 7 ou d'une formulation selon la revendication 9 dans un dispositif électronique, en particulier dans un dispositif électroluminescent électronique.

11. Dispositif électronique comprenant au moins un composé selon une ou plusieurs des revendications 1 à 7 ou au moins une formulation selon la revendication 9.

12. Dispositif électronique selon la revendication 11, **caractérisé en ce qu'**il est sélectionné parmi les circuits intégrés organiques (O-IC), les transistors à effet de champ organiques (O-FET), les transistors à film mince organiques (O-TFT), les transistors à émission de lumière organiques (O-LET), les cellules solaires organiques (O-SC), les détecteurs optiques organiques, les photorécepteurs organiques, les dispositifs à extinction de champ organiques (O-FQD), les cellules électrochimiques à émission de lumière (LEC), les diodes laser organiques (O-laser) et les dispositifs électroluminescents organiques (OLED).

13. Dispositif électronique selon la revendication 12, sélectionné parmi les dispositifs électroluminescents organiques (OLED), **caractérisé en ce que** le composé selon une ou plusieurs des revendications 1 à 7 ou au moins une formulation selon la revendication 9 est utilisé(s) en tant que matériau de transport de trous dans une couche de transport de trous ou d'injection de trous, ou en tant que couche de blocage d'électrons, ou en tant que couche de blocage d'excitons ou en tant qu'émetteur pour des couches d'émission fluorescente ou en tant que matériau de matrice dans une couche d'émission.
